# EUROPEAN PATENT APPLICATION

(11) **EP 4 631 935 A1**
(43) Date of publication of application: **15.10.2025**
(21) Application number: 24890788.3
(22) Date of filing: 15.11.2024
(51) Int. Cl.: C07D 271/113, C07D 413/10, C07D 413/14, A01N 43/824, A01P 13/00

(54) **OXADIAZOLONE COMPOUND, HERBICIDAL COMPOSITION, AND USE THEREOF**

(30) Priority: 17.11.2023 CN 202311532732
(71) Applicant: Shandong Binnong Technology Co., Ltd., Binzhou, Shandong 256651 (CN)
(72) Inventor: HUANG, Yanchang, Binzhou, Shandong 256651 (CN); XU, Jiajun, Binzhou, Shandong 256651 (CN); YIN, Yunji, Binzhou, Shandong 256651 (CN); YI, Xiaowei, Binzhou, Shandong 256651 (CN); WANG, Lei, Binzhou, Shandong 256651 (CN)
(74) Representative: LLR
(86) International application number: PCT/CN2024/132155
(87) International publication number: WO 2025/103433

(57) **Abstract**

Provided herein are oxadiazolone compounds, herbicidal compositions, and uses thereof. In the present application, an oxadiazolone group of Formula (a) is provided as a substituting group on the position between the sulfonyl group and Cl on the benzene ring of a benzoyl compound to obtain an oxadiazolone compound, which not only exhibits unexpectedly high herbicidal activity against various weeds, but also is safe for various crops such as corn and rice. The experimental results show that the compound provided herein exhibits good herbicidal activity and can effectively control weeds (such as barnyard grass, crabgrass, and abutilon) at a low dose to achieve an excellent herbicidal effect, in the meanwhile, it demonstrates higher safety for crops.

## Description

### Cross-Reference to Related Applications

The present application claims priority to Chinese patent application No. 202311532732.0 filed on November 17, 2023 and entitled "Oxadiazolone compounds, herbicidal compositions and uses thereof ", which is incorporated by reference herein in its entirety.

### Technical Field

The present application relates to the technical field of herbicides, in particular to an oxadiazolone compound, a herbicidal composition, and uses thereof.

### Background Art

It has been found in research that benzoyl compounds have herbicidal activity, and their structural modification and improvement can further improve their herbicidal activity. However, compounds with high herbicidal activity have lower safety for crops. Therefore, it is one of the current research focus of benzoyl-based herbicides to develop compounds with both higher herbicidal activity and crop safety.

### Summary of the Invention

### Purposes

In view of this, the technical problem to be solved by the present application is to provide an oxadiazolone compound with both high herbicidal activity and crop safety, a composition comprising the same, and a use thereof.

### Solutions

An oxadiazolone compound of Formula (I) or tautomer thereof is provided herein: 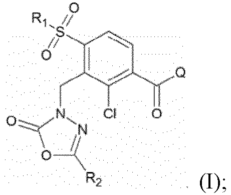
in Formula (I), R₁ and R₂ are independently selected from the group consisting of unsubstituted or substituted C₁-C₆ alkyl and C₃-C₆ cycloalkyl, wherein the substituents are 1 to 3 groups independently selected from the group consisting of halogen, nitro, C₁-C₆ alkyl, C₁-C₆ haloalkyl, C₁-C₆ alkoxy and C₁-C₆ haloalkoxy;
Q is selected from the structures of Q1, Q2 and Q3: 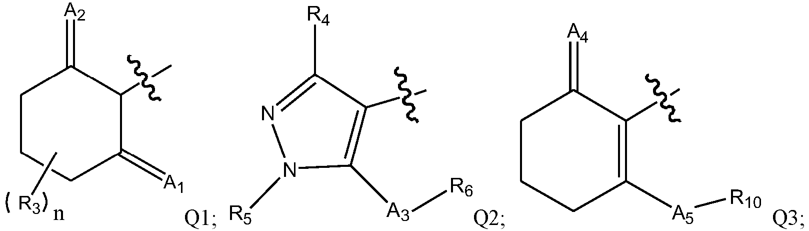
   wherein, A₁, A₂, A₃, A₄, and A₅ are independently selected from O and S;
   R₃ and R₁₀ are independently selected from the group consisting of hydrogen, unsubstituted or substituted C₁-C₆ alkyl, C₃-C₆ cycloalkyl, C₁-C₆ alkoxy, phenyl, and benzyl, wherein the substituents are 1 to 3 groups independently selected from the group consisting of halogen, nitro, C₁-C₆ alkyl, C₁-C₆ haloalkyl, C₁-C₆ alkoxy, C₁-C₆ haloalkoxy, phenyl, and halophenyl;
   n is an integer from 1 to 6;
   R₄ is selected from the group consisting of hydrogen, halogen, unsubstituted or substituted C₁-C₆ alkyl, C₃-C₆ cycloalkyl, or C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy;
   R₅ is selected from the group consisting of hydrogen, unsubstituted or substituted C₁-C₆ alkyl, and C₃-C₆ cycloalkyl, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy;
   R₆ is selected from the group consisting of hydrogen, the structure of Formula (1), unsubstituted or substituted C₁-C₆ alkyl, C₃-C₆ cycloalkyl, and C₃-C₈ heterocycloalkyl, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy; 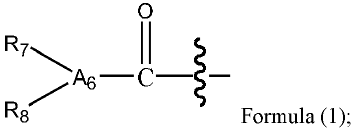
      in Formula (1), A₆ is selected from the group consisting of N and CR₉, R₉ is selected from the group consisting of hydrogen, unsubstituted or substituted C₁-C₆ alkyl, C₃-C₆ cycloalkyl, and C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy;
      R₇ and R₈ are independently selected from the group consisting of hydrogen, unsubstituted or substituted C₁-C₆ alkyl, C₃-C₆ cycloalkyl, and C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy; R₇ and R₈ are not both H;
      or, R₇ and R₈ together with A₆ to which they are attached form an unsubstituted or substituted C₃-C₈ ring comprising 0-3 heteroatoms, wherein the substituents are 1 to 3 groups independently selected from the group consisting of halogen, nitro, C₁-C₆ alkyl, C₃-C₆ cycloalkyl, C₁-C₆ haloalkyl, C₁-C₆ alkoxy, and C₁-C₆ haloalkoxy; the heteroatoms are selected from the group consisting of oxygen, sulfur, and nitrogen.

The present applicant has found that the oxadiazolone compound obtained by providing a substituting group of Formula (a) on the position between the sulfonyl group and Cl on the benzene ring of a benzoyl compound not only has excellent herbicidal activity against various weeds, but also is safer for various crops such as corn and rice: 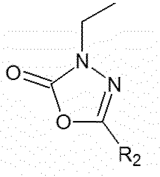 the group of Formula (a).

In some embodiments, in Formula (I), R₁ and R₂ are independently selected from the group consisting of unsubstituted or substituted C₁-C₄ alkyl and C₃-C₆ cycloalkyl, wherein the substituents are 1 to 3 groups independently selected from the group consisting of halogen, nitro, C₁-C₄ alkyl, C₁-C₄ haloalkyl, C₁-C₄ alkoxy, and C₁-C₄ haloalkoxy; in some embodiments, R₁ and R₂ are independently selected from the group consisting of -CH₃, -CH₂CH₃, -CH₂CH₂CH₃, -CH(CH₃)₂, -C(CH₃)₃, -CH₂CH₂CH₂CH₃, -CF₃, -CH₂CF₃, -CH₂CH₂CF₃, -CH(CF₃)₂, -C(CF₃)₃, -CHCH₃CF₃, -C(CH₃)₂CF₃, and CCH₂(CF₃)₂; in some embodiments, R₁ is selected from the group consisting of methyl, ethyl, n-propyl, isopropyl, n-butyl, isobutyl, and tert-butyl; R₂ is selected from the group consisting of methyl, ethyl, n-propyl, tert-butyl, and trifluoromethyl.

In some embodiments, Q is selected from the structure of Q1: 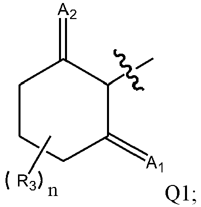 wherein, A₁ and A₂ are independently selected from O and S. In some embodiments, A₁ and A₂ are both O.

In the structure of Q1, R₃ is preferably selected from the group consisting of hydrogen, unsubstituted or substituted C₁-C₄ alkyl, C₃-C₆ cycloalkyl, C₁-C₄ alkoxy, phenyl, and benzyl, wherein the substituents are 1 to 3 groups independently selected from the group consisting of halogen, nitro, C₁-C₄ alkyl, C₁-C₄ haloalkyl, C₁-C₄ alkoxy, C₁-C₄ haloalkoxy, phenyl, and halophenyl; in some embodiments, R₃ is selected from the group consisting of hydrogen, C₁-C₄ alkyl, unsubstituted or substituted phenyl and unsubstituted or substituted benzyl, wherein the substituents are 1 to 3 halogens; in some embodiments, R₃ is selected from the group consisting of hydrogen, methyl, ethyl, propyl, isopropyl, n-butyl, isobutyl, tert-butyl, phenyl, p-chlorophenyl, 2,4-dichlorophenyl, benzyl, p-chlorobenzyl, and 2,4-dichlorobenzyl.

In the structure of Q1, n is the number of the substituent R₃, which is an integer from 1 to 6, and may be 1, 2, 3, 4, 5, or 6, and preferably 1, 2, or 3.

In some embodiments, Q is selected from the structure of Q2: 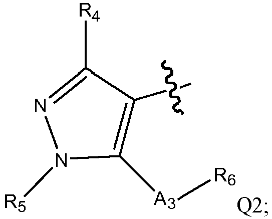 in the structure of Q2, A₃ is selected from O or S. In some embodiments, A₃ is O.

In the structure of Q2, R₄ is preferably selected from the group consisting of hydrogen, halogen, unsubstituted or substituted C₁-C₄ alkyl, C₃-C₆ cycloalkyl, and C₁-C₄ alkoxy, wherein the substituents are 1 to 3 groups independently selected from the group consisting of halogen, nitro, C₁-C₄ alkyl, C₁-C₄ haloalkyl, C₁-C₄ alkoxy, and C₁-C₄ haloalkoxy. In some embodiments, R₄ is selected from the group consisting of hydrogen, halogen, C₁-C₄ alkyl, C₁-C₄ haloalkyl, C₃-C₆ cycloalkyl, and C₁-C₄ alkoxy. In some embodiments, R₄ is selected from the group consisting of hydrogen, chlorine, C₁-C₄ alkyl, and C₃-C₆ cycloalkyl. In some embodiments, R₄ is selected from the group consisting of hydrogen, methyl, ethyl, isopropyl, and cyclopropyl.

In the structure of Q2, R₅ is preferably selected from the group consisting of hydrogen, unsubstituted or substituted C₁-C₄ alkyl and C₃-C₆ cycloalkyl, wherein the substituents are 1 to 3 groups independently selected from the group consisting of halogen, nitro, C₁-C₄ alkyl, C₁-C₄ haloalkyl, C₁-C₄ alkoxy, and C₁-C₄ haloalkoxy; in some embodiments, R₅ is selected from the group consisting of hydrogen, C₁-C₄ alkyl, C₁-C₄ haloalkyl, and C₃-C₆ cycloalkyl; in some embodiments, R₅ is selected from the group consisting of methyl, ethyl, and cyclopropyl.

In the structure of Q2, R₆ is preferably selected from the group consisting of hydrogen, the structure of Formula (1), unsubstituted or substituted C₁-C₄ alkyl, C₃-C₆ cycloalkyl, C₃-C₈ heterocycloalkyl, wherein the substituents are 1 to 3 groups independently selected from the group consisting of halogen, nitro, C₁-C₄ alkyl, C₁-C₄ haloalkyl, C₁-C₄ alkoxy, and C₁-C₄ haloalkoxy; in some embodiments, R₆ is selected from the group consisting of hydrogen, the structure of Formula (1), C₁-C₄ alkyl, C₃-C₆ cycloalkyl, C₃-C₈ heterocycloalkyl, and C₁-C₄ haloalkyl.

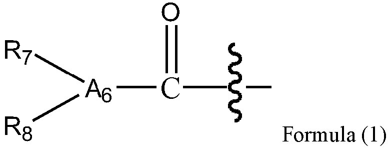

In Formula (1), A₆ is selected from the group consisting of N and CR₉, R₉ is selected from the group consisting of hydrogen, unsubstituted or substituted C₁-C₆ alkyl, C₃-C₆ cycloalkyl and C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy; in some embodiments, R₉ is selected from the group consisting of hydrogen, C₁-C₄ alkyl, C₃-C₆ cycloalkyl, C₁-C₄ alkoxy, C₁-C₄ haloalkyl, and C₁-C₄ haloalkoxy. In some embodiments, R₉ is selected from the group consisting of methyl, ethyl, n-propyl, isopropyl, n-butyl, isobutyl, and tert-butyl.

In Formula (1), R₇ and R₈ are independently selected from the group consisting of hydrogen, unsubstituted or substituted C₁-C₆ alkyl, C₃-C₆ cycloalkyl and C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy; in some embodiments, R₇ and R₈ are independently selected from the group consisting of hydrogen, C₁-C₄ alkyl, C₃-C₆ cycloalkyl, C₁-C₄ alkoxy, C₁-C₄ haloalkyl, and C₁-C₄haloalkoxy. In some embodiments, R₇ and R₈ are independently selected from the group consisting of methyl, ethyl, n-propyl, isopropyl, n-butyl, isobutyl, tert-butyl, methoxy, ethoxy, n-propoxy, isopropoxy, n-butoxy, isobutoxy, and tert-butoxy. In Formula (1), R₇ and R₈ are not both H.

In Formula (1), preferably, R₇ and R₈ together with A₆ to which they are attached form an unsubstituted or substituted C3-C₈ ring comprising 1-3 heteroatoms, wherein the substituents are 1 to 3 groups independently selected from the group consisting of halogen, nitro, C₁-C₄ alkyl, C₃-C₆ cycloalkyl, C₁-C₄ haloalkyl, C₁-C₄ alkoxy, and C₁-C₄ haloalkoxy; wherein the heteroatoms are selected from the group consisting of oxygen, sulfur, and nitrogen. In some embodiments, preferably, R₇ and R₈ together with A₆ to which they are attached form an unsubstituted or substituted heterocyclic ring, pyrazole ring, furan ring, tetrahydrofuran ring, thiophene ring, and tetrahydrothiophene ring, wherein the substituents are 1 to 3 groups independently selected from the group consisting of halogen, nitro, C₁-C₄ alkyl, C₃-C₆ cycloalkyl, C₁-C₄ haloalkyl, C₁-C₄ alkoxy, and C₁-C₄ haloalkoxy. In some embodiments, preferably, R₇ and R₈ together with A₆ to which they are attached form a ring of the structures below: 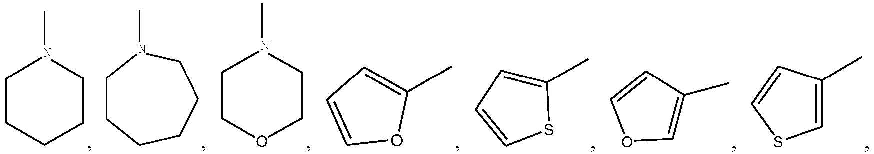 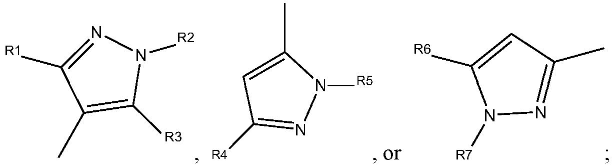 wherein R¹, R³, R⁴, and R⁶ are independently selected from the group consisting of hydrogen, halogen, C₁-C₄ alkyl, C₁-C₄ haloalkyl, and C₃-C₆ cycloalkyl, and preferably selected from the group consisting of hydrogen, chlorine, trifluoromethyl, methyl, ethyl, isopropyl, and cyclopropyl; R², R⁵ and R⁷ are independently selected from the group consisting of hydrogen, C₁-C₄ alkyl, C₁-C₄ haloalkyl, and C₃-C₆ cycloalkyl, and preferably selected from the group consisting of hydrogen, methyl, ethyl, and cyclopropyl.

In some embodiments, R₆ is preferably selected from the group consisting of the following structures: 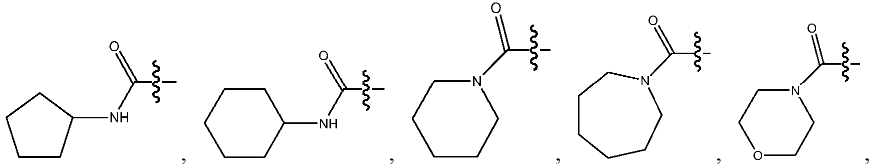 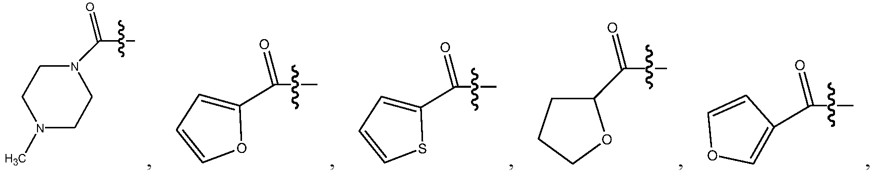 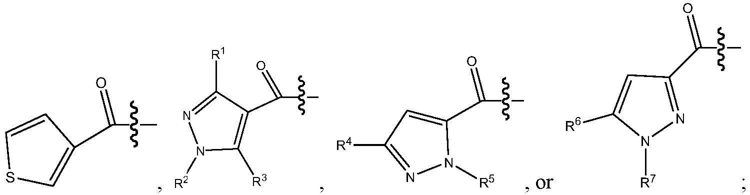 wherein R¹, R³, R⁴, and R⁶ are independently selected from the group consisting of hydrogen, halogen, C₁-C₄ alkyl, C₁-C₄ haloalkyl, and C₃-C₆ cycloalkyl, and preferably selected from the group consisting of hydrogen, chlorine, trifluoromethyl, methyl, ethyl, isopropyl, and cyclopropyl; R², R⁵ and R⁷ are independently selected from the group consisting of hydrogen, C₁-C₄ alkyl, C₁-C₄ haloalkyl, and C₃-C₆ cycloalkyl, and preferably selected from the group consisting of hydrogen, methyl, ethyl, and cyclopropyl.

In some embodiments, Q is selected from the structure of Q3: 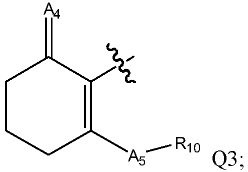 in the structure of Q3, A₄ and A₅ are independently selected from O and S; in some embodiments, A₄ is selected from O, A₅ is selected from O or S.

In the structure of Q3, R₁₀ is preferably selected from the group consisting of hydrogen, unsubstituted or substituted C₁-C₄ alkyl, C₃-C₆ cycloalkyl, C₁-C₄ alkoxy, phenyl, and benzyl, wherein the substituents are 1 to 3 groups independently selected from the group consisting of halogen, nitro, C₁-C₄ alkyl, C₁-C₄ haloalkyl, C₁-C₄ alkoxy, C₁-C₄ haloalkoxy, phenyl, and halophenyl. In some embodiments, R₁₀ is selected from the group consisting of hydrogen, C₁-C₄ alkyl, unsubstituted or substituted phenyl and unsubstituted or substituted benzyl, wherein the substituents are 1 to 3 halogens; in some embodiments, R₁₀ is selected from the group consisting of hydrogen, methyl, ethyl, propyl, isopropyl, n-butyl, isobutyl, tert-butyl, phenyl, p-chlorophenyl, 2,4-dichlorophenyl, benzyl, p-chlorobenzyl, and 2,4-dichlorobenzyl. In some embodiments, R₁₀ is selected from the group consisting of H, -CH₃, -CH₂CH₃, -CH₂CH₂CH₃, -CH(CH₃)₂, -C(CH₃)₃, -CH₂CH₂CH₂CH₃, phenyl, chlorophenyl, dichlorophenyl, C₆H₅CH₂-, C₆H₄ClCH₂-, and C₆H₃Cl₂CH₂-.

In some embodiments, Q is selected from a structure of Q1-1:

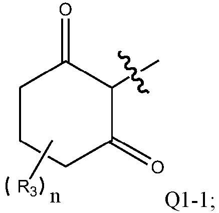

R₃ is selected from the group consisting of H, C₁-C₄ alkyl, and C₁-C₄ haloalkyl.

In some embodiments, Q is selected from a structure of Q3-1:

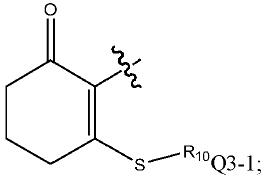

R₁₀ is selected from the group consisting of H, C₁-C₄ alkyl, C₁-C₄ haloalkyl, phenyl, halophenyl, phenyl-substituted C₁-C₄ alkyl, and halophenyl-substituted C₁-C₄ alkyl.

In some embodiments, Q is selected from a structure of Q2-1: 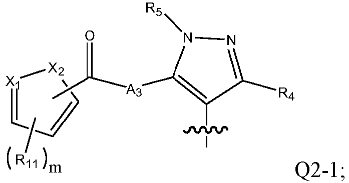
X₁ is selected from C or N; X₂ is selected from C, N, O, or S; X₁ and X₂ are not both C;
R₁₁ is selected from the group consisting of H, halogen, C₁-C₄ alkyl, C₃-C₆ cycloalkyl, and C₁-C₄ haloalkyl; in some embodiments, R₁₁ is selected from the group consisting of H, CH₃, CF₃, -CH₂CH₃, cyclopropyl, and Cl;
m is the number of the substituent R₁₁, which is an integer from 1 to 3;
R₄ is selected from the group consisting of hydrogen, halogen, C₁-C₄ alkyl, C₁-C₄ haloalkyl, C₃-C₆ cycloalkyl, C₃-C₆ halocycloalkyl, C₁-C₄ alkoxy, and C₁-C₄ haloalkoxy;
R₅ is selected from the group consisting of hydrogen, C₁-C₄ alkyl, and C₁-C₄ haloalkyl.

In some embodiments, it is selected from the group consisting of the following compounds:
Compound 1: Q is selected from the structure of Q1-1, R₁=CH₃, R₂=CH₃, R₃=H;
Compound 2: Q is selected from the structure of Q1-1, R₁=CH₃, R₂=CH₂CH₃, R₃=H;
Compound 3: Q is selected from the structure of Q1-1, R₁=CH₃, R₂=CH₂CH₂CH₃, R₃=H;
Compound 4: Q is selected from the structure of Q1-1, R₁=CH₃, R₂=C(CH₃)₃, R₃=H;
Compound 5: Q is selected from the structure of Q1-1, R₁=CH₃, R₂=CF₃, R₃=H;
Compound 6: Q is selected from the structure of Q1-1, R₁=CH₂CH₃, R₂=CH₃, R₃=H;
Compound 7: Q is selected from the structure of Q1-1, R₁=CH₂CH₃, R₂= CH₂CH₃, R₃=H;
Compound 8: Q is selected from the structure of Q1-1, R₁=CH₂CH₃, R₂=CH₂CH₂CH₃, R₃=H;
Compound 9: Q is selected from the structure of Q1-1, R₁=CH₂CH₃, R₂=C(CH₃)₃, R₃=H;
Compound 10: Q is selected from the structure of Q1-1, R₁=CH₂CH₃, R₂=CF₃, R₃=H;
Compound 11: Q is selected from the structure of Q1-1, R₁=CH₂CH₂CH₃, R₂=CH₃, R₃=H;
Compound 12: Q is selected from the structure of Q1-1, R₁=CH₂CH₂CH₃, R₂=CH₂CH₃, R₃=H;
Compound 13: Q is selected from the structure of Q1-1, R₁= CH₂CH₂CH₃, R₂=CH₂CH₂CH₃, R₃=H;
Compound 14: Q is selected from the structure of Q1-1, R₁=CH₂CH₂CH₃, R₂=C(CH₃)₃, R₃=H;
Compound 15: Q is selected from the structure of Q1-1, R₁=CH₂CH₂CH₃, R₂=CF₃, R₃=H;
Compound 16: Q is selected from the structure of Q1-1, R₁=CH(CH₃)₂, R₂=CH₃, R₃=H;
Compound 17: Q is selected from the structure of Q1-1, R₁=CH(CH₃)₂, R₂=CH₂CH₃, R₃=H;
Compound 18: Q is selected from the structure of Q1-1, R₁=CH(CH₃)₂, R₂=CH₂CH₂CH₃, R₃=H;
Compound 19: Q is selected from the structure of Q1-1, R₁=CH(CH₃)₂, R₂=C(CH₃)₃, R₃=H;
Compound 20: Q is selected from the structure of Q1-1, R₁=CH(CH₃)₂, R₂=CF₃, R₃=H;
Compound 21: Q is selected from the structure of Q1-1, R₁=CH₂CH₂CH₂CH₃, R₂=CH₃, R₃=H;
Compound 22: Q is selected from the structure of Q1-1, R₁=CH₂CH₂CH₂CH₃, R₂=CH₂CH₃, R₃=H;
Compound 23: Q is selected from the structure of Q1-1, R₁=CH₂CH₂CH₂CH₃, R₂=CH₂CH₂CH₃, R₃=H;
Compound 24: Q is selected from the structure of Q1-1, R₁=CH₂CH₂CH₂CH₃, R₂=C(CH₃)₃, R₃=H;
Compound 25: Q is selected from the structure of Q1-1, R₁=CH₂CH₂CH₂CH₃, R₂=CF₃, R₃=H;
Compound 26: Q is selected from the structure of Q1-1, R₁=C(CH₃)₃, R₂=CH₃, R₃=H;
Compound 27: Q is selected from the structure of Q1-1, R₁=C(CH₃)₃, R₂=CH₂CH₃, R₃=H;
Compound 28: Q is selected from the structure of Q1-1, R₁=C(CH₃)₃, R₂=CH₂CH₂CH₃, R₃=H;
Compound 29: Q is selected from the structure of Q1-1, R₁=C(CH₃)₃, R₂=C(CH₃)₃, R₃=H;
Compound 30: Q is selected from the structure of Q1-1, R₁=C(CH₃)₃, R₂=CF₃, R₃=H;
Compound 31: Q is selected from the structure of Q3, R₁=CH₃, R₂=CH₃, A₄=O, A₅=O, R₁₀=CH₃;
Compound 32: Q is selected from the structure of Q3, R₁=CH₃, R₂=CH₃, A₄=O, A₅=O, R₁₀= CH₂CH₃;
Compound 33: Q is selected from the structure of Q3, R₁=CH₃, R₂=CH₃, A₄=O, A₅=O, R₁₀= CH₂CH₂CH₃;
Compound 34: Q is selected from the structure of Q3, R₁=CH₃, R₂=CH₃, A₄=O, A₅=O, R₁₀= CH(CH₃)₂;
Compound 35: Q is selected from the structure of Q3, R₁=CH₃, R₂=CH₃, A₄=O, A₅=O, R₁₀= CH₂CH₂CH₂CH₃;
Compound 36: Q is selected from the structure of Q3, R₁=CH₃, R₂=CH₃, A₄=O, A₅=O, R₁₀=C(CH₃)₃;
Compound 37: Q is selected from the structure of Q3, R₁=CH₃, R₂=CH₃, A₄=O, A₅=O, 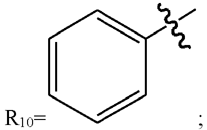
Compound 38: Q is selected from the structure of Q3, R₁=CH₃, R₂=CH₃, A₄=O, A₅=O, 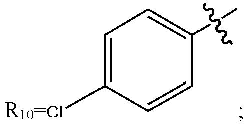
Compound 39: Q is selected from the structure of Q3, R₁=CH₃, R₂=CH₃, A₄=O, A₅=O, R₁₀= 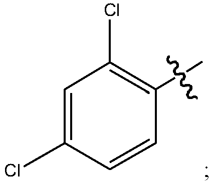
Compound 40: Q is selected from the structure of Q3, R₁=CH₃, R₂=CH₃, A₄=O, A₅=O, 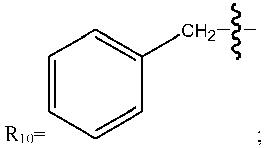
Compound 41: Q is selected from the structure of Q3, R₁=CH₃, R₂=CH₃, A₄=O, A₅=O, 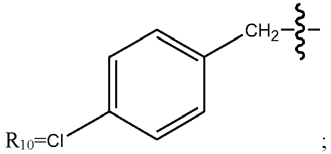
Compound 42: Q is selected from the structure of Q3, R₁=CH₃, R₂=CH₃, A₄=O, A₅=O, R₁₀= 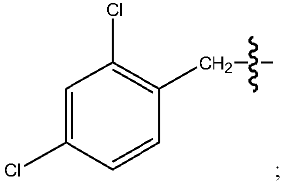
Compound 43: Q is selected from the structure of Q3, R₁=CH₃, R₂=CH₃, A₄=O, A₅=O, R₁₀= 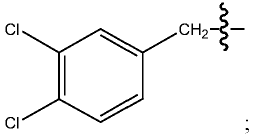
Compound 44: Q is selected from the structure of Q3, R₁=CH₃, R₂=CH₃, A₄=O, A₅=S, R₁₀= CH₃;
Compound 45: Q is selected from the structure of Q3, R₁=CH₃, R₂=CH₃, A₄=O, A₅=S, R₁₀= CH₂CH₃;
Compound 46: Q is selected from the structure of Q3, R₁=CH₃, R₂=CH₃, A₄=O, A₅=S, R₁₀= CH₂CH₂CH₃;
Compound 47: Q is selected from the structure of Q3, R₁=CH₃, R₂=CH₃, A₄=O, A₅=S, R₁₀= CH(CH₃)₂;
Compound 48: Q is selected from the structure of Q3, R₁=CH₃, R₂=CH₃, A₄=O, A₅=S, R₁₀= CH₂CH₂CH₂CH₃;
Compound 49: Q is selected from the structure of Q3, R₁=CH₃, R₂=CH₃, A₄=O, A₅=S, R₁₀= C(CH₃)₃,
Compound 50: Q is selected from the structure of Q3, R₁=CH₃, R₂=CH₃, A₄=O, A₅=S, 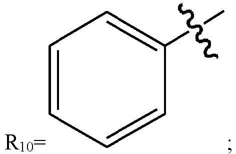
Compound 51: Q is selected from the structure of Q3, R₁=CH₃, R₂=CH₃, A₄=O, A₅=S, 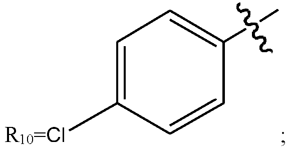
Compound 52: Q is selected from the structure of Q3, R₁=CH₃, R₂=CH₃, A₄=O, A₅=S, R₁₀= 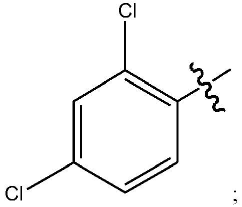
Compound 53: Q is selected from the structure of Q3, R₁=CH₃, R₂=CH₃, A₄=O, A₅=S, 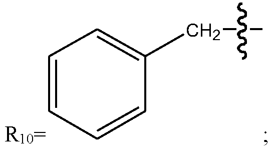
Compound 54: Q is selected from the structure of Q3, R₁=CH₃, R₂=CH₃, A₄=O, A₅=S, 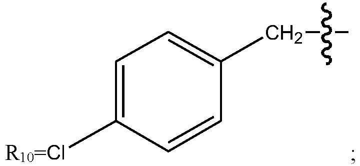
Compound 55: Q is selected from the structure of Q3, R₁=CH₃, R₂=CH₃, A₄=O, A₅=S, R₁₀= 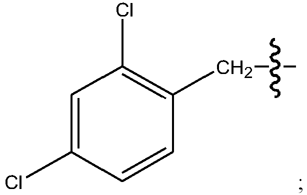
Compound 56: Q is selected from the structure of Q2, A₃=O, R₁=CH₃, R₂=CH₃, R₄=H, R₅=CH₃, R₆=H;
Compound 57: Q is selected from the structure of Q2, A₃=O, R₁=CH₃, R₂=CH₃, R₄=H, R₅=CH₂CH₃, R₆=H;
Compound 58: Q is selected from the structure of Q2, A₃=O, R₁=CH₃, R₂=CH₃, R₄=CH₃, R₅=CH₃, R₆=H;
Compound 59: Q is selected from the structure of Q2, A₃=O, R₁=CH₃, R₂=CH₃, R₄=CH₃, R₅=CH₂CH₃, R₆=H;
Compound 60: Q is selected from the structure of Q2, A₃=O, R₁=CH₃, R₂=CH₃, R₄= CH₂CH₃, R₅=CH₃, R₆=H;
Compound 61: Q is selected from the structure of Q2, A₃=O, R₁=CH₃, R₂=CH₃, R₄=CH₂CH₃, R₅=CH₂CH₃, R₆=H;
Compound 62: Q is selected from the structure of Q2, A₃=O, R₁=CH₃, R₂=CH₃, R₄=CH₂CH₂CH₃, R₅=CH₃, R₆=H;
Compound 63: Q is selected from the structure of Q2, A₃=O, R₁=CH₃, R₂=CH₃, R₄=CH₂CH₂CH₃, R₅=CH₂CH₃, R₆=H;
Compound 64: Q is selected from the structure of Q2, A₃=O, R₁=CH₃, R₂=CH₃, R₄=CH(CH₃)₂, R₅=CH₃, R₆=H;
Compound 65: Q is selected from the structure of Q2, A₃=O, R₁=CH₃, R₂=CH₃, R₄=CH(CH₃)₂, R₅=CH₂CH₃, R₆=H;
Compound 66: Q is selected from the structure of Q2, A₃=O, R₁=CH₃, R₂=CH₃, R₄=CF₃, R₅=CH₃, R₆=H;
Compound 67: Q is selected from the structure of Q2, A₃=O, R₁=CH₃, R₂=CH₃, R₄=CF₃, R₅=CH₂CH₃, R₆=H;
Compound 68: Q is selected from the structure of Q2, A₃=O, R₁=CH₃, R₂=CH₃, 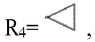 R₅=CH₃, R₆=H;
Compound 69: Q is selected from the structure of Q2, A₃=O, R₁=CH₃, R₂=CH₃, 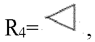 R₅=CH₂CH₃, R₆=H;
Compound 70: Q is selected from the structure of Q2, A₃=O, R₁=CH₂CH₃, R₂=CH₃, R₄=H, R₅=CH₃, R₆=H;
Compound 71: Q is selected from the structure of Q2, A₃=O, R₁= CH₂CH₃, R₂=CH₃, R₄=H, R₅=CH₂CH₃, R₆=H;
Compound 72: Q is selected from the structure of Q2, A₃=O, R₁= CH₂CH₃, R₂=CH₃, R₄=CH₃, R₅=CH₃, R₆=H;
Compound 73: Q is selected from the structure of Q2, A₃=O, R₁= CH₂CH₃, R₂=CH₃, R₄=CH₃, R₅=CH₂CH₃, R₆=H;
Compound 74: Q is selected from the structure of Q2, A₃=O, R₁=CH₂CH₃, R₂=CH₃, R₄= CH₂CH₃, R₅=CH₃, R₆=H;
Compound 75: Q is selected from the structure of Q2, A₃=O, R₁=CH₂CH₃, R₂=CH₃, R₄=CH₂CH₃, R₅=CH₂CH₃, R₆=H;
Compound 76: Q is selected from the structure of Q2, A₃=O, R₁=CH₂CH₃, R₂=CH₃, R₄=CH₂CH₂CH₃, R₅=CH₃, R₆=H;
Compound 77: Q is selected from the structure of Q2, A₃=O, R₁=CH₂CH₃, R₂=CH₃, R₄=CH₂CH₂CH₃, R₅=CH₂CH₃, R₆=H;
Compound 78: Q is selected from the structure of Q2, A₃=O, R₁=CH₂CH₃, R₂=CH₃, R₄=CH(CH₃)₂, R₅=CH₃, R₆=H;
Compound 79: Q is selected from the structure of Q2, A₃=O, R₁=CH₂CH₃, R₂=CH₃, R₄=CH(CH₃)₂, R₅=CH₂CH₃, R₆=H;
Compound 80: Q is selected from the structure of Q2, A₃=O, R₁=CH₂CH₃, R₂=CH₃, R₄=CF₃, R₅=CH₃, R₆=H;
Compound 81: Q is selected from the structure of Q2, A₃=O, R₁=CH₂CH₃, R₂=CH₃, R₄=CF₃, R₅=CH₂CH₃, R₆=H;
Compound 82: Q is selected from the structure of Q2, A₃=O, R₁=CH₂CH₃, R₂=CH₃, 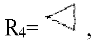 R₅=CH₃, R₆=H;
Compound 83: Q is selected from the structure of Q2, A₃=O, R₁=CH₂CH₃, R₂=CH₃, 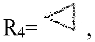
Compound 84: Q is selected from the stucture of Q2, A₃=O, R₁=CH₂CH₂CH₃,R₂=CH₃, r₄=H, R₅=CH₃, R₆=H;
Compound 85: Q is selected from the structure of Q2, A₃=O, R₁=CH₂CH₂CH₃, R₂=CH₃, R₄=H, R₅=CH₂CH₃, R₆=H;
Compound 86: Q is selected from the structure of Q2, A₃=O, R₁=CH₂CH₂CH₃, R₂=CH₃, R₄=CH₃, R₅=CH₃, R₆=H;
Compound 87: Q is selected from the structure of Q2, A₃=O, R₁=CH₂CH₂CH₃, R₂=CH₃, R₄=CH₃, R₅=CH₂CH₃, R₆=H;
Compound 88: Q is selected from the structure of Q2, A₃=O, R₁=CH₂CH₂CH₃, R₂=CH₃, R₄= CH₂CH₃, R₅=CH₃, R₆=H;
Compound 89: Q is selected from the structure of Q2, A₃=O, R₁=CH₂CH₂CH₃, R₂=CH₃, R₄=CH₂CH₃, R₅=CH₂CH₃, R₆=H;
Compound 90: Q is selected from the structure of Q2, A₃=O, R₁=CH₂CH₂CH₃, R₂=CH₃, R₄=CH₂CH₂CH₃, R₅=CH₃, R₆=H;
Compound 91: Q is selected from the structure of Q2, A₃=O, R₁=CH₂CH₂CH₃, R₂=CH₃, R₄=CH₂CH₂CH₃, R₅=CH₂CH₃, R₆=H;
Compound 92: Q is selected from the structure of Q2, A₃=O, R₁=CH₂CH₂CH₃, R₂=CH₃, R₄=CH(CH₃)₂, R₅=CH₃, R₆=H;
Compound 93: Q is selected from the structure of Q2, A₃=O, R₁=CH₂CH₂CH₃, R₂=CH₃, R₄=CH(CH₃)₂, R₅=CH₂CH₃, R₆=H;
Compound 94: Q is selected from the structure of Q2, A₃=O, R₁=CH₂CH₂CH₃, R₂=CH₃, R₄=CF₃, R₅=CH₃, R₆=H;
Compound 95: Q is selected from the structure of Q2, A₃=O, R₁=CH₂CH₂CH₃, R₂=CH₃, R₄=CF₃, R₅=CH₂CH₃, R₆=H;
Compound 96: Q is selected from the structure of Q2, A₃=O, R₁=CH₂CH₂CH₃, R₂=CH₃, 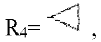 R₅=CH₃, R₆=H;
Compound 97: Q is selected from the structure of Q2, A₃=O, R₁=CH₂CH₂CH₃, R₂=CH₃, 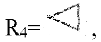 R₅=CH₂CH₃, R₆=H;
Compound 98: Q is selected from the structure of Q2, A₃=O, R₁=CH(CH₃)₂, R₂=CH₃, R₄=H, R₅=CH₃, R₆=H;
Compound 99: Q is selected from the structure of Q2, A₃=O, R₁=CH(CH₃)₂, R₂=CH₃, R₄=H, R₅=CH₂CH₃, R₆=H;
Compound 100: Q is selected from the structure of Q2, A₃=O, R₁=CH(CH₃)₂, R₂=CH₃, R₄=CH₃, R₅=CH₃, R₆=H;
Compound 101: Q is selected from the structure of Q2, A₃=O, R₁=CH(CH₃)₂, R₂=CH₃, R₄=CH₃, R₅=CH₂CH₃, R₆=H;
Compound 102: Q is selected from the structure of Q2, A₃=O, R₁=CH(CH₃)₂, R₂=CH₃, R₄= CH₂CH₃, R₅=CH₃, R₆=H;
Compound 103: Q is selected from the structure of Q2, A₃=O, R₁=CH(CH₃)₂, R₂=CH₃, R₄=CH₂CH₃, R₅=CH₂CH₃, R₆=H;
Compound 104: Q is selected from the structure of Q2, A₃=O, R₁=CH(CH₃)₂, R₂=CH₃, R₄=CH₂CH₂CH₃, R₅=CH₃, R₆=H;
Compound 105: Q is selected from the structure of Q2, A₃=O, R₁=CH(CH₃)₂, R₂=CH₃, R₄=CH₂CH₂CH₃, R₅=CH₂CH₃, R₆=H;
Compound 106: Q is selected from the structure of Q2, A₃=O, R₁=CH(CH₃)₂, R₂=CH₃, R₄=CH(CH₃)₂, R₅=CH₃, R₆=H;
Compound 107: Q is selected from the structure of Q2, A₃=O, R₁=CH(CH₃)₂, R₂=CH₃, R₄=CH(CH₃)₂, R₅=CH₂CH₃, R₆=H;
Compound 108: Q is selected from the structure of Q2, A₃=O, R₁=CH(CH₃)₂, R₂=CH₃, R₄=CF₃, R₅=CH₃, R₆=H;
Compound 109: Q is selected from the structure of Q2, A₃=O, R₁=CH(CH₃)₂, R₂=CH₃, R₄=CF₃, R₅=CH₂CH₃, R₆=H;
Compound 110: Q is selected from the structure of Q2, A₃=O, R₁=CH(CH₃)₂, R₂=CH₃, 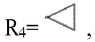 R₅=CH₃, R₆=H;
Compound 111: Q is selected from the structure of Q2, A₃=O, R₁=CH(CH₃)₂, R₂=CH₃, 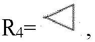 R₅=CH₂CH₃, R₆=H;
Compound 112: Q is selected from the structure of Q2, A₃=O, R₁=CH₂CH₂CH₂CH₃, R₂=CH₃, R₄=H, R₅=CH₃, R₆=H;
Compound 113: Q is selected from the structure of Q2, A₃=O, R₁=CH₂CH₂CH₂CH₃, R₂=CH₃, R₄=H, R₅=CH₂CH₃, R₆=H;
Compound 114: Q is selected from the structure of Q2, A₃=O, R₁=CH₂CH₂CH₂CH₃, R₂=CH₃, R₄=CH₃, R₅=CH₃, R₆=H;
Compound 115: Q is selected from the structure of Q2, A₃=O, R₁=CH₂CH₂CH₂CH₃, R₂=CH₃, R₄=CH₃, R₅=CH₂CH₃, R₆=H;
Compound 116: Q is selected from the structure of Q2, A₃=O, R₁=CH₂CH₂CH₂CH₃, R₂=CH₃, R₄= CH₂CH₃, R₅=CH₃, R₆=H;
Compound 117: Q is selected from the structure of Q2, A₃=O, R₁=CH₂CH₂CH₂CH₃, R₂=CH₃, R₄=CH₂CH₃, R₅=CH₂CH₃, R₆=H;
Compound 118: Q is selected from the structure of Q2, A₃=O, R₁=CH₂CH₂CH₂CH₃, R₂=CH₃, R₄=CH₂CH₂CH₃, R₅=CH₃, R₆=H;
Compound 119: Q is selected from the structure of Q2, A₃=O, R₁=CH₂CH₂CH₂CH₃, R₂=CH₃, R₄=CH₂CH₂CH₃, R₅=CH₂CH₃, R₆=H;
Compound 120: Q is selected from the structure of Q2, A₃=O, R₁=CH₂CH₂CH₂CH₃, R₂=CH₃, R₄=CH(CH₃)₂, R₅=CH₃, R₆=H;
Compound 121: Q is selected from the structure of Q2, A₃=O, R₁= CH₂CH₂CH₂CH₃, R₂=CH₃, R₄=CH(CH₃)₂, R₅=CH₂CH₃, R₆=H;
Compound 122: Q is selected from the structure of Q2, A₃=O, R₁= CH₂CH₂CH₂CH₃, R₂=CH₃, R₄=CF₃, R₅=CH₃, R₆=H;
Compound 123: Q is selected from the structure of Q2, A₃=O, R₁=CH₂CH₂CH₂CH₃, R₂=CH₃, R₄=CF₃, R₅=CH₂CH₃, R₆=H;
Compound 124: Q is selected from the structure of Q2, A₃=O, R₁=CH₂CH₂CH₂CH₃, R₂=CH₃, R₄= 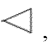 R₅=CH₃, R₆=H;
Compound 125: Q is selected from the structure of Q2, A₃=O, R₁=CH₂CH₂CH₂CH₃, R₂=CH₃, 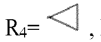 R₅=CH₂CH₃, R₆=H;
Compound 126: Q is selected from the structure of Q2, A₃=O, R₁=C(CH₃)₃, R₂=CH₃, R₄=H, R₅=CH₃, R₆=H;
Compound 127: Q is selected from the structure of Q2, A₃=O, R₁= C(CH₃)₃, R₂=CH₃, R₄=H, R₅=CH₂CH₃, R₆=H;
Compound 128: Q is selected from the structure of Q2, A₃=O, R₁= C(CH₃)₃, R₂=CH₃, R₄=CH₃, R₅=CH₃, R₆=H;
Compound 129: Q is selected from the structure of Q2, A₃=O, R₁= C(CH₃)₃, R₂=CH₃, R₄=CH₃, R₅=CH₂CH₃, R₆=H;
Compound 130: Q is selected from the structure of Q2, A₃=O, R₁=C(CH₃)₃, R₂=CH₃, R₄= CH₂CH₃, R₅=CH₃, R₆=H;
Compound 131: Q is selected from the structure of Q2, A₃=O, R₁=C(CH₃)₃, R₂=CH₃, R₄=CH₂CH₃, R₅=CH₂CH₃, R₆=H;
Compound 132: Q is selected from the structure of Q2, A₃=O, R₁=C(CH₃)₃, R₂=CH₃, R₄=CH₂CH₂CH₃, R₅=CH₃, R₆=H;
Compound 133: Q is selected from the structure of Q2, A₃=O, R₁=C(CH₃)₃, R₂=CH₃, R₄=CH₂CH₂CH₃, R₅=CH₂CH₃, R₆=H;
Compound 134: Q is selected from the structure of Q2, A₃=O, R₁= C(CH₃)₃, R₂=CH₃, R₄=CH(CH₃)₂, R₅=CH₃, R₆=H;
Compound 135: Q is selected from the structure of Q2, A₃=O, R₁= C(CH₃)₃, R₂=CH₃, R₄=CH(CH₃)₂, R₅=CH₂CH₃, R₆=H;
Compound 136: Q is selected from the structure of Q2, A₃=O, R₁= C(CH₃)₃, R₂=CH₃, R₄=CF₃, R₅=CH₃, R₆=H;
Compound 137: Q is selected from the structure of Q2, A₃=O, R₁= C(CH₃)₃, R₂=CH₃, R₄=CF₃, R₅=CH₂CH₃, R₆=H;
Compound 138: Q is selected from the structure of Q2, A₃=O, R₁= C(CH₃)₃, R₂=CH₃, 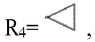 R₅=CH₃, R₆=H;
Compound 139: Q is selected from the structure of Q2, A₃=O, R₁= C(CH₃)₃, R₂=CH₃, 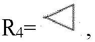 R₅=CH₂CH₃, R₆=H;
Compound 140: Q is selected from the structure of Q2, A₃=S, R₁=CH₃, R₂=CH₃, R₄=CH₃, R₅=CH₃, R₆=(CH₃CH₂)₂NCO;
Compound 141: Q is selected from the structure of Q2, A₃=O, R₁=CH₃, R₂=CH₃, R₄=CH₃, R₅=CH₃, R₆=(CH₃CH₂CH₂)₂NCO;
Compound 142: Q is selected from the structure of Q2, A₃=O, R₁=CH₂CH₃, R₂=CH₃, R₄=CH₃, R₅=CH₃, R₆=(CH₃CH₂CH₂)₂NCO;
Compound 143: Q is selected from the structure of Q2, A₃=O, R₁=CH(CH₃)₂, R₂=CH₃, R₄=CH₃, R₅=CH₃, R₆=(CH₃CH₂CH₂)₂NCO;
Compound 144: Q is selected from the structure of Q2, A₃=O, R₁=CH₃, R₂=CH₃, 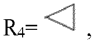 R₅=CH₃, R₆=(CH₃CH₂CH₂)₂NCO;
Compound 145: Q is selected from the structure of Q2, A₃=S, R₁=CH₃, R₂=CH₃, R₄=CH₃, R₅=CH₃, R₆=(CH₃OCH₂CH₂)₂NCO;
Compound 146: Q is selected from the structure of Q2, A₃=S, R₁=CH₃, R₂=CH₃, R₄=CH₃, R₅=CH₃, 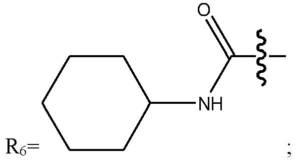
Compound 147: Q is selected from the structure of Q2, A₃=S, R₁=CH₃, R₂=CH₃, R₄=CH₃, R₅=CH₃, 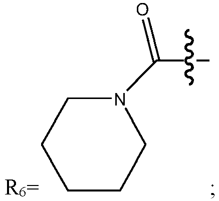
Compound 148: Q is selected from the structure of Q2, A₃=S, R₁=CH₃, R₂=CH₃, R₄=CH₃, R₅=CH₃, 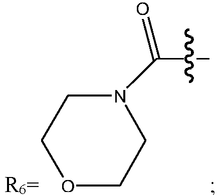
Compound 149: Q is selected from the structure of Q2, A₃=O, R₁=CH₃, R₂=CH₃, R₄=CH₃, R₅=CH₃, R₆= 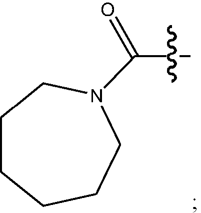
Compound 150: Q is selected from the structure of Q2, A₃=O, R₁=CH₂CH₃, R₂=CH₃, R₄=CH₃, R₅=CH₃ 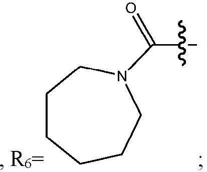
Compound 151: Q is selected from the structure of Q2, A₃=O, R₁=CH(CH₃)₂, R₂=CH₃, R₄=CH₃, R₅=CH₃, R₆= 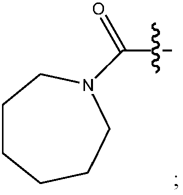
Compound 152: Q is selected from the structure of Q2, A₃=O, R₁=CH₃, R₂=CH₃, 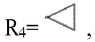 R₅=CH₃, 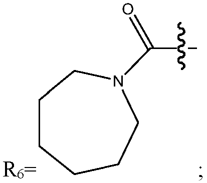
Compound 153: Q is selected from the structure of Q2, A₃=O, R₁=CH₂CH₃, R₂=CH₃, 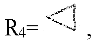 R₅=CH₃, 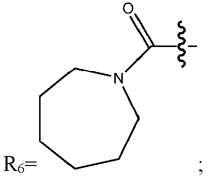
Compound 154: Q is selected from the structure of Q2, A₃=O, R₁=CH₃, R₂=CH₃, R₄=CH₃, R₅=CH₃, 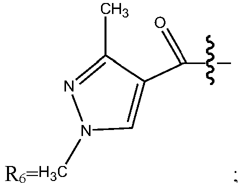
Compound 155: Q is selected from the structure of Q2, A₃=O, R₁=CH₂CH₃, R₂=CH₃, R₄=CH₃, R₅=CH₃, 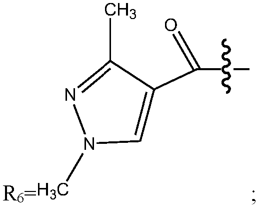
Compound 156: Q is selected from the structure of Q2, A₃=O, R₁=CH₂CH₂CH₃, R₂=CH₃, R₄=CH₃, R₅=CH₃. 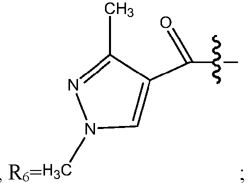
Compound 157: Q is selected from the structure of Q2, A₃=O, R₁=CH(CH₃)₂, R₂=CH₃, R₄=CH₃, R₅=CH₃, 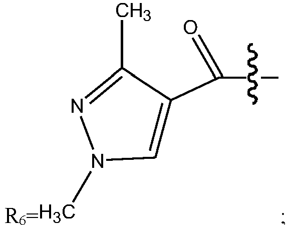
Compound 158: Q is selected from the structure of Q2, A₃=O, R₁=CH₃, R₂=CH₃, R₄=H, R₅=CH₃, 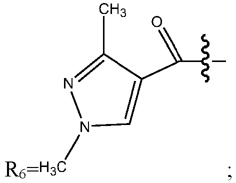
Compound 159: Q is selected from the structure of Q2, A₃=O, R₁=CH₂CH₃, R₂=CH₃, R₄=H, R₅=CH₃, 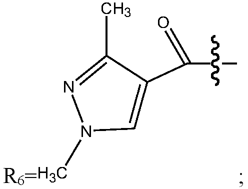
Compound 160: Q is selected from the structure of Q2, A₃=O, R₁=CH₂CH₂CH₃, R₂=CH₃, R₄=H, R₅=CH₃, 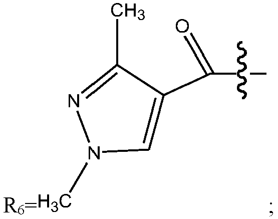
Compound 161: Q is selected from the structure of Q2, A₃=O, R₁=CH(CH₃)₂, R₂=CH₃, R₄=H, R₅=CH₃, 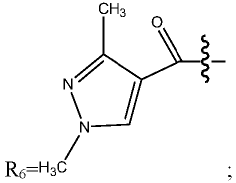
Compound 162: Q is selected from the structure of Q2, A₃=O, R₁=CH₃, R₂=CH₃, 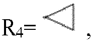 R₅=CH₃, R₆= 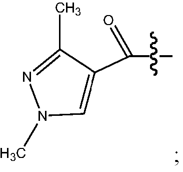
Compound 163: Q is selected from the structure of Q2, A₃=O, R₁=CH₂CH₃, R₂=CH₃, 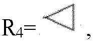 R₅=CH₃, 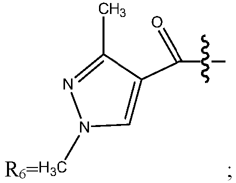
Compound 164: Q is selected from the structure of Q2, A₃=O, R₁=CH₂CH₂CH₃, R₂=CH₃, 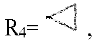 R₅=CH₃, 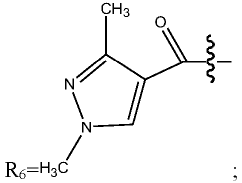
Compound 165: Q is selected from the structure of Q2, A₃=O, R₁=CH(CH₃)₂, R₂=CH₃, 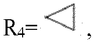 R₅=CH₃, 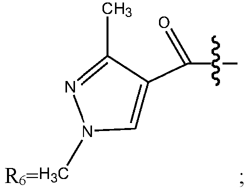
Compound 166: Q is selected from the structure of Q2, A₃=O, R₁=CH₃, R₂=CH₃, R₄= CH₃, R₅=CH₃, 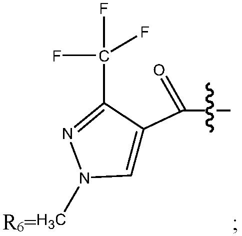
Compound 167: Q is selected from the structure of Q2, A₃=O, R₁=CH₂CH₃, R₂=CH₃, R₄=CH₃, R₅=CH₃, 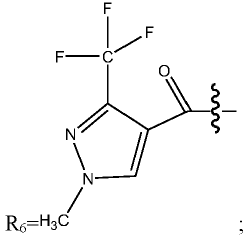
Compound 168: Q is selected from the structure of Q2, A₃=O, R₁=CH₂CH₂CH₃, R₂=CH₃, R₄=CH₃, R₅=CH₃, 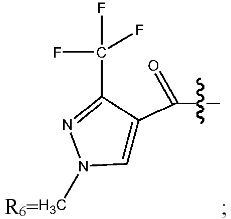
Compound 169: Q is selected from the structure of Q2, A₃=O, R₁=CH(CH₃)₂, R₂=CH₃, R₄=CH₃, R₅=CH₃. 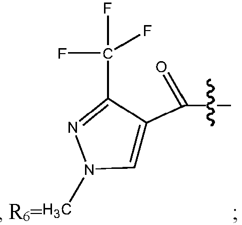
Compound 170: Q is selected from the structure of Q2, A₃=O, R₁=CH₃, R₂=CH₃, R₄=H, R₅=CH₃, 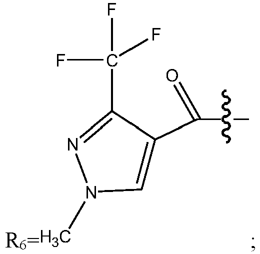
Compound 171: Q is selected from the structure of Q2, A₃=O, R₁=CH₂CH₃, R₂=CH₃, R₄=H, R₅=CH₃, 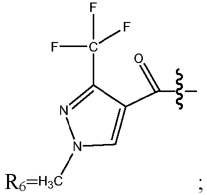
Compound 172: Q is selected from the structure of Q2, A₃=O, R₁=CH₂CH₂CH₃, R₂=CH₃, R₄=H, R₅=CH₃, 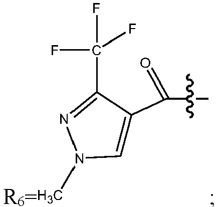
Compound 173: Q is selected from the structure of Q2, A₃=O, R₁=CH(CH₃)₂, R₂=CH₃, R₄=H, R₅=CH₃, 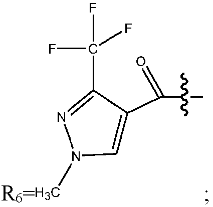
Compound 174: Q is selected from the structure of Q2, A₃=O, R₁=CH₃, R₂=CH₃, 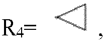 R₅=CH₃, 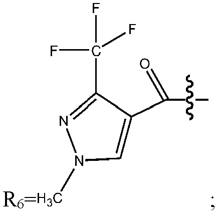
Compound 175: Q is selected from the structure of Q2, A₃=O, R₁=CH₂CH₃, R₂=CH₃, 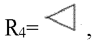 R₅=CH₃, 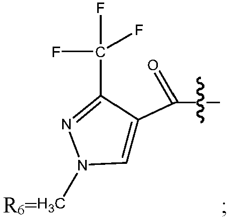
Compound 176: Q is selected from the structure of Q2, A₃=O, R₁=CH₂CH₂CH₃, R₂=CH₃, 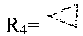 , R₅=CH₃, 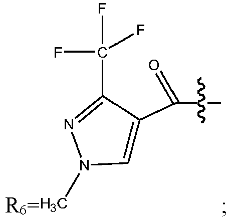
Compound 177: Q is selected from the structure of Q2, A₃=O, R₁=CH(CH₃)₂, R₂=CH₃, 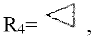 R₅=CH₃, 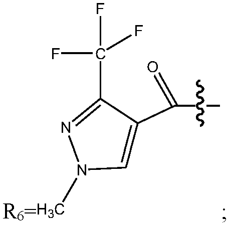
Compound 178: Q is selected from the structure of Q2, A₃=O, R₁=CH₃, R₂=CH₃, R₄=CH₃, R₅=CH₃, 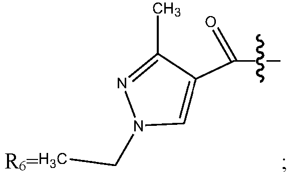
Compound 179: Q is selected from the structure of Q2, A₃=O, R₁=CH₂CH₂CH₃, R₂=CH₃, R₄=CH₃, R₅=CH₃, 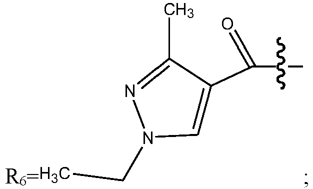
Compound 180: Q is selected from the structure of Q2, A₃=O, R₁=CH₃, R₂=CH₃, R₄=H, R₅=CH₃, 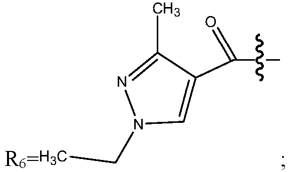
Compound 181: Q is selected from the structure of Q2, A₃=O, R₁=CH₂CH₂CH₃, R₂=CH₃, R₄=H, R₅=CH₃, 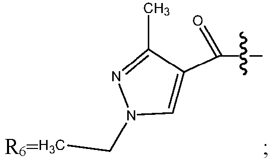
Compound 182: Q is selected from the structure of Q2, A₃=O, R₁=CH₃, R₂=CH₃, 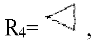 R₅=CH₃, 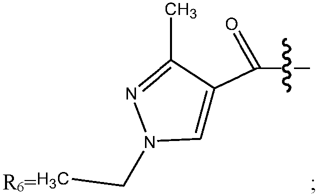
Compound 183: Q is selected from the structure of Q2, A₃=O, R₁=CH₂CH₂CH₃, R₂=CH₃, 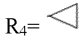 , R₅=CH₃. 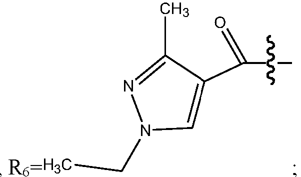
Compound 184: Q is selected from the structure of Q2, A₃=O, R₁=CH₂CH₃, R₂=CH₃, R₄=CH₃, R₅=CH₃, 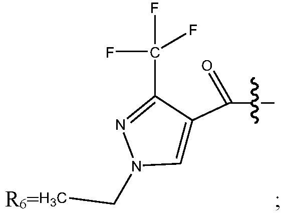
Compound 185: Q is selected from the structure of Q2, A₃=O, R₁=CH(CH₃)₂, R₂=CH₃, R₄=CH₃, R₅=CH₃, 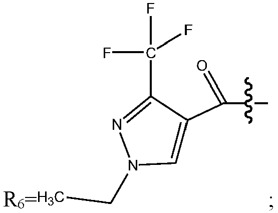
Compound 186: Q is selected from the structure of Q2, A₃=O, R₁=CH₂CH₃, R₂=CH₃, R₄=H, R₅=CH₃, 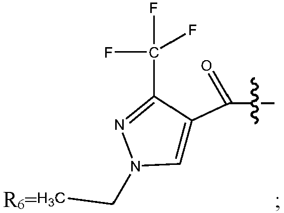
Compound 187: Q is selected from the structure of Q2, A₃=O, R₁=CH(CH₃)₂, R₂=CH₃, R₄=H, R₅=CH₃, 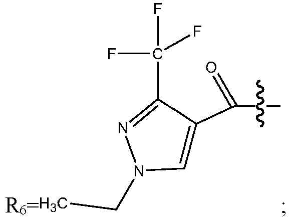
Compound 188: Q is selected from the structure of Q2, A₃=O, R₁=CH₂CH₃, R₂=CH₃, 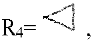 R₅=CH₃, 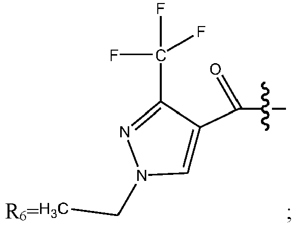
Compound 189: Q is selected from the structure of Q2, A₃=O, R₁=CH(CH₃)₂, R₂=CH₃, 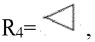 R₅=CH₃, 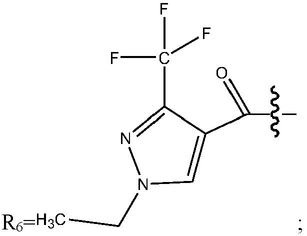
Compound 190: Q is selected from the structure of Q2, A₃=O, R₁=CH₂CH₃, R₂=CH₃, R₄=CH₃, R₅=CH2CH₃, 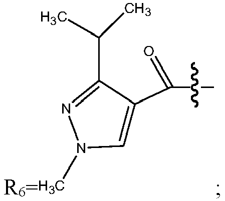
Compound 191: Q is selected from the structure of Q2, A₃=O, R₁=CH₃, R₂=CH₃, R₄=H, R₅=CH2CH₃, 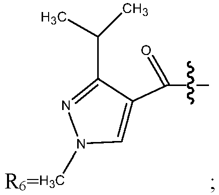
Compound 192: Q is selected from the structure of Q2, A₃=O, R₁=CH₂CH₂CH₃, R₂=CH₃, 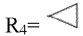 , R₅=CH2CH₃, 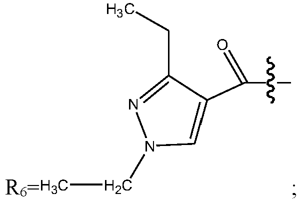
Compound 193: Q is selected from the structure of Q2, A₃=O, R₁=CH(CH₃)₂, R₂=CH₃, 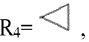 R₅=CH2CH₃, 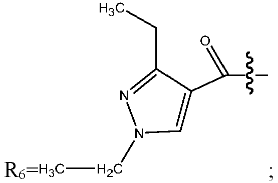
Compound 194: Q is selected from the structure of Q2, A₃=O, R₁=CH₃, R₂=CH₃, R₄=CH₃, R₅=CH₃, 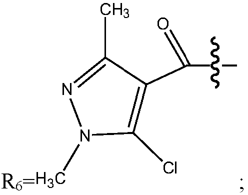
Compound 195: Q is selected from the structure of Q2, A₃=O, R₁=CH₂CH₃, R₂=CH₃, R₄=CH₃, R₅=CH₃, 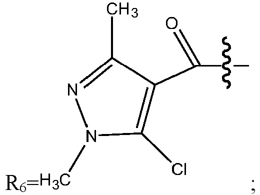
Compound 196: Q is selected from the structure of Q2, A₃=O, R₁=CH₂CH₂CH₃, R₂=CH₃, R₄=CH₃, R₅=CH₃, 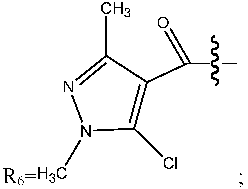
Compound 197: Q is selected from the structure of Q2, A₃=O, R₁=CH(CH₃)₂, R₂=CH₃, R₄=CH₃, R₅=CH₃, 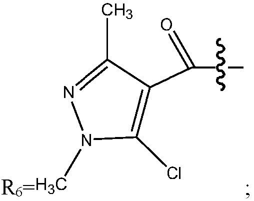
Compound 198: Q is selected from the structure of Q2, A₃=O, R₁=CH₃, R₂=CH₃, R₄=H, R₅=CH₃, 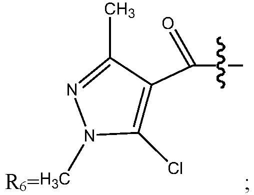
Compound 199: Q is selected from the structure of Q2, A₃=O, R₁=CH₂CH₃, R₂=CH₃, R₄=H, R₅=CH₃, 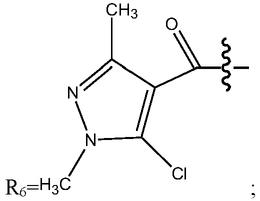
Compound 200: Q is selected from the structure of Q2, A₃=O, R₁=CH(CH₃)₂, R₂=CH₃, R₄=H, R₅=CH₃, 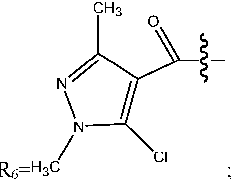
Compound 201: Q is selected from the structure of Q2, A₃=O, R₁=CH₃, R₂=CH₃, 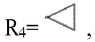 R₅=CH₃, 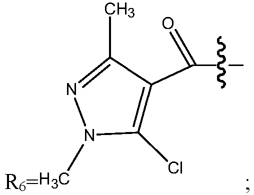
Compound 202: Q is selected from the structure of Q2, A₃=O, R₁=CH₂CH₃, R₂=CH₃, 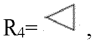 R₅=CH₃, 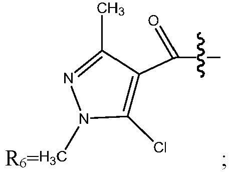
Compound 203: Q is selected from the structure of Q2, A₃=O, R₁=CH(CH₃)₂, R₂=CH₃, 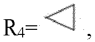 R₅=CH₃, 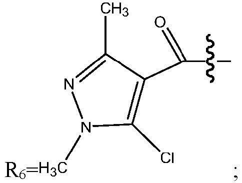
Compound 204: Q is selected from the structure of Q2, A₃=O, R₁=CH₃, R₂=CH₃, R₄=CH₃, R₅=CH₃, 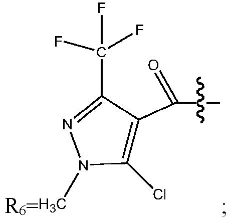
Compound 205: Q is selected from the structure of Q2, A₃=O, R₁=CH₂CH₃, R₂=CH₃, R₄=CH₃, R₅=CH₃. 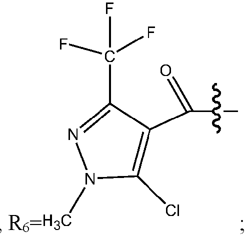
Compound 206: Q is selected from the structure of Q2, A₃=O, R₁=CH(CH₃)₂, R₂=CH₃, R₄=CH₃, R₅=CH₃, 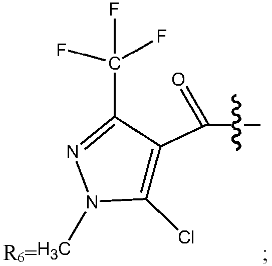
Compound 207: Q is selected from the structure of Q2, A₃=O, R₁=CH₃, R₂=CH₃, R₄=H, R₅=CH₃, 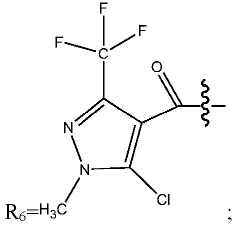
Compound 208: Q is selected from the structure of Q2, A₃=O, R₁=CH₂CH₃, R₂=CH₃, R₄=H, R₅=CH₃, 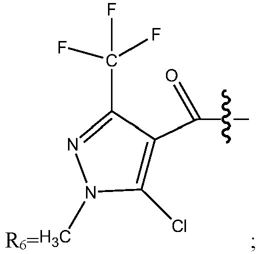
Compound 209: Q is selected from the structure of Q2, A₃=O, R₁=CH₂CH₂CH₃, R₂=CH₃, R₄=H, R₅=CH₃, 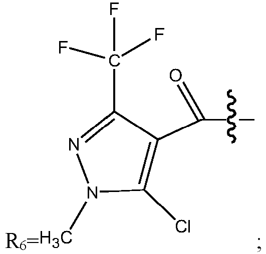
Compound 210: Q is selected from the structure of Q2, A₃=O, R₁=CH(CH₃)₂, R₂=CH₃, R₄=H, R₅=CH₃. 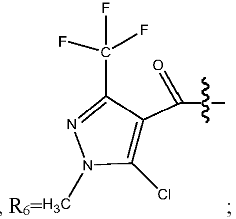
Compound 211: Q is selected from the structure of Q2, A₃=O, R₁=CH₂CH₃, R₂=CH₃, 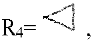 R₅=CH₃, 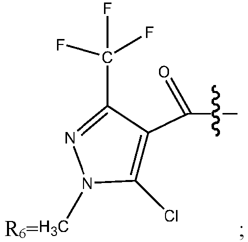
Compound 212: Q is selected from the structure of Q2, A₃=O, R₁=CH(CH₃)₂, R₂=CH₃, 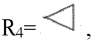 R₅=CH₃, 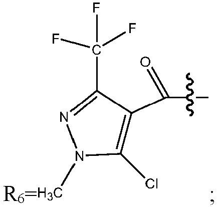
Compound 213: Q is selected from the structure of Q2, A₃=O, R₁=CH₃, R₂=CH₃, R₄=CH₃, R₅=CH₃, 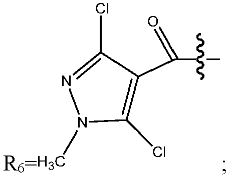
Compound 214: Q is selected from the structure of Q2, A₃=O, R₁=CH₂CH₃, R₂=CH₃, R₄=CH₃, R₅=CH₃. 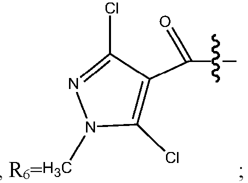
Compound 215: Q is selected from the structure of Q2, A₃=O, R₁=CH(CH₃)₂, R₂=CH₃, R₄=CH₃, R₅=CH₃, 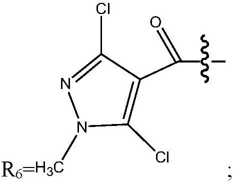
Compound 216: Q is selected from the structure of Q2, A₃=O, R₁=CH₃, R₂=CH₃, 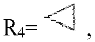 R₅=CH₃, 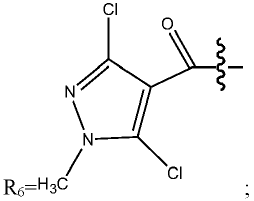
Compound 217: Q is selected from the structure of Q2, A₃=O, R₁=CH(CH₃)₂, R₂=CH₃, 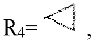 R₅=CH₃, 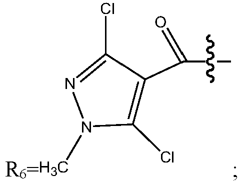
Compound 218: Q is selected from the structure of Q2, A₃=O, R₁=CH₃, R₂=CH₃, R₄=CH₃, R₅=CH₃, 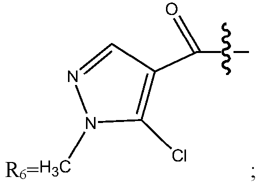
Compound 219: Q is selected from the structure of Q2, A₃=O, R₁=CH₂CH₃, R₂=CH₃, R₄=CH₃, R₅=CH₃, 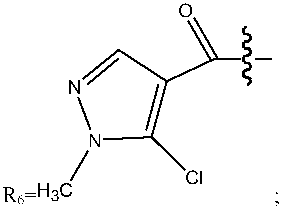
Compound 220: Q is selected from the structure of Q2, A₃=O, R₁=CH(CH₃)₂, R₂=CH₃, R₄=CH₃, R₅=CH₃, 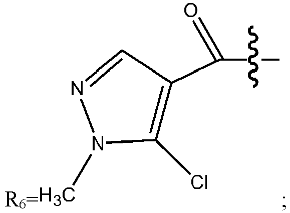
Compound 221: Q is selected from the structure of Q2, A₃=O, R₁=CH₃, R₂=CH₃, 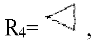 R₅=CH₃, 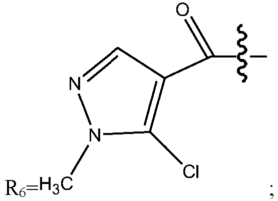
Compound 222: Q is selected from the structure of Q2, A₃=O, R₁=CH(CH₃)₂, R₂=CH₃, 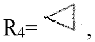 R₅=CH₃, 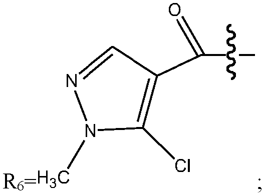
Compound 223: Q is selected from the structure of Q2, A₃=O, R₁=CH₃, R₂=CH₃, R₄=CH₃, R₅=CH₃, 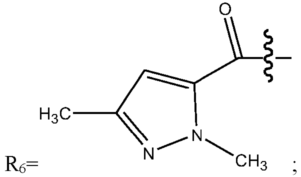
Compound 224: Q is selected from the structure of Q2, A₃=O, R₁=CH₂CH₃, R₂=CH₃, R₄=CH₃, R₅=CH₃, 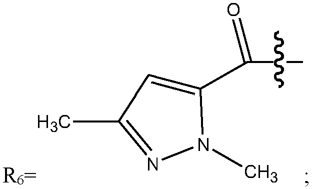
Compound 225: Q is selected from the structure of Q2, A₃=O, R₁=CH(CH₃)₂, R₂=CH₃, R₄=CH₃, R₅=CH₃, 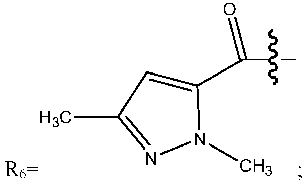
Compound 226: Q is selected from the structure of Q2, A₃=O, R₁=CH(CH₃)₂, R₂=CH₃, 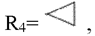 R₅=CH₃, 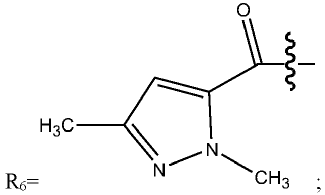
Compound 227: Q is selected from the structure of Q2, A₃=O, R₁=CH₃, R₂=CH₃, R₄=CH₃, R₅=CH₃, 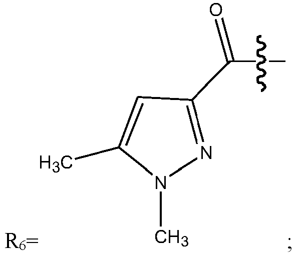
Compound 228: Q is selected from the structure of Q2, A₃=O, R₁=CH₂CH₃, R₂=CH₃, R₄=CH₃, R₅=CH₃, 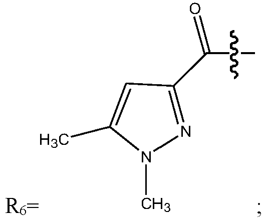
Compound 229: Q is selected from the structure of Q2, A₃=O, R₁=CH(CH₃)₂, R₂=CH₃, R₄=CH₃, R₅=CH₃, 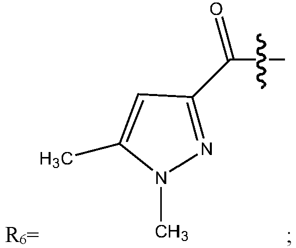
Compound 230: Q is selected from the structure of Q2, A₃=O, R₁=CH(CH₃)₂, R₂=CH₃, 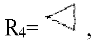 R₅=CH₃, 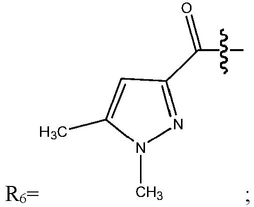
Compound 231: Q is selected from the structure of Q2, A₃=O, R₁=CH₃, R₂=CH₃, R₄=CH₃, R₅=CH₃. 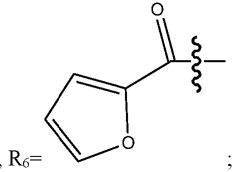
Compound 232: Q is selected from the structure of Q2, A₃=S, R₁=CH₃, R₂=CH₃, R₄=CH₃, R₅=CH₃, 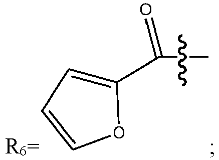
Compound 233: Q is selected from the structure of Q2, A₃=O, R₁=CH₂CH₃, R₂=CH₃, R₄=CH₃, R₅=CH₃, 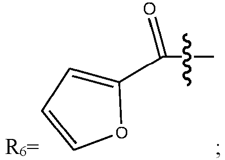
Compound 234: Q is selected from the structure of Q2, A₃=O, R₁=CH(CH₃)₂, R₂=CH₃, R₄=CH₃, R₅=CH₃, 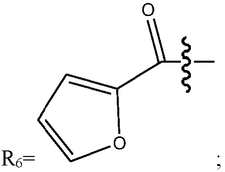
Compound 235: Q is selected from the structure of Q2, A₃=O, R₁=CH₃, R₂=CH₃, 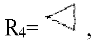 R₅=CH₃, 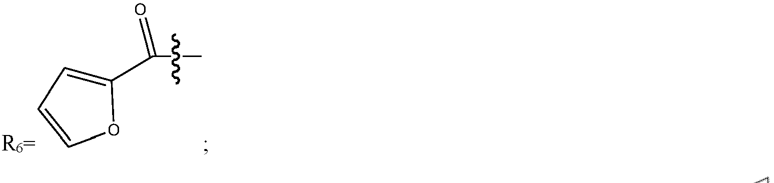
Compound 236: Q is selected from the structure of Q2, A₃=O, R₁=CH₂CH₃, R₂=CH₃, 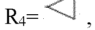 R₅=CH₃, 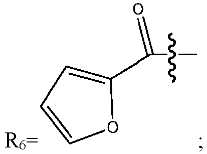
Compound 237: Q is selected from the structure of Q2, A₃=O, R₁=CH(CH₃)₂, R₂=CH₃, 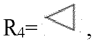 R₅=CH₃, 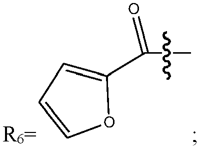
Compound 238: Q is selected from the structure of Q2, A₃=O, R₁=CH₃, R₂=CH₃, R₄=CH₃, R₅=CH₃, 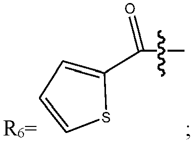
Compound 239: Q is selected from the structure of Q2, A₃=O, R₁=CH₂CH₃, R₂=CH₃, R₄=CH₃, R₅=CH₃, 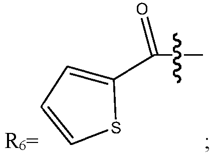
Compound 240: Q is selected from the structure of Q2, A₃=O, R₁=CH(CH₃)₂, R₂=CH₃, R₄=CH₃, R₅=CH₃, 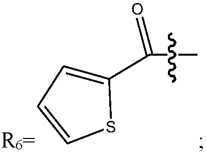
Compound 241: Q is selected from the structure of Q2, A₃=O, R₁=CH₃, R₂=CH₃, 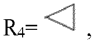 R₅=CH₃, 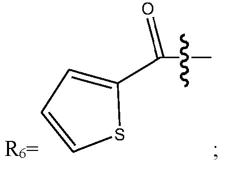
Compound 242: Q is selected from the structure of Q2, A₃=O, R₁=CH₂CH₃, R₂=CH₃, 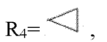 R₅=CH₃, 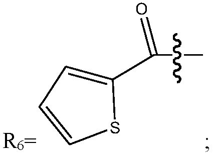
Compound 243: Q is selected from the structure of Q2, A₃=O, R₁=CH(CH₃)₂, R₂=CH₃, 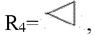 R₅=CH₃ 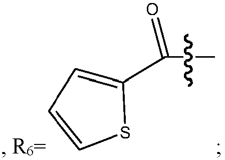
Compound 244: Q is selected from the structure of Q2, A₃=O, R₁=CH₃, R₂=CH₃, R₄=CH₃, R₅=CH₃. 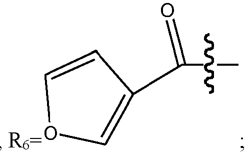
Compound 245: Q is selected from the structure of Q2, A₃=S, R₁=CH₃, R₂=CH₃, R₄=CH₃, R₅=CH₃, 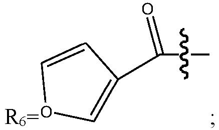
Compound 246: Q is selected from the structure of Q2, A₃=O, R₁=CH(CH₃)₂, R₂=CH₃, R₄=CH₃, R₅=CH₃, 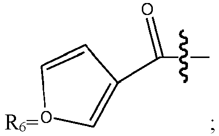
Compound 247: Q is selected from the structure of Q2, A₃=O, R₁=CH₃, R₂=CH₃, 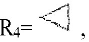 R₅=CH₃, 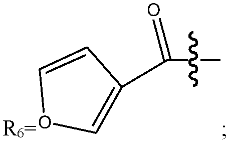
Compound 248: Q is selected from the structure of Q2, A₃=O, R₁=CH₂CH₃, R₂=CH₃, 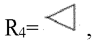 R₅=CH₃ 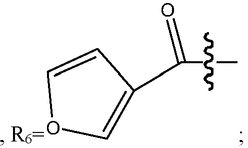
Compound 249: Q is selected from the structure of Q2, A₃=O, R₁=CH(CH₃)₂, R₂=CH₃, 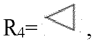 R₅=CH₃, 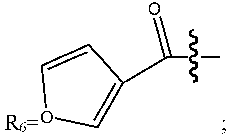
Compound 250: Q is selected from the structure of Q2, A₃=O, R₁=CH₃, R₂=CH₃, R₄=CH₃, R₅=CH₃ 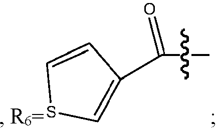
Compound 251: Q is selected from the structure of Q2, A₃=O, R₁=CH(CH₃)₂, R₂=CH₃, R₄=CH₃, R₅=CH₃. 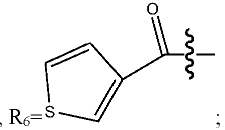
Compound 252: Q is selected from the structure of Q2, A₃=O, R₁=CH₃, R₂=CH₃, 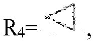 R₅=CH₃, 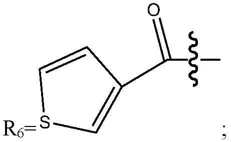
Compound 253: Q is selected from the structure of Q2, A₃=O, R₁=CH₂CH₃, R₂=CH₃, 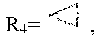 R₅=CH₃, 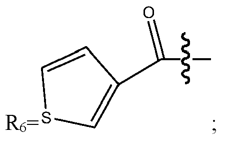
Compound 254: Q is selected from the structure of Q2, A₃=O, R₁=CH(CH₃)₂, R₂=CH₃, 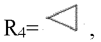 R₅=CH₃, 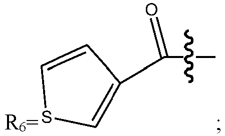
Compound 255: Q is selected from the structure of Q2, A₃=O, R₁=CH₃, R₂=CH₃, R₄=CH₃, R₅=CH₃, 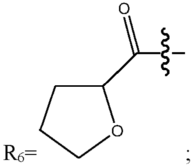
Compound 256: Q is selected from the structure of Q2, A₃=S, R₁=CH₃, R₂=CH₃, R₄=CH₃, R₅=CH₃, 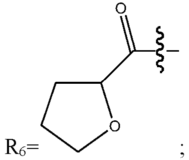
Compound 257: Q is selected from the structure of Q2, A₃=O, R₁=CH(CH₃)₂, R₂=CH₃, R₄=CH₃, R₅=CH₃, 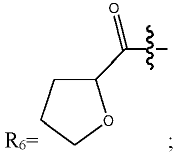
Compound 258: Q is selected from the structure of Q2, A₃=O, R₁=CH₃, R₂=CH₃, R₅=CH₃, 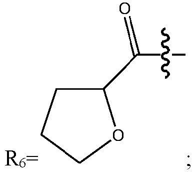 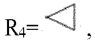
Compound 259: Q is selected from the structure of Q2, A₃=O, R₁=CH₂CH₃, R₂=CH₃, 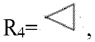 R₅=CH₃, 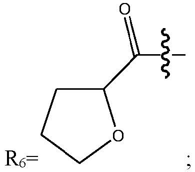
Compound 260: Q is selected from the structure of Q2, A₃=O, R₁=CH(CH₃)₂, R₂=CH₃, 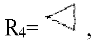 R₅=CH₃,

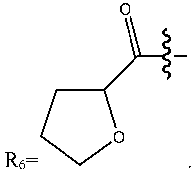

The compounds of Formula (I) or tautomers thereof of the present application have herbicidal activity, which can be used for controlling various weeds in agriculture.

The present application also provides the use of the oxadiazolone compound or tautomer thereof according to the above technical solutions for controlling weeds.

In some embodiments, the weed is one or more selected from the group consisting of broadleaf weeds and gramineous weeds.

In some embodiments, the weed is one or more selected from the group consisting of abutilon, *Solanum nigrum,* crabgrass, and barnyard grass.

The present application also provides a herbicidal composition comprising the oxadiazolone compound or tautomer thereof according to the above technical solutions and an excipient. The present application has no special limitations on the selection of the excipient, and the corresponding excipient may be selected according to the desired dosage form. The compound of Formula (I) or tautomer thereof as an active component may be used in a percentage by weight of 0.1 to 99%, preferably 0.5 to 50%.

The herbicidal composition of the present application may be applied in a variety of formulations. The compounds of the present application are typically formulated by dissolving or dispersing in a carrier so as to be more readily dispersed when used as herbicides. For example, these chemicals can be made into wettable powder or missible oil. Thus, in these compositions, at least one liquid or solid carrier is added, and an appropriate surfactant is usually required.

The present application also provides a method for controlling weeds, comprising applying the herbicidal composition according to the above technical solutions to crops.

Specifically, the present application applies a herbicide with an effective amount of the herbicidal composition of the present application to the surface of the weed or a site where the weed grows or the surface of a growth medium thereof. A more suitable effective dose is 1 gram to 1000 gram per hectare, preferably 10 to 500 gram per hectare. For certain applications, one or more additional herbicides may be added to the herbicidal composition of the present application, thereby producing additional advantages and effects.

### Beneficial effects

In the present application, an oxadiazolone group of Formula (a) is provided as a substituting group on the position between the sulfonyl group and Cl on the benzene ring of a benzoyl compound to obtain an oxadiazolone compound, which not only has unexpectedly high herbicidal activity against various weeds, but also is safe for various crops such as corn and rice. The experimental results show that the compound of the present application exhibits good herbicidal activity and can effectively control weeds (such as barnyard grass, crabgrass, and abutilon) at a low dose to achieve an excellent herbicidal effect, in the meanwhile, it demonstrates higher safety for crops. 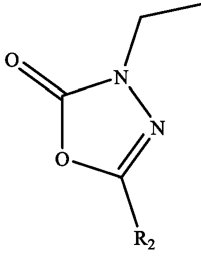 the group of Formula (a)

### Detailed Description of the Invention

In order to make the purpose, technical solutions and advantages of the present application clearer, the technical solutions in the embodiments of the present application will be described clearly and completely. Obviously, the described embodiments are some of the embodiments of the present application, but not all of them. Based on the embodiments of the present application, other embodiments obtained by those of ordinary skill in the art without creative work are all within the scope of the present invention.

In addition, in order to better explain the present invention, a lot of specific details are given in the following embodiments. It will be understood by those skilled in the art that the present invention may be practiced without certain specific details. In some embodiments, materials, elements, methods, means, etc., well known to those skilled in the art, are not described in detail so as to highlight the spirit of the present application.

In this application, the terms used are generally defined as follows:
Halogen refers to fluorine, chlorine, bromine, or iodine.

Alkyl refers to linear or branched alkyl, such as methyl, ethyl, propyl, isopropyl, n-butyl, or tert-butyl.

Cycloalkyl refers to substituted or unsubstituted cyclic alkyl, such as cyclopropyl, cyclopentyl, or cyclohexyl, wherein the substituent may be methyl, halogen, etc.

Haloalkyl refers to linear or branched alkyl in which the hydrogen atoms may be partially or completely substituted with a halogen atom, such as chloromethyl, dichloromethyl, trichloromethyl, fluoromethyl, difluoromethyl, or trifluoromethyl.

Alkoxy refers to a group in which linear or branched alkyl is attached to a structure via an oxygen atom.

Haloalkoxy refers to linear or branched alkoxy in which the hydrogen atoms may be partially or completely substituted with a halogen atom, such as chloromethoxy, dichloromethoxy, trichloromethoxy, fluoromethoxy, difluoromethoxy, or trifluoromethoxy.

Alkylamino refers to a group in which linear or branched alkyl is attached to a structure via a nitrogen atom.

Haloalkylamino refers to linear or branched alkylamino in which the hydrogen atoms may be partially or completely substituted with a halogen atom.

Cycloalkylamino refers to cyclic alkylamino attached to a structure via a nitrogen atom, for example, cyclopentylamino or cyclohexylamino.

Heterocycle refers to a cyclic hydrocarbon containing a heteroatom such as N, O, or S in the ring, for example, furan, thiophene, morpholine, hexamethyleneimine, pyrazole or piperidine, and the like.

Heterocycloamino refers to a group in which a heterocycle is attached to a structure via a nitrogen atom.

Tautomer refers to a structural isomer with different energies that can be converted to each other through low energy barriers. If a tautomer is possible (such as in a solution), the chemical equilibrium of tautomers can be achieved. For example, proton tautomers include tautomers that are transformed to each other by proton migration, such as keto-enol isomerization and imine-enamine isomerization. In the present application, a keto-enol interconversion exists depending on extrinsic conditions such as solvent, PH, etc.

Some of the compounds of the present application may be illustrated by the specific compounds listed in Tables 1, 2, and 3, but the present application is not limited to these compounds.

**Table 1 Compounds 1-30**

| 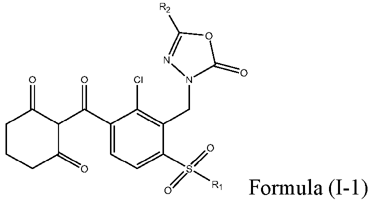 | | |
|---|---|---|
| Compound | R₁ | R₂ |
| 1 | CH₃ | CH₃ |
| 2 | CH₃ | CH₂CH₃ |
| 3 | CH₃ | CH₂CH₂CH₃ |
| 4 | CH₃ | C(CH₃)₃ |
| 5 | CH₃ | CF₃ |
| 6 | CH₂CH₃ | CH₃ |
| 7 | CH₂CH₃ | CH₂CH₃ |
| 8 | CH₂CH₃ | CH₂CH₂CH₃ |
| 9 | CH₂CH₃ | C(CH₃)₃ |
| 10 | CH₂CH₃ | CF₃ |
| 11 | CH₂CH₂CH₃ | CH₃ |
| 12 | CH₂CH₂CH₃ | CH₂CH₃ |
| 13 | CH₂CH₂CH₃ | CH₂CH₂CH₃ |
| 14 | CH₂CH₂CH₃ | C(CH₃)₃ |
| 15 | CH₂CH₂CH₃ | CF₃ |
| 16 | CH(CH₃)₂ | CH₃ |
| 17 | CH(CH₃)₂ | CH₂CH₃ |
| 18 | CH(CH₃)₂ | CH₂CH₂CH₃ |
| 19 | CH(CH₃)₂ | C(CH₃)₃ |
| 20 | CH(CH₃)₂ | CF₃ |
| 21 | CH₂CH₂CH₂CH₃ | CH₃ |
| 22 | CH₂CH₂CH₂CH₃ | CH₂CH₃ |
| 23 | CH₂CH₂CH₂CH₃ | CH₂CH₂CH₃ |
| 24 | CH₂CH₂CH₂CH₃ | C(CH₃)₃ |
| 25 | CH₂CH₂CH₂CH₃ | CF₃ |
| 26 | C(CH₃)₃ | CH₃ |
| 27 | C(CH₃)₃ | CH₂CH₃ |
| 28 | C(CH₃)₃ | CH₂CH₂CH₃ |
| 29 | C(CH₃)₃ | C(CH₃)₃ |
| 30 | C(CH₃)₃ | CF₃ |

**Table 2 Compounds 31-55**

| | | | | |
|---|---|---|---|---|
| 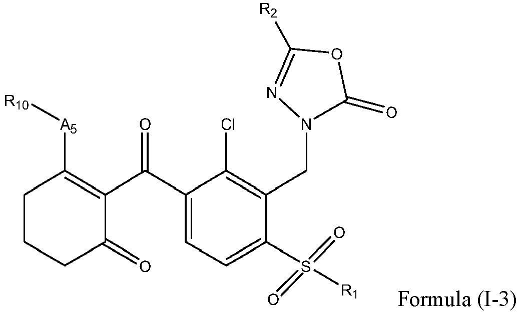 | | | | |

| Compound | R₁ | R₂ | A₅ | R₁₀ |
|---|---|---|---|---|
| 31 | CH₃ | CH₃ | O | CH₃ |
| 32 | CH₃ | CH₃ | O | CH₂CH₃ |
| 33 | CH₃ | CH₃ | O | CH₂CH₂CH₃ |
| 34 | CH₃ | CH₃ | O | CH(CH₃)₂ |
| 35 | CH₃ | CH₃ | O | CH₂CH₂CH₂CH₃ |
| 36 | CH₃ | CH₃ | O | C(CH₃)₃ |
| 37 | CH₃ | CH₃ | O | 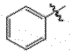 |
| 38 | CH₃ | CH₃ | O | 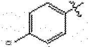 |
| 39 | CH₃ | CH₃ | O | 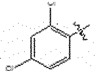 |
| 40 | CH₃ | CH₃ | O | 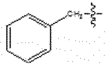 |
| 41 | CH₃ | CH₃ | O | 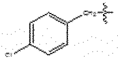 |
| 42 | CH₃ | CH₃ | O | 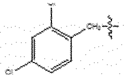 |
| 43 | CH₃ | CH₃ | O | 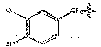 |
| 44 | CH₃ | CH₃ | S | CH₃ |
| 45 | CH₃ | CH₃ | S | CH₂CH₃ |
| 46 | CH₃ | CH₃ | S | CH₂CH₂CH₃ |
| 47 | CH₃ | CH₃ | S | CH(CH₃)₂ |
| 48 | CH₃ | CH₃ | S | CH₂CH₂CH₂CH₃ |
| 49 | CH₃ | CH₃ | S | C(CH₃)₃ |
| 50 | CH₃ | CH₃ | S | 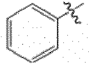 |
| 51 | CH₃ | CH₃ | S | 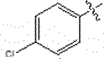 |
| 52 | CH₃ | CH₃ | S | 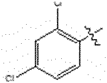 |
| 53 | CH₃ | CH₃ | S | 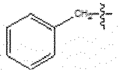 |
| 54 | CH₃ | CH₃ | S | 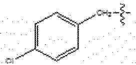 |
| 55 | CH₃ | CH₃ | S | 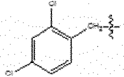 |

**Table 3 Compounds 56-260**

| 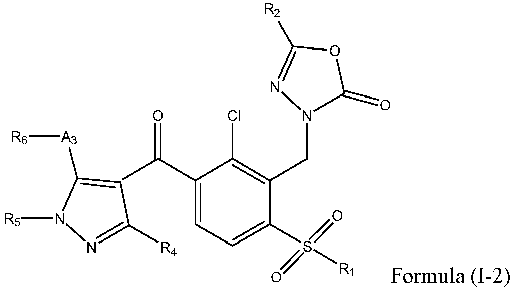 | | | | | | |
|---|---|---|---|---|---|---|
| Compound | R₁ | R₂ | A₃ | R₄ | R₅ | R₆ |
| 56 | CH₃ | CH₃ | O | H | CH₃ | H |
| 57 | CH₃ | CH₃ | O | H | CH₂CH₃ | H |
| 58 | CH₃ | CH₃ | O | CH₃ | CH₃ | H |
| 59 | CH₃ | CH₃ | O | CH₃ | CH₂CH₃ | H |
| 60 | CH₃ | CH₃ | O | CH₂CH₃ | CH₃ | H |
| 61 | CH₃ | CH₃ | O | CH₂CH₃ | CH₂CH₃ | H |
| 62 | CH₃ | CH₃ | O | CH₂CH₂CH₃ | CH₃ | H |
| 63 | CH₃ | CH₃ | O | CH₂CH₂CH₃ | CH₂CH₃ | H |
| 64 | CH₃ | CH₃ | O | CH(CH₃)₂ | CH₃ | H |
| 65 | CH₃ | CH₃ | O | CH(CH₃)₂ | CH₂CH₃ | H |
| 66 | CH₃ | CH₃ | O | CF₃ | CH₃ | H |
| 67 | CH₃ | CH₃ | O | CF₃ | CH₂CH₃ | H |
| 68 | CH₃ | CH₃ | O | 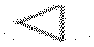 | CH₃ | H |
| 69 | CH₃ | CH₃ | O | 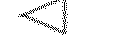 | CH₂CH₃ | H |
| 70 | CH₂CH₃ | CH₃ | O | H | CH₃ | H |
| 71 | CH₂CH₃ | CH₃ | O | H | CH₂CH₃ | H |
| 72 | CH₂CH₃ | CH₃ | O | CH₃ | CH₃ | H |
| 73 | CH₂CH₃ | CH3 | O | CH₃ | CH₂CH₃ | H |
| 74 | CH₂CH₃ | CH₃ | O | CH₂CH₃ | CH₃ | H |
| 75 | CH₂CH₃ | CH₃ | O | CH₂CH₃ | CH₂CH₃ | H |
| 76 | CH₂CH₃ | CH₃ | O | CH₂CH₂CH₃ | CH₃ | H |
| 77 | CH₂CH₃ | CH₃ | O | CH₂CH₂CH₃ | CH₂CH₃ | H |
| 78 | CH₂CH₃ | CH₃ | O | CH(CH₃)₂ | CH₃ | H |
| 79 | CH₂CH₃ | CH₃ | O | CH(CH₃)₂ | CH₂CH₃ | H |
| 80 | CH₂CH₃ | CH₃ | O | CF₃ | CH₃ | H |
| 81 | CH₂CH₃ | CH₃ | O | CF₃ | CH₂CH₃ | H |
| 82 | CH₂CH₃ | CH₃ | O | 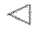 | CH₃ | H |
| 83 | CH₂CH₃ | CH₃ | O | 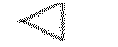 | CH₂CH₃ | H |
| 84 | CH₂CH₂CH₃ | CH₃ | O | H | CH₃ | H |
| 85 | CH₂CH₂CH₃ | CH₃ | O | H | CH₂CH₃ | H |
| 86 | CH₂CH₂CH₃ | CH3 | O | CH₃ | CH₃ | H |
| 87 | CH₂CH₂CH₃ | CH₃ | O | CH₃ | CH₂CH₃ | H |
| 88 | CH₂CH₂CH₃ | CH₃ | O | CH₂CH₃ | CH₃ | H |
| 89 | CH₂CH₂CH₃ | CH₃ | O | CH₂CH₃ | CH₂CH₃ | H |
| 90 | CH₂CH₂CH₃ | CH₃ | O | CH₂CH₂CH₃ | CH₃ | H |
| 91 | CH₂CH₂CH₃ | CH₃ | O | CH₂CH₂CH₃ | CH₂CH₃ | H |
| 92 | CH₂CH₂CH₃ | CH₃ | O | CH(CH₃)₂ | CH₃ | H |
| 93 | CH₂CH₂CH₃ | CH₃ | O | CH(CH₃)₂ | CH₂CH₃ | H |
| 94 | CH₂CH₂CH₃ | CH₃ | O | CF₃ | CH₃ | H |
| 95 | CH₂CH₂CH₃ | CH₃ | O | CF₃ | CH₂CH₃ | H |
| 96 | CH₂CH₂CH₃ | CH₃ | O | 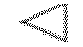 | CH₃ | H |
| 97 | CH₂CH₂CH₃ | CH₃ | O | 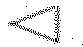 | CH₂CH₃ | H |
| 98 | CH(CH₃)₂ | CH₃ | O | H | CH₃ | H |
| 99 | CH(CH₃)₂ | CH₃ | O | H | CH₂CH₃ | H |
| 100 | CH(CH₃)₂ | CH₃ | O | CH₃ | CH₃ | H |
| 101 | CH(CH₃)₂ | CH₃ | O | CH₃ | CH₂CH₃ | H |
| 102 | CH(CH₃)₂ | CH₃ | O | CH₂CH₃ | CH₃ | H |
| 103 | CH(CH₃)₂ | CH₃ | O | CH₂CH₃ | CH₂CH₃ | H |
| 104 | CH(CH₃)₂ | CH₃ | O | CH₂CH₂CH₃ | CH₃ | H |
| 105 | CH(CH₃)₂ | CH₃ | O | CH₂CH₂CH₃ | CH₂CH₃ | H |
| 106 | CH(CH₃)₂ | CH₃ | O | CH(CH₃)₂ | CH₃ | H |
| 107 | CH(CH₃)₂ | CH₃ | O | CH(CH₃)₂ | CH₂CH₃ | H |
| 108 | CH(CH₃)₂ | CH₃ | O | CF₃ | CH₃ | H |
| 109 | CH(CH₃)₂ | CH₃ | O | CF₃ | CH₂CH₃ | H |
| 110 | CH(CH₃)₂ | CH₃ | O | 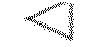 | CH₃ | H |
| 111 | CH(CH₃)₂ | CH₃ | O | 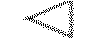 | CH₂CH₃ | H |
| 112 | CH₂CH₂CH₂CH₃ | CH₃ | O | H | CH₃ | H |
| 113 | CH₂CH₂CH₂CH₃ | CH₃ | O | H | CH₂CH₃ | H |
| 114 | CH₂CH₂CH₂CH₃ | CH₃ | O | CH₃ | CH₃ | H |
| 115 | CH₂CH₂CH₂CH₃ | CH₃ | O | CH₃ | CH₂CH₃ | H |
| 116 | CH₂CH₂CH₂CH₃ | CH₃ | O | CH₂CH₃ | CH₃ | H |
| 117 | CH₂CH₂CH₂CH₃ | CH₃ | O | CH₂CH₃ | CH₂CH₃ | H |
| 118 | CH₂CH₂CH₂CH₃ | CH₃ | O | CH₂CH₂CH₃ | CH₃ | H |
| 119 | CH₂CH₂CH₂CH₃ | CH₃ | O | CH₂CH₂CH₃ | CH₂CH₃ | H |
| 120 | CH₂CH₂CH₂CH₃ | CH₃ | O | CH(CH3)₂ | CH₃ | H |
| 121 | CH₂CH₂CH₂CH₃ | CH₃ | O | CH(CH₃)₂ | CH₂CH₃ | H |
| 122 | CH₂CH₂CH₂CH₃ | CH₃ | O | CF₃ | CH₃ | H |
| 123 | CH₂CH₂CH₂CH₃ | CH₃ | O | CF₃ | CH₂CH₃ | H |
| 124 | CH₂CH₂CH₂CH₃ | CH₃ | O | 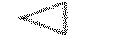 | CH₃ | H |
| 125 | CH₂CH₂CH₂CH₃ | CH₃ | O | 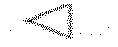 | CH₂CH₃ | H |
| 126 | C(CH₃)₃ | CH₃ | O | H | CH₃ | H |
| 127 | C(CH₃)₃ | CH₃ | O | H | CH₂CH₃ | H |
| 128 | C(CH₃)₃ | CH₃ | O | CH₃ | CH₃ | H |
| 129 | C(CH₃)₃ | CH₃ | O | CH₃ | CH₂CH₃ | H |
| 130 | C(CH₃)₃ | CH₃ | O | CH₂CH₃ | CH₃ | H |
| 131 | C(CH₃)₃ | CH₃ | O | CH₂CH₃ | CH₂CH₃ | H |
| 132 | C(CH₃)₃ | CH₃ | O | CH₂CH₂CH₃ | CH₃ | H |
| 133 | C(CH₃)₃ | CH₃ | O | CH₂CH₂CH₃ | CH₂CH₃ | H |
| 134 | C(CH₃)₃ | CH₃ | O | CH(CH₃)₂ | CH₃ | H |
| 135 | C(CH₃)₃ | CH₃ | O | CH(CH₃)₂ | CH₂CH₃ | H |
| 136 | C(CH₃)₃ | CH₃ | O | CF₃ | CH₃ | H |
| 137 | C(CH₃)₃ | CH₃ | O | CF₃ | CH₂CH₃ | H |
| 138 | C(CH₃)₃ | CH₃ | O | 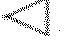 | CH₃ | H |
| 139 | C(CH₃)₃ | CH₃ | O | 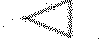 | CH₂CH₃ | H |
| 140 | CH₃ | CH₃ | S | CH₃ | CH₃ | (CH₃CH₂)₂NCO |
| 141 | CH₃ | CH₃ | O | CH₃ | CH₃ | (CH₃CH₂CH₂)₂NCO |
| 142 | CH₂CH₃ | CH₃ | O | CH₃ | CH₃ | (CH₃CH₂CH₂)₂NCO |
| 143 | CH(CH₃)₂ | CH₃ | O | CH₃ | CH₃ | (CH₃CH₂CH₂)₂NCO |
| 144 | CH₃ | CH₃ | O | 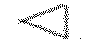 | CH₃ | (CH₃CH₂CH₂)₂NCO |
| 145 | CH₃ | CH₃ | S | CH₃ | CH₃ | (CH₃OCH₂CH₂)₂NCO |
| 146 | CH₃ | CH₃ | S | CH₃ | CH₃ | 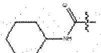 |
| 147 | CH₃ | CH₃ | S | CH₃ | CH₃ | 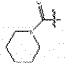 |
| 148 | CH₃ | CH₃ | S | CH₃ | CH₃ | 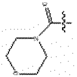 |
| 149 | CH₃ | CH₃ | O | CH₃ | CH₃ | 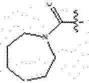 |
| 150 | CH₂CH₃ | CH₃ | O | CH₃ | CH₃ | 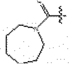 |
| 151 | CH(CH₃)₂ | CH₃ | O | CH₃ | CH₃ | 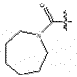 |
| 152 | CH₃ | CH₃ | O | 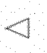 | CH₃ | 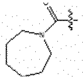 |
| 153 | CH₂CH₃ | CH₃ | O | 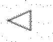 | CH₃ | 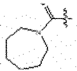 |
| 154 | CH₃ | CH₃ | O | CH₃ | CH₃ | 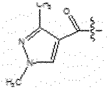 |
| 155 | CH₂CH₃ | CH₃ | O | CH₃ | CH₃ | 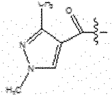 |
| 156 | CH₂CH₂CH₃ | CH₃ | O | CH₃ | CH₃ | 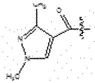 |
| 157 | CH(CH₃)₂ | CH₃ | O | CH₃ | CH₃ | 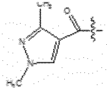 |
| 158 | CH₃ | CH₃ | O | H | CH₃ | 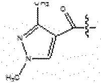 |
| 159 | CH₂CH₃ | CH₃ | O | H | CH₃ | 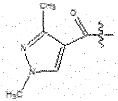 |
| 160 | CH₂CH₂CH₃ | CH₃ | O | H | CH₃ | 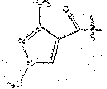 |
| 161 | CH(CH₃)₂ | CH₃ | O | H | CH₃ | 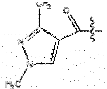 |
| 162 | CH₃ | CH₃ | O | 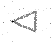 | CH₃ | 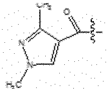 |
| 163 | CH₂CH₃ | CH₃ | O | 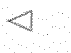 | CH₃ | 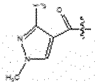 |
| 164 | CH₂CH₂CH₃ | CH₃ | O | 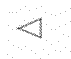 | CH₃ | 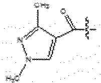 |
| 165 | CH(CH₃)₂ | CH₃ | O | 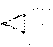 | CH₃ | 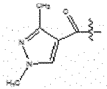 |
| 166 | CH₃ | CH₃ | O | CH₃ | CH₃ | 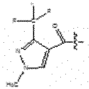 |
| 167 | CH₂CH₃ | CH₃ | O | CH₃ | CH₃ | 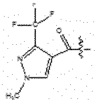 |
| 168 | CH₂CH₂CH₃ | CH₃ | O | CH₃ | CH₃ | 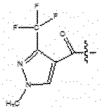 |
| 169 | CH(CH₃)₂ | CH₃ | O | CH₃ | CH₃ | 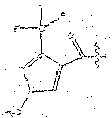 |
| 170 | CH₃ | CH₃ | O | H | CH₃ | 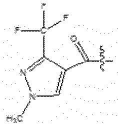 |
| 171 | CH₂CH₃ | CH₃ | O | H | CH₃ | 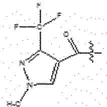 |
| 172 | CH₂CH₂CH₃ | CH₃ | O | H | CH₃ | 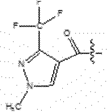 |
| 173 | CH(CH₃)₂ | CH₃ | O | H | CH₃ | 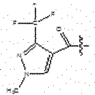 |
| 174 | CH₃ | CH₃ | O | 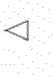 | CH₃ | 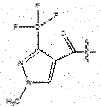 |
| 175 | CH₂CH₃ | CH₃ | O | 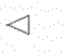 | CH₃ | 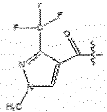 |
| 176 | CH₂CH₂CH₃ | CH₃ | O | 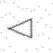 | CH₃ | 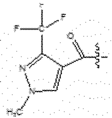 |
| 177 | CH(CH₃)₂ | CH₃ | O | 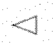 | CH₃ | 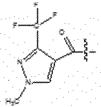 |
| 178 | CH₃ | CH₃ | O | CH₃ | CH₃ | 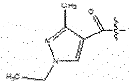 |
| 179 | CH₂CH₂CH₃ | CH₃ | O | CH₃ | CH₃ | 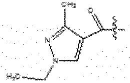 |
| 180 | CH₃ | CH₃ | O | H | CH₃ | 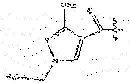 |
| 181 | CH₂CH₂CH₃ | CH₃ | O | H | CH₃ | 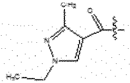 |
| 182 | CH₃ | CH₃ | O | 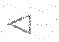 | CH₃ | 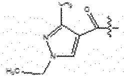 |
| 183 | CH₂CH₂CH₃ | CH₃ | O | 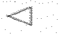 | CH₃ | 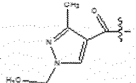 |
| 184 | CH₂CH₃ | CH₃ | O | CH₃ | CH₃ | 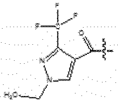 |
| 185 | CH(CH₃)₂ | CH₃ | O | CH₃ | CH₃ | 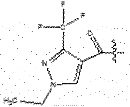 |
| 186 | CH₂CH₃ | CH₃ | O | H | CH₃ | 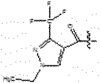 |
| 187 | CH(CH₃)₂ | CH₃ | O | H | CH₃ | 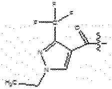 |
| 188 | CH₂CH₃ | CH₃ | O | 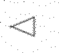 | CH₃ | 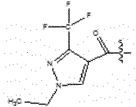 |
| 189 | CH(CH₃)₂ | CH₃ | O | 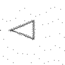 | CH₃ | 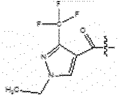 |
| 190 | CH₂CH₃ | CH₃ | O | CH₃ | CH₂CH₃ | 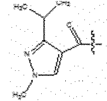 |
| 191 | CH₃ | CH₃ | O | H | CH₂CH₃ | 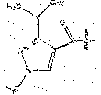 |
| 192 | CH₂CH₂CH₃ | CH₃ | O | 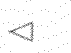 | CH₂CH₃ | 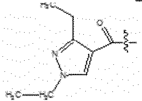 |
| 193 | CH(CH₃)₂ | CH₃ | O | 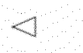 | CH₂CH₃ | 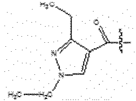 |
| 194 | CH₃ | CH₃ | O | CH₃ | CH₃ | 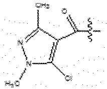 |
| 195 | CH₂CH₃ | CH₃ | O | CH3 | CH3 | 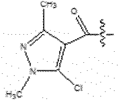 |
| 196 | CH₂CH₂CH₃ | CH₃ | O | CH3 | CH₃ | 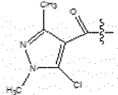 |
| 197 | CH(CH₃)₂ | CH₃ | O | CH₃ | CH₃ | 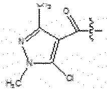 |
| 198 | CH₃ | CH₃ | O | H | CH₃ | 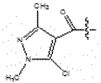 |
| 199 | CH₂CH₃ | CH₃ | O | H | CH₃ | 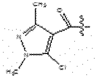 |
| 200 | CH(CH₃)₂ | CH3 | O | H | CH3 | 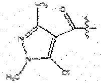 |
| 201 | CH₃ | CH₃ | O | 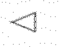 | CH₃ | 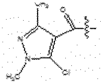 |
| 202 | CH₂CH₃ | CH₃ | O | 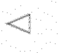 | CH₃ | 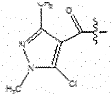 |
| 203 | CH(CH₃)₂ | CH₃ | O | 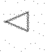 | CH₃ | 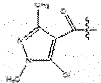 |
| 204 | CH₃ | CH₃ | O | CH₃ | CH₃ | 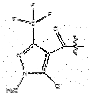 |
| 205 | CH₂CH₃ | CH₃ | O | CH₃ | CH₃ | 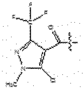 |
| 206 | CH(CH₃)₂ | CH₃ | O | CH₃ | CH₃ | 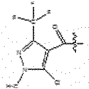 |
| 207 | CH₃ | CH₃ | O | H | CH₃ | 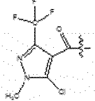 |
| 208 | CH₂CH₃ | CH3 | O | H | CH₃ | 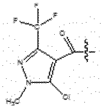 |
| 209 | CH₂CH₂CH₃ | CH₃ | O | H | CH₃ | 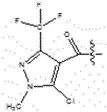 |
| 210 | CH(CH₃)₂ | CH₃ | O | H | CH₃ | 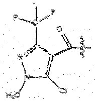 |
| 211 | CH₂CH₃ | CH₃ | O | 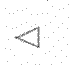 | CH₃ | 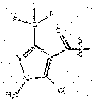 |
| 212 | CH(CH₃)₂ | CH₃ | O | 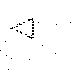 | CH3 | 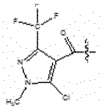 |
| 213 | CH₃ | CH₃ | O | CH₃ | CH3 | 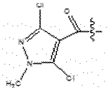 |
| 214 | CH₂CH₃ | CH₃ | O | CH₃ | CH₃ | 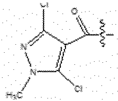 |
| 215 | CH(CH₃)₂ | CH₃ | O | CH₃ | CH₃ | 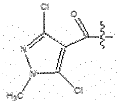 |
| 216 | CH₃ | CH₃ | O | 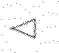 | CH3 | 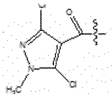 |
| 217 | CH(CH₃)₂ | CH₃ | O | 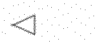 | CH₃ | 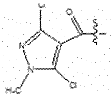 |
| 218 | CH₃ | CH₃ | O | CH₃ | CH₃ | 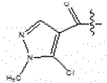 |
| 219 | CH₂CH₃ | CH₃ | O | CH₃ | CH₃ | 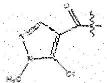 |
| 220 | CH(CH₃)₂ | CH₃ | O | CH₃ | CH₃ | 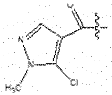 |
| 221 | CH₃ | CH₃ | O | 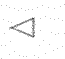 | CH₃ | 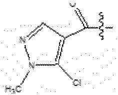 |
| 222 | CH(CH₃)₂ | CH₃ | O | 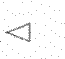 | CH₃ | 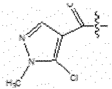 |
| 223 | CH₃ | CH₃ | O | CH3 | CH₃ | 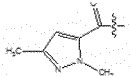 |
| 224 | CH₂CH₃ | CH₃ | O | CH₃ | CH₃ | 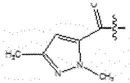 |
| 225 | CH(CH₃)₂ | CH₃ | O | CH₃ | CH₃ | 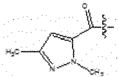 |
| 226 | CH(CH₃)₂ | CH₃ | O | 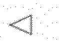 | CH₃ | 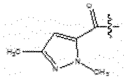 |
| 227 | CH₃ | CH₃ | O | CH₃ | CH₃ | 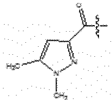 |
| 228 | CH₂CH₃ | CH₃ | O | CH₃ | CH₃ | 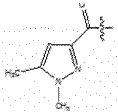 |
| 229 | CH(CH₃)₂ | CH₃ | O | CH₃ | CH₃ | 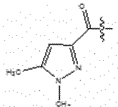 |
| 230 | CH(CH₃)₂ | CH₃ | O | 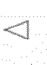 | CH₃ | 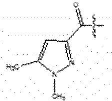 |
| 231 | CH₃ | CH₃ | O | CH3 | CH₃ | 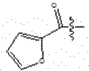 |
| 232 | CH3 | CH₃ | s | CH₃ | CH₃ | 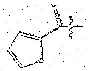 |
| 233 | CH₂CH₃ | CH₃ | O | CH₃ | CH₃ | 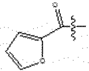 |
| 234 | CH(CH₃)₂ | CH₃ | O | CH3 | CH₃ | 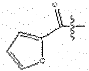 |
| 235 | CH₃ | CH₃ | O | 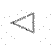 | CH₃ | 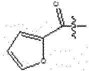 |
| 236 | CH₂CH₃ | CH₃ | O | 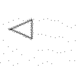 | CH₃ | 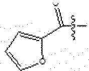 |
| 237 | CH(CH₃)₂ | CH₃ | O | 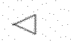 | CH₃ | 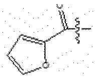 |
| 238 | CH₃ | CH3 | O | CH₃ | CH3 | 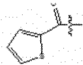 |
| 239 | CH₂CH₃ | CH₃ | O | CH₃ | CH₃ | 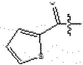 |
| 240 | CH(CH₃)₂ | CH₃ | O | CH₃ | CH₃ | 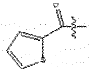 |
| 241 | CH₃ | CH₃ | O | 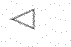 | CH₃ | 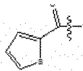 |
| 242 | CH₂CH₃ | CH₃ | O | 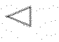 | CH₃ | 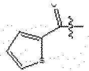 |
| 243 | CH(CH₃)₂ | CH₃ | O | 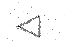 | CH₃ | 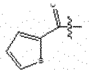 |
| 244 | CH₃ | CH₃ | O | CH₃ | CH₃ | 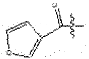 |
| 245 | CH₃ | CH₃ | S | CH₃ | CH₃ | 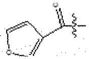 |
| 246 | CH(CH₃)₂ | CH₃ | O | CH₃ | CH₃ | 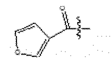 |
| 247 | CH₃ | CH₃ | O | 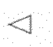 | CH₃ | 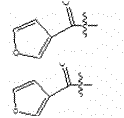 |
| 248 | CH₂CH₃ | CH₃ | O | 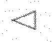 | CH₃ | 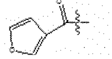 |
| 249 | CH(CH₃)₂ | CH₃ | O | 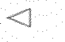 | CH₃ | 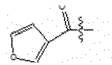 |
| 250 | CH₃ | CH₃ | O | CH₃ | CH₃ | 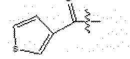 |
| 251 | CH(CH₃)₂ | CH₃ | O | CH₃ | CH₃ | 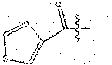 |
| 252 | CH₃ | CH₃ | O | 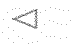 | CH₃ | 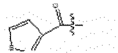 |
| 253 | CH₂CH₃ | CH₃ | O | 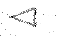 | CH₃ | 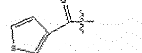 |
| 254 | CH(CH₃)₂ | CH₃ | O | 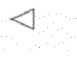 | CH₃ | 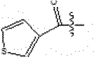 |
| 255 | CH₃ | CH₃ | O | CH3 | CH₃ | 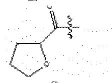 |
| 256 | CH₃ | CH3 | S | CH3 | CH3 | 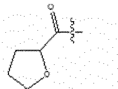 |
| 257 | CH(CH₃)₂ | CH₃ | O | CH₃ | CH3 | 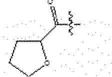 |
| 258 | CH₃ | CH₃ | O | 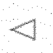 | CH₃ | 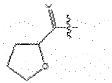 |
| 259 | CH₂CH₃ | CH₃ | O | 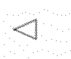 | CH₃ | 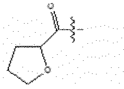 |
| 260 | CH(CH₃)₂ | CH₃ | O | 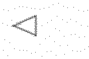 | CH₃ | 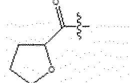 |

The molecular formulas, and the data of mass spectra, ¹H NMR and ¹³C NMR for some compounds are shown in Table 4.

**Table 4 The molecular formulas, and the data of mass spectra, ¹H NMR and ¹³C NMR for some compounds**

| Compound | Molecular Formula | Mass spectra M/Z:[M+H]+ | 1H NMR | 13C NMR |
|---|---|---|---|---|
| 1 | C₁₈H₁₇ClN₂O₇S | 441.0518 | 1H-NMR (400 MHz, CHLOROFORM-D) δ 8.16 (d, J = 8.2 Hz, 1H), 7.35 (d, J = 8.2 Hz, 1H), 5.57 (s, 2H), 3.35 (t, J = 15.0 Hz, 3H), 2.82 (t, J = 6.5 Hz, 2H), 2.43 (t, J = 6.5 Hz, 2H), 2.19 (t, J = 15.0 Hz, 3H), 2.08 (q, J = 6.4 Hz, 2H) | 13C-NMR(101 MHz, ACETONITRILE-D 3) δ 195.6, 154.0, 153.5, 145.8, 142.5, 132.9, 131.9, 128.8, 127.0, 113.7, 44.8, 42.2, 18.8, 11.4 |
| 5 | C₁₈H₁₄ClF₃N₂O₇ S | 495.0235 | 1H-NMR (400 MHz, CHLOROFORM-D) δ 8.17 (d, J = 8.0 Hz, 1H), 7.40 (d, J = 8.0 Hz, 1H), 5.74 (s, 2H), 3.30 (t, J = 15.1 Hz, 3H), 2.83 (t, J = 6.5 Hz, 2H), 2.42 (t, J = 6.5 Hz, 2H), 2.10-2.04 (m, 2H) | 13C-NMR (101 MHz, CHLOROFORM-D) δ 197.5, 195.6, 193.5, 150.9, 145.7, 142.6, 133.6, 130.3, 129.4, 127.6, 113.8, 113.6, 45.6, 42.9, 37.5, 32.2, 19.1 |
| 6 | C₁₉H₁₉ClN₂O₇S | 455.0674 | 1H-NMR (400 MHz, CHLOROFORM-D) δ 8.14-8.08 (m, 1H), 7.37-7.32 (m, 1H), 5.62 (d, J = 67.3 Hz, 2H), 3.53-3.42 (m, 2H), 2.87-2.77 (m, 2H), 2.52-2.35 (m, 2H), 2.17 (d, J = 4.1 Hz, 3H), 2.12-2.01 (m, 2H), 1.37-1.30 (m, 3H) | 13C-NMR (101 MHz, CHLOROFORM-D) δ 197.3, 196.0, 193.5, 153.7, 153.4, 145.5, 140.5, 133.4, 132.2, 130.1, 126.6, 113.8, 51.3, 42.3, 37.6, 32.2, 19.1, 12.5, 7.3 |
| 9 | C₂₂H₂₄ClN₂O₇S | 497.1144 | 1H-NMR (400 MHz, CHLOROFORM-D) δ 8.12 (d, J = 8.2 Hz, 1H), 7.35 (d, J = 8.0 Hz, 1H), 5.58 (d, J = 11.8 Hz, 2H), 3.58 (td, J = 14.6, 7.2 Hz, 2H), 2.87-2.78 (m, 2H), 2.40 (t, J = 6.5 Hz, 2H), 2.11-2.02 (m, 2H), 1.30 (t, J = 7.4 Hz, 3H), 1.22 (s, 9H) | 13C-NMR (101 MHz, CHLOROFORM-D) δ 197.1,196.1, 193.3, 162.9, 153.6, 145.4, 140.3, 133.6, 132.4, 130.3, 126.5, 113.8, 51.1, 42.2, 37.6, 32.8, 32.2, 26.9, 19.1, 7.7 |
| 10 | C₁₉H₁₆ClF₃N₂O₇ S | 509.0392 | 1H-NMR (400 MHz, CHLOROFORM-D) δ 8.15-8.09 (m, 1H), 7.42-7.36 (m, 1H), 5.82-5.58 (m, 2H), 3.40 (td, J = 14.9, 7.5 Hz, 2H), 2.88-2.76 (m, 2H), 2.47-2.36 (m, 2H), 2.12-2.01 (m, 2H), 1.37-1.29 (m, 3H) | 13C-NMR (101 MHz, CHLOROFORM-D) δ 197.4, 195.6, 193.4, 150.9, 145.6, 140.6, 133.6, 130.7, 130.4, 127.3, 116.5, 113.8, 113.7, 51.6, 43.2, 37.5, 32.2, 19.1, 7.2 |
| 11 | C₂₀H₂₁ClN₂O₇S | 469.0831 | 1H-NMR (400 MHz, CHLOROFORM-D) δ 8.11 (d, J = 8.2 Hz, 1H), 7.34 (d, J = 8.2 Hz, 1H), 5.53 (s, 2H), 3.43 (t, J = 8.1 Hz, 2H), 2.82 (t, J = 6.3 Hz, 2H), 2.42 (t, J = 6.6 Hz, 2H), 2.21-2.14 (m, 3H), 2.10-2.03 (m, 2H), 1.81-1.75 (m, 2H), 1.07-0.99 (m, 3H) | 13C-NMR (101 MHz, CHLOROFORM-D) δ 197.3, 196.0, 193.4, 153.7, 153.4, 145.4, 141.1, 133.4, 132.1, 129.9, 126.6, 113.8, 58.4, 42.3, 37.6, 32.2, 19.1, 16.6, 12.9, 12.4 |
| 16 | C₂₀H₂₁ClN₂O₇S | 469.0831 | 1H-NMR (400 MHz, CHLOROFORM-D) δ 8.09 (d, J = 8.2 Hz, 1H), 7.34 (d, J = 8.2 Hz, 1H), 5.49 (s, 2H), 3.67 (t, J = 6.7 Hz, 1H), 2.82 (t, J = 6.3 Hz, 2H), 2.42 (t, J = 6.5 Hz, 2H), 2.17 (t, J = 15.0 Hz, 3H), 2.06 (t, J = 6.5 Hz, 2H), 1.36 (d, J = 6.9 Hz, 6H) | 13C-NMR (101 MHz, CHLOROFORM-D) δ 197.3, 196.0, 193.4, 153.6, 153.5, 145.3, 139.8, 133.4, 132.4, 130.5, 126.4, 113.8, 56.0, 42.6, 37.6, 32.2, 19.1, 15.3, 12.4 |
| 21 | C₂₁H₂₃ClN₂O₇S | 483.0987 | 1H-NMR (400 MHz, CHLOROFORM-D) δ 8.14-8.08 (m, 1H), 7.37-7.31 (m, 1H), 5.75-5.54 (m, 2H), 3.46-3.37 (m, 2H), 2.86-2.78 (m, 2H), 2.48-2.37 (m, 2H), 2.18-2.16 (m, 3H), 2.10-2.03 (m, 2H), 1.78-1.70 (m, 2H), 1.44 (td, J = 14.8, 7.4 Hz, 2H), 0.97-0.90 (m, 3H) | 13C-NMR (101 MHz, CHLOROFORM-D) δ 197.3, 196.1, 193.4, 153.7, 153.4, 145.4, 141.2, 133.4, 132.1, 129.9, 126.7, 113.8, 56.7, 42.2, 37.6, 32.2, 24.5, 21.6, 19.1, 13.6, 12.4 |
| 26 | C₂₁H₂₃ClN₂O₇S | 483.0987 | 1H-NMR (400 MHz, CHLOROFORM-D) δ 8.06 (dd, J = 15.0, 6.7 Hz, 1H), 7.37-7.31 (m, 1H), 5.47 (t, J = 53.0 Hz, 2H), 2.87-2.76 (m, 2H), 2.48-2.37 (m, 2H), 2.16 (s, 3H), 2.09-2.01 (m, 2H), 1.39 (s, 9H) | 13C-NMR (101 MHz, CHLOROFORM-D) δ 197.3, 196.0, 193.4, 153.9, 153.3, 145.4, 137.2, 133.5, 133.5, 132.2, 126.3, 113.8, 61.9, 43.0, 37.6, 23.8, 19.1, 12.4 |
| 45 | C₂₀H₂₁ClN₂O₆S₂ | 485.0602 | 1H-NMR (400 MHz, CHLOROFORM-D) δ 8.06 (d, J = 8.2 Hz, 1H), 7.47-7.41 (m, 1H), 5.48 (s, 2H), 3.26 (s, 3H), 2.95-2.86 (m, 4H), 2.38-2.35 (m, 2H), 2.10 (t, J = 4.0 Hz, 3H), 2.07-2.01 (m, 2H), 1.31 (t, J = 7.4 Hz, 3H) | 13C-NMR (101 MHz, CHLOROFORM-D) δ 193.4, 191.8, 179.0, 153.7, 153.4, 147.3, 142.5, 133.4, 131.7, 130.6, 128.9, 45.6, 42.0, 37.1, 31.3, 26.3, 21.8, 13.6, 12.4 |
| 46 | C₂₁H₂₃ClN₂O₆S₂ | 499.0759 | 1H-NMR (400 MHz, CHLOROFORM-D) δ 8.05-8.09 (1H), 7.47-7.51 (1H), 5.46-5.50 (2H), 3.48-3.57 (1H), 3.24-3.27 (3H), 2.88-2.94 (2H), 2.34-2.41 (2H), 2.09-2.11 (3H), 2.00-2.08 (2H), 1.30-1.35 (6H) | 13C-NMR (101 MHz, CHLOROFORM-D) δ 193.5, 192.0, 176.3, 153.7, 153.4, 146.8, 142.8, 133.6, 131.8, 131.8, 129.4, 128.9, 45.6, 42.0, 37.2, 36.2, 30.8, 23.9, 22.0, 12.4 |
| 47 | C₂₁H₂₃ClN₂O₆S₂ | 499.0759 | 1H-NMR (400 MHz, CHLOROFORM-D) δ 8.02-8.14 (1H), 7.35-7.55 (1H), 5.42-5.55 (2H), 3.22-3.33 (3H), 2.78-2.97 (4H), 2.28-2.45 (2H), 2.07-2.13 (3H), 1.96-2.07 (2H), 1.57-1.76 (2H), 0.92-1.09 (3H) | 13C-NMR (101 MHz, CHLOROFORM-D) δ 193.4, 191.8, 178.9, 153.7, 153.4, 147.2, 142.5, 133.4, 131.7, 130.9, 128.9, 128.9, 45.6, 42.1, 37.1, 34.0, 31.4, 22.2, 21.8, 13.6, 12.3 |
| 48 | C₂₂H₂₅ClN₂O₆S₂ | 513.0915 | 1H-NMR (400 MHz, CHLOROFORM-D) δ 8.04-8.09 (1H), 7.42-7.46 (1H), 5.45-5.50 (2H), 3.23-3.27 (3H), 2.83-2.91 (4H), 2.33-2.39 (2H), 2.08-2.11 (3H), 1.99-2.08 (2H), 1.56-1.65 (2H), 1.38-1.46 (2H), 0.86-0.93 (3H) | 13C-NMR (101 MHz, CHLOROFORM-D) δ 193.4, 191.8, 178.9, 153.6, 153.4, 147.3, 142.5, 133.4, 131.8, 130.8, 128.9, 128.9, 45.6, 42.1, 37.1, 31.8, 31.4, 30.6, 22.1, 21.8, 13.7, 12.4 |
| 49 | C₂₂H₂₅ClN₂O₆S₂ | 519.0915 | 1H-NMR (400 MHz, CHLOROFORM-D) δ 8.07 (d, J = 8.2 Hz, 1H), 7.64 (d, J = 8.2 Hz, 1H), 5.50 (s, 2H), 3.26 (s, 3H), 2.89 (t, J = 5.9 Hz, 2H), 2.42 (t, J = 6.6 Hz, 2H), 2.10 (s, 3H), 2.08-2.04 (m, 2H), 1.36 (d, J = 2.2 Hz, 9H) | 13C-NMR (101 MHz, CHLOROFORM-D) δ 194.0, 192.2, 168.5, 153.8, 153.4, 144.8, 143.8, 137.5, 134.9, 132.4, 130.9, 128.5, 49.8, 45.5, 42.1, 37.4, 32.8, 32.4, 30.7, 22.1, 12.4 |
| 51 | C₂₄H₂₀Cl₂N₂O₆S ₂ | 567.0213 | 1H-NMR (400 MHz, CHLOROFORM-D) δ 8.10 (d, J = 8.2 Hz, 1H), 7.50 (d, J = 8.2 Hz, 1H), 7.43-7.37 (m, 4H), 5.50 (s, 2H), 3.27 (s, 3H), 2.39-2.32 (m, 4H), 2.11 (s, 3H), 1.91-1.84 (m, 2H) | 13C-NMR (101 MHz, CHLOROFORM-D) δ 193.6, 192.0, 177.3, 153.7, 153.4, 146.8, 142.8, 137.4, 136.8, 133.4, 131.9, 131.0, 130.2, 129.1, 129.1, 128.0, 45.6, 42.0, 37.2, 33.0, 29.8, 21.8, 12.4 |
| 53 | C₂₅H₂₃ClN₂O₆S₂ | 547.0759 | 1H-NMR (400 MHz, CHLOROFORM-D) δ 8.05 (d, J = 8.2 Hz, 1H), 7.42 (d, J = 8.2 Hz, 1H), 7.29-7.24 (m, 5H), 5.47 (s, 2H), 4.12 (s, 2H), 3.25 (s, 3H), 2.89 (t, J = 6.0 Hz, 2H), 2.35 (t, J = 6.7 Hz, 2H), 2.09 (d, J = 4.4 Hz, 3H), 2.04-1.98 (m, 2H) | 13C-NMR (101 MHz, CHLOROFORM-D) δ 193.4, 191.7, 178.1, 153.7, 153.4, 147.1, 142.5, 134.7, 133.4, 131.8, 131.0, 129.2, 129.1, 128.9, 128.2, 77.4, 77.1, 76.8, 45.6, 42.0, 37.1, 37.0, 31.8, 21.7, 12.4 |
| 56 | C₁₆H₁₅ClN₄O₆S | 427.0474 | 1H-NMR (400 MHz, ACETONITRILE-D3) δ 8.18 (d, J = 8.2 Hz, 1H), 7.68 (d, J = 8.2 Hz, 1H), 7.25 (s, 1H), 5.48 (s, 2H), 3.62 (s, 3H), 3.31 (s, 3H), 2.14 (s, 3H) | 13C-NMR (101 MHz, CHLOROFORM-D) δ 188.1, 158.3, 154.0, 153.3, 144.0, 143.1, 139.0, 134.7, 133.1, 128.9, 103.8, 45.5, 42.4, 33.4, 12.4 |
| 57 | C₁₇H₁₇ClN₄O₆S | 441.063 | 1H-NMR (400 MHz, ACETONITRILE-D3) δ 8.18 (d, J = 8.2 Hz, 1H), 7.70-7.66 (m, 1H), 7.27 (s, 1H), 5.49 (s, 2H), 4.01 (td, J = 14.8, 7.5 Hz, 2H), 3.31 (s, 3H), 2.14 (t, J = 4.0 Hz, 3H), 1.36 (t, J = 7.3 Hz, 3H) | 13C-NMR (101 MHz, CHLOROFORM-D) δ 188.2, 157.7, 153.9, 153.3, 144.0, 143.1, 138.9, 134.7, 133.1, 129.0, 128.9, 103.8, 45.5, 42.4, 41.9, 14.2, 12.4 |
| 58 | C₁₇H₁₇ClN₄O₆S | 441.063 | 1H-NMR (400 MHz, CHLOROFORM-D) δ 8.24 (t, J = 4.1 Hz, 1H), 7.53 (d, J = 8.0 Hz, 1H), 5.60 (s, 2H), 3.65 (s, 3H), 3.35 (s, 3H), 2.19 (s, 3H), 1.67 (s, 3H) | 13C-NMR (101 MHz, CHLOROFORM-D) δ 188.6, 159.3, 153.9, 153.3, 147.3, 144.5, 143.6, 134.0, 132.9, 129.2, 128.0, 102.8, 45.5, 42.3, 32.8, 13.7, 12.3 |
| 59 | C₁₈H₁₉ClN₄O₆S | 455.0787 | 1H-NMR (400 MHz, CHLOROFORM-D) δ 8.24 (d, J = 8.2 Hz, 1H), 7.53 (d, J = 8.2 Hz, 1H), 5.64-5.58 (m, 2H), 4.01 (q, J = 7.2 Hz, 2H), 3.34 (d, J = 6.9 Hz, 3H), 2.17 (d, J = 7.7 Hz, 3H), 1.67 (s, 3H), 1.43 (t, J = 7.3 Hz, 3H) | 13C-NMR (101 MHz, CHLOROFORM-D) δ 188.7,158.7, 153.9, 153.3, 147.1, 144.5, 143.6, 134.0, 132.9, 129.2, 128.0, 102.8, 45.5, 42.3, 41.3, 14.2, 13.8, 12.3 |
| 60 | C₁₈H₁₉ClN₄O₆S | 455.0787 | 1H-NMR (400 MHz, CHLOROFORM-D) δ 8.24 (d, J = 8.0 Hz, 1H), 7.54 (d, J = 8.0 Hz, 1H), 5.60 (s, 2H), 3.74 (s, 1H), 3.66 (d, J = 4.9 Hz, 3H), 3.35 (d, J = 3.0 Hz, 3H), 2.19 (s, 3H), 1.99 (d, J = 7.1 Hz, 2H), 0.92 (t, J = 7.4 Hz, 3H) | 13C-NMR (101 MHz, CHLOROFORM-D) δ 188.6, 159.6, 153.9, 153.3, 152.3, 144.6, 143.6, 134.1, 132.9, 129.2, 128.0, 102.0, 45.5, 42.3, 32.9, 21.2, 12.4, 12.0 |
| 68 | C₁₉H₁₉ClN₄O₆S | 467.0787 | 1H-NMR (400 MHz, ACETONITRILE-D3) δ 8.18 (d, J = 8.0 Hz, 1H), 7.66 (d, J = 8.2 Hz, 1H), 5.48 (s, 2H), 3.53 (d, J = 11.3 Hz, 3H), 3.28 (s, 3H), 2.12 (s, 3H), 0.93 (s, 1H), 0.58 (d, J = 2.7 Hz, 2H), 0.36 (d, J = 5.2 Hz, 2H) | 13C-NMR (101 MHz, ACETONITRILE-D 3) δ 188.6, 158.5, 154.0, 153.4, 151.7, 144.9, 143.3, 133.3, 132.6, 129.3, 128.4, 103.0, 44.8, 42.4, 32.4, 11.4, 8.2, 6.2 |
| 69 | C₂₀H₂₁ClN₄O₆S | 449.1222 | 1H-NMR (400 MHz, ACETONITRILE-D3) δ 8.18 (d, J = 8.2 Hz, 1H), 7.66 (d, J = 8.2 Hz, 1H), 5.52-5.44 (m, 2H), 3.91 (q, J = 7.3 Hz, 2H), 3.79 (d, J = 14.8 Hz, 1H), 3.32-3.25 (m, 3H), 2.16-2.08 (m, 3H), 1.29 (td, J = 14.8, 7.3 Hz, 3H), 1.00-0.98 (m, 1H), 0.59 (d, J = 4.4 Hz, 2H), 0.37 (d, J = 6.6 Hz, 2H) | 13C-NMR (101 MHz, ACETONITRILE-D 3) δ 188.7, 158.0, 154.0, 153.4, 151.7, 145.0, 143.3, 133.3, 132.6, 129.3, 128.4, 103.1, 44.8, 44.7, 42.4, 41.0, 13.4, 11.4, 8.3, 6.2 |
| 70 | C₁₇H₁₇ClN₄O₆S | 441.063 | 1H-NMR (400 MHz, ACETONITRILE-D3) δ 8.13 (d, J = 8.0 Hz, 1H), 7.67 (d, J = 8.0 Hz, 1H), 7.26 (s, 1H), 5.44 (d, J = 6.0 Hz, 2H), 3.62 (s, 3H), 3.47-3.41 (m, 2H), 2.13 (d, J = 5.5 Hz, 3H), 1.25-1.19 (m, 3H) | 13C-NMR (101 MHz, CHLOROFORM-D) δ 188.2,158.2, 153.9, 153.4, 143.0, 142.1, 139.0, 134.7, 133.5, 130.0, 128.6, 103.8, 51.3, 42.6, 33.4, 12.5, 7.3 |
| 71 | C₁₈H₁₉ClN₄O₆S | 455.0787 | 1H-NMR (400 MHz, METHANOL-D4) δ 8.13 (d, J = 8.2 Hz, 1H), 7.67 (d, J = 8.2 Hz, 1H), 7.27 (s, 1H), 5.72 (s, 1H), 5.45 (t, J = 3.4 Hz, 2H), 4.01 (q, J = 7.2 Hz, 2H), 2.14-2.12 (m, 3H), 1.73-1.64 (m, 2H), 1.36 (q, J = 7.3 Hz, 3H), 1.03-0.95 (m, 3H) | 13C-NMR (101 MHz, METHANOL-D4) δ 189.0, 157.8, 155.4, 144.9, 143.6, 140.6, 135.3, 134.6, 131.2, 130.2, 105.3, 59.1, 44.0, 42.8, 17.9, 14.8, 13.4, 12.9 |
| 72 | C₁₈H₁₉ClN₄O₆S | 455.0787 | 1H-NMR (400 MHz, CHLOROFORM-D) δ 8.19 (d, J = 8.2 Hz, 1H), 7.52 (d, J = 8.2 Hz, 1H), 5.56 (s, 2H), 3.65 (s, 3H), 3.44 (t, J = 7.4 Hz, 2H), 2.17 (s, 3H), 1.65 (s, 3H), 1.33 (t, J = 7.3 Hz, 3H) | 13C-NMR (101 MHz, CHLOROFORM-D) δ 188.7,159.3, 153.9, 153.3, 147.3, 144.4, 141.6, 134.1, 133.2, 130.4, 127.6, 102.8, 51.3, 42.5, 32.8, 13.6, 12.4,7.3 |
| 73 | C₁₉H₂₁ClN₄O₆S | 469.0943 | 1H-NMR (400 MHz, ACETONITRILE-D3) δ 8.14 (d, J = 8.2 Hz, 1H), 7.60 (d, J = 8.0 Hz, 1H), 5.45 (d, J = 3.8 Hz, 2H), 3.93 (q, J = 7.2 Hz, 2H), 3.42 (q, J = 7.4 Hz, 2H), 2.16-2.08 (m, 3H), 1.61 (s, 3H), 1.32 (t, J = 7.1 Hz, 3H), 1.24-1.17 (m, 3H) | 13C-NMR (101 MHz, ACETONITRILE-D 3) δ 188.5, 158.0, 154.1, 153.5, 147.1, 144.7, 141.1, 133.3, 133.3, 130.5, 127.9, 102.6, 50.8, 42.6, 40.8, 13.4, 13.0, 11.4, 6.8 |
| 78 | C₂₀H₂₃ClN₄O₆S | 483.11 | 1H-NMR (400 MHz, CHLOROFORM-D) δ 8.18 (d, J = 8.0 Hz, 1H), 7.54 (d, J = 8.0 Hz, 1H), 5.56 (s, 2H), 3.66 (s, 3H), 3.47 (q, J = 7.4 Hz, 2H), 2.18 (s, 3H), 2.14 (s, 1H), 1.34-1.30 (m, 3H), 0.96 (d, J = 6.9 Hz, 6H) | 13C-NMR (101 MHz, CHLOROFORM-D) δ 188.7,159.7, 156.4, 153.8, 153.3, 144.5, 141.4, 134.2, 133.1, 130.2, 127.7, 101.4, 51.3, 42.5, 32.9, 27.0, 21.8, 12.4, 7.4 |
| 82 | C₂₀H₂₁ClN₄O₆S | 481.0943 | 1H-NMR (400 MHz, CHLOROFORM-D) δ 8.18 (d, J = 8.2 Hz, 1H), 7.59 (d, J = 8.0 Hz, 1H), 5.55 (s, 2H), 5.31 (s, 0H), 3.61 (s, 3H), 3.45 (d, J = 7.4 Hz, 2H), 2.17 (s, 3H), 1.32 (t, J = 7.4 Hz, 3H), 0.89 (s, 1H), 0.71 (s, 2H), 0.41 (dd, J = 8.2, 2.2 Hz, 2H) | 13C-NMR (101 MHz, CHLOROFORM-D) δ 188.7,159.2, 153.8, 153.3, 152.0, 144.8, 141.4, 134.3, 132.9, 130.1, 127.9, 103.2, 51.3, 42.5, 32.8, 12.4, 8.5, 7.3, 7.0, 6.8 |
| 83 | C₂₁H₂₃ClN₄O₆S | 495.11 | 1H-NMR (400 MHz, ACETONITRILE-D3) δ 8.14 (d, J = 8.2 Hz, 1H), 7.66 (d, J = 8.0 Hz, 1H), 5.45 (t, J = 6.0 Hz, 2H), 3.90 (q, J = 7.3 Hz, 2H), 3.81 (s, 1H), 3.41 (q, J = 7.4 Hz, 2H), 2.15-2.08 (m, 3H), 1.34-1.26 (m, 3H), 1.24-1.16 (m, 3H), 0.97 (t, J = 4.5 Hz, 1H), 0.59 (d, J = 4.7 Hz, 2H), 0.37-0.35 (m, 2H) | 13C-NMR (101 MHz, ACETONITRILE-D 3) δ 188.6, 158.0, 154.0, 153.4, 151.7, 145.0, 141.1, 133.5, 133.0, 130.3, 128.2, 103.1, 50.9, 44.7, 42.5, 41.0, 13.4, 11.4, 8.3, 6.8, 6.2 |
| 84 | C₁₈H₁₉ClN₄O₆S | 455.0787 | 1H-NMR (400 MHz, ACETONITRILE-D3) δ 8.13 (d, J = 8.2 Hz, 1H), 7.67 (d, J = 8.0 Hz, 1H), 7.25 (s, 1H), 5.45 (s, 2H), 3.62 (s, 3H), 3.44-3.40 (m, 2H), 2.14 (s, 3H), 1.71-1.63 (m, 2H), 0.99 (t, J = 7.4 Hz, 3H) | 13C-NMR (101 MHz, ACETONITRILE-D 3) δ 187.5, 156.9, 154.1, 153.5, 143.5, 142.1, 139.0, 133.9, 133.3, 130.0, 128.7, 103.9, 57.6, 42.6, 16.5, 12.0, 11.5 |
| 85 | C₁₉H₂₁ClN₄O₆S | 469.0943 | 1H-NMR (400 MHz, CHLOROFORM-D) δ 8.66-8.38 (m, 1H), 8.18 (d, J = 8.2 Hz, 1H), 7.63 (d, J = 8.2 Hz, 1H), 5.58 (s, 2H), 4.09 (q, J = 7.2 Hz, 2H), 3.47-3.43 (m, 2H), 2.20 (s, 3H), 1.83-1.73 (m, 2H), 1.46 (t, J = 7.1 Hz, 3H), 1.05 (t, J = 7.4 Hz, 3H) | 13C-NMR (101 MHz, CHLOROFORM-D) δ 188.3, 157.7, 153.9, 153.3, 143.0, 142.6, 139.0, 134.7, 133.4, 129.8, 128.7, 103.8, 58.2, 42.5, 41.8, 16.7, 14.2, 12.9, 12.4 |
| 86 | C₁₉H₂₁ClN₄O₆S | 469.0943 | 1H-NMR (400 MHz, CHLOROFORM-D) δ 8.19 (d, J = 8.0 Hz, 1H), 7.51 (d, J = 8.0 Hz, 1H), 5.56 (s, 2H), 3.64 (s, 3H), 3.41 (t, J = 7.8 Hz, 2H), 2.21-2.13 (m, 3H), 1.79-1.73 (m, 2H), 1.67 (d, J = 15.1 Hz, 3H), 1.04 (t, J = 7.4 Hz, 3H) | 13C-NMR (101 MHz, CHLOROFORM-D) δ 188.7, 159.3, 153.8, 153.3, 147.3, 144.4, 142.1, 134.0, 133.1, 130.2, 127.6, 102.8, 58.3, 42.5, 32.8, 16.7, 13.7, 12.9, 12.3 |
| 87 | C₂₀H₂₃ClN₄O₆S | 483.11 | 1H-NMR (400 MHz, ACETONITRILE-D3) δ 8.17-8.11 (m, 1H), 7.63-7.57 (m, 1H), 5.48-5.41 (m, 2H), 3.98-3.87 (m, 2H), 3.44-3.35 (m, 2H), 2.14-2.11 (m, 3H), 1.73-1.62 (m, 3H), 1.60 (s, 2H), 1.36-1.28 (m, 3H), 1.01-0.94 (m, 3H) | 13C-NMR (101 MHz, ACETONITRILE-D 3) δ 188.5, 158.0, 154.1, 153.5, 147.1, 144.7, 141.7, 133.3, 133.2, 130.3, 127.9, 102.6, 57.6, 42.5, 40.8, 16.6, 13.4, 13.0, 12.0, 11.4 |
| 89 | C₂₁H₂₅ClN₄O₆S | 497.1256 | 1H-NMR (400 MHz, CHLOROFORM-D) δ 8.21-8.15 (m, 1H), 7.55-7.50 (m, 1H), 5.55 (d, J = 14.8 Hz, 2H), 4.02 (td, J = 14.7, 7.6 Hz, 2H), 3.48-3.40 (m, 2H), 2.17 (d, J = 4.1 Hz, 3H), 2.05-1.89 (m, 2H), 1.75 (d, J = 6.9 Hz, 2H), 1.47-1.40 (m, 3H), 1.08-1.00 (m, 3H), 0.93-0.86 (m, 3H) | 13C-NMR (101 MHz, CHLOROFORM-D) δ 188.7,159.0, 153.8, 153.3, 152.1, 144.5, 141.9, 134.1, 133.1, 130.1, 127.7, 102.1, 58.3, 42.5, 41.4, 21.3, 16.8, 14.2, 12.9, 12.4, 12.1 |
| 96 | C₂₁H₂₃ClN₄O₆S | 495.11 | 1H-NMR (400 MHz, CHLOROFORM-D) δ 8.17 (d, J = 8.0 Hz, 1H), 7.57 (d, J = 8.0 Hz, 1H), 5.56 (s, 2H), 3.61 (s, 3H), 3.41 (t, J = 7.7 Hz, 2H), 2.17 (s, 3H), 1.74 (d, J = 7.4 Hz, 2H), 1.03 (t, J = 7.4 Hz, 3H), 0.87-0.85 (m, 1H), 0.71 (s, 2H), 0.41 (dd, J = 8.2, 2.2 Hz, 2H) | 13C-NMR (101 MHz, CHLOROFORM-D) δ 188.8, 159.3, 153.8, 153.3, 152.1, 144.7, 141.9, 134.3, 132.9, 130.0, 127.9, 103.2, 58.3, 42.5, 32.9, 16.8, 12.9, 12.4, 8.5, 6.8 |
| 97 | C₂₂H₂₅ClN₄O₆S | 509.1256 | 1H-NMR (400 MHz, ACETONITRILE-D3) δ 8.14 (dd, J = 15.0, 6.7 Hz, 1H), 7.66 (dd, J = 14.8, 6.9 Hz, 1H), 5.45 (s, 2H), 3.91 (td, J = 14.7, 7.6 Hz, 2H), 3.41-3.37 (m, 2H), 2.11 (t, J = 4.0 Hz, 3H), 1.68-1.58 (m, 2H), 1.34-1.27 (m, 3H), 1.00-0.91 (m, 4H), 0.60 (q, J = 4.9 Hz, 2H), 0.36 (dd, J = 8.2, 2.2 Hz, 2H) | 13C-NMR (101 MHz, ACETONITRILE-D 3) δ 188.7, 158.0, 154.0, 153.4, 151.7, 144.9, 141.7, 133.5, 132.9, 130.2, 128.2, 103.1, 57.7, 42.5, 41.0, 16.6, 13.3, 11.9, 11.5, 8.2, 6.2 |
| 98 | C₁₈H₁₉ClN₄O₆S | 455.0787 | 1H-NMR (400 MHz, ACETONITRILE-D3) δ 8.11 (d, J = 8.2 Hz, 1H), 7.67 (d, J = 8.0 Hz, 1H), 7.26 (s, 1H), 5.42 (d, J = 3.6 Hz, 2H), 3.67 (q, J = 6.8 Hz, 1H), 3.62 (s, 3H), 2.13 (s, 3H), 1.28 (d, J = 6.6 Hz, 6H) | 13C-NMR (101 MHz, CHLOROFORM-D) δ 188.3, 158.2, 153.9, 153.4, 142.9, 141.4, 138.9, 134.7, 133.6, 130.4, 128.5, 128.3, 103.8, 56.0, 55.9, 42.9, 15.3, 12.5 |
| 99 | C₁₉H₂₁ClN₄O₆S | 469.0943 | 1H-NMR (400 MHz, ACETONITRILE-D3) δ 8.14-8.09 (m, 1H), 7.70-7.64 (m, 1H), 7.29 (q, J = 14.9 Hz, 1H), 5.42 (d, J = 7.1 Hz, 2H), 4.01 (td, J = 14.7, 7.6 Hz, 2H), 3.69-3.62 (m, 1H), 2.13-2.12 (m, 3H), 1.37-1.33 (m, 3H), 1.31-1.27 (m, 6H) | 13C-NMR (101 MHz, ACETONITRILE-D 3) δ 187.6, 156.4, 154.0, 153.5, 143.5, 140.9, 139.3, 134.0, 133.5, 130.5, 128.6, 103.9, 55.7, 42.8, 41.4, 14.4, 13.4, 11.5 |
| 100 | C₁₉H₂₁ClN₄O₆S | 469.0943 | 1H-NMR (400 MHz, ACETONITRILE-D3) δ 8.13 (d, J = 8.0 Hz, 1H), 7.59 (d, J = 8.0 Hz, 1H), 5.45-5.41 (m, 2H), 3.61 (q, J = 6.9 Hz, 1H), 3.54 (s, 3H), 2.13-2.11 (m, 3H), 1.60 (s, 3H), 1.30-1.24 (m, 6H) | 13C-NMR (101 MHz, CHLOROFORM-D) δ 188.8, 159.3, 153.8, 153.4, 147.3, 144.3, 140.9, 134.0, 133.4, 130.9, 127.4, 102.8, 56.1, 42.8, 32.8, 15.2, 13.6, 12.5, 12.4 |
| 101 | C₂₀H₂₃ClN₄O₆S | 483.11 | 1H-NMR (400 MHz, ACETONITRILE-D3) δ 8.13 (d, J = 8.2 Hz, 1H), 7.60 (d, J = 8.0 Hz, 1H), 5.44 (d, J = 11.0 Hz, 2H), 3.93 (q, J = 7.2 Hz, 2H), 3.62 (t, J = 6.7 Hz, 1H), 2.11 (t, J = 15.0 Hz, 3H), 1.60 (s, 3H), 1.34-1.30 (m, 3H), 1.25 (dd, J = 15.1, 6.9 Hz, 6H) | 13C-NMR (101 MHz, ACETONITRILE-D 3) δ 188.5, 158.0, 154.0, 153.5, 147.0, 144.6, 140.5, 133.5, 133.4, 131.0, 127.7, 102.6, 55.6, 42.7, 40.8, 14.4, 13.4, 13.0, 11.4 |
| 107 | C₂₂H₂₇ClN₄O₆S | 511.1413 | 1H-NMR (400 MHz, CHLOROFORM-D) δ 8.19-8.15 (m, 1H), 7.53 (d, J = 8.2 Hz, 1H), 5.53 (t, J = 14.8 Hz, 2H), 4.02 (q, J = 7.3 Hz, 2H), 3.69-3.62 (m, 1H), 2.17 (t, J = 15.0 Hz, 3H), 2.12-2.05 (m, 1H), 1.48-1.29 (m, 9H), 0.95 (td, J = 14.8, 6.9 Hz, 6H) | 13C-NMR (101 MHz, CHLOROFORM-D) δ 188.7, 159.1, 156.1, 153.7, 153.4, 144.4, 140.7, 134.2, 133.3, 130.6, 127.5, 101.4, 56.1, 42.8, 41.5, 27.1, 22.0, 21.6, 15.5, 14.9, 14.1, 12.5 |
| 110 | C₂₁H₂₃ClN₄O₆S | 495.11 | 1H-NMR (400 MHz, ACETONITRILE-D3) δ 8.11 (d, J = 8.0 Hz, 1H), 7.64 (d, J = 8.0 Hz, 1H), 5.41 (d, J = 4.4 Hz, 2H), 4.06-4.66 (1H), 3.65-3.58 (m, 1H), 3.51 (s, 3H), 2.11 (d, J = 3.0 Hz, 3H), 1.28-1.20 (m, 6H), 0.91 (s, 1H), 0.58 (s, 2H), 0.34 (d, J = 5.8 Hz, 2H) | 13C-NMR (101 MHz, CHLOROFORM-D) δ 188.8, 159.3, 153.7, 153.3, 152.1, 144.7, 140.7, 134.3, 133.1, 130.6, 127.7, 103.2, 56.0, 42.8, 32.8, 15.2, 12.4, 8.5, 6.8 |
| 111 | C₂₂H₂₅ClN₄O₆S | 509.1256 | 1H-NMR (400 MHz, CHLOROFORM-D) δ 8.16 (dd, J = 15.0, 6.7 Hz, 1H), 7.60-7.56 (m, 1H), 5.52 (t, J = 15.0 Hz, 2H), 4.02-3.95 (m, 2H), 3.67-3.60 (m, 1H), 2.16 (s, 3H), 1.42-1.35 (m, 9H), 0.92-0.86 (m, 1H), 0.71 (s, 2H), 0.40 (dd, J = 8.2, 2.2 Hz, 2H) | 13C-NMR (101 MHz, CHLOROFORM-D) δ 188.9, 158.7, 153.7, 153.3, 151.9, 144.7, 140.6, 134.3, 133.1, 130.6, 127.7, 103.3, 56.0, 43.6, 42.8, 41.4, 15.3, 14.1, 12.4, 8.6, 6.7 |
| 112 | C₁₉H₂₁ClN₄O₆S | 469.0943 | 1H-NMR (400 MHz, ACETONITRILE-D3) δ 8.13 (d, J = 8.0 Hz, 1H), 7.67 (d, J = 8.0 Hz, 1H), 7.25 (s, 1H), 5.57-6.04 (1H), 5.45 (d, J = 1.9 Hz, 2H), 3.62 (s, 3H), 3.46-3.42 (m, 2H), 2.13 (s, 3H), 1.66-1.62 (m, 2H), 1.41 (q, J = 7.4 Hz, 2H), 0.90 (t, J = 7.3 Hz, 3H) | 13C-NMR (101 MHz, ACETONITRILE-D 3) δ 187.5,157.0, 154.1, 153.5, 143.5, 142.2, 139.2, 133.9, 133.2, 129.9, 128.8, 103.9, 56.0, 42.6, 32.8, 24.5, 21.1, 12.9, 11.5 |
| 113 | Ca₂₀H₂₃ClN₄O₆S | 483.11 | 1H-NMR(400 MHz, ACETONITRILE-D3) δ 8.13 (d, J = 8.0 Hz, 1H), 7.68 (d, J = 8.0 Hz, 1H), 7.27 (s, 1H), 5.46 (s, 2H), 4.01 (td, J = 14.8, 7.5 Hz, 2H), 3.46-3.40 (m, 2H), 2.13-2.11 (m, 3H), 1.70-1.58 (m, 2H), 1.46-1.38 (m, 2H), 1.36 (t, J = 7.3 Hz, 3H), 0,91 (q, J = 7.5 Hz, 3H) | 13C-NMR (101 MHz, ACETONITRILE-D 3) δ 187.6, 156.4, 154.1, 143.5, 142.2, 139.2, 133.9, 133.3, 129.9, 128.8, 103.9, 55.9, 54.4, 42.5, 41.4, 24.5. 21.1, 13.4, 12.9, 11.5 |
| 114 | C₂₀H₂₃ClN₄O₆S | 483.11 | 1H-NMR (400 MHz, CHLOROFORM-D) δ 8.19 (d, J = 8.0 Hz, 1H), 7.51 (d, J = 8.0 Hz, 1H), 5.57 (s, 2H), 3.64 (t, J = 15.0 Hz, 3H), 3.42 (t, J = 8.0 Hz, 2H), 2.16 (t, J = 15.1 Hz, 3H), 1.71 (t, J = 7.3 Hz, 2H), 1.63 (d, J = 14.8 Hz, 3H), 1.43 (q, J = 7.5 Hz, 2H), 0.93 (t, J = 7.4 Hz, 3H) | 13C-NMR (101 MHz, CHLOROFORM-D) δ 188.8, 159.3, 153.8, 153.3,147.2, 144.4, 142.2, 134.0, 133.1, 130.2, 127.6, 102.8, 56.6, 42.5, 32.8,24.7,21.6, 13.7, 13.6, 12.3 |
| 115 | C₂₁H₂₃ClN₄O₆S | 497.1256 | 1H-NMR (400 MHz, ACETONITRILE-D3) δ 8.14 (dd, J = 15.0, 7.0 Hz, 1H), 7.60 (dd, J = 14.8, 6.9 Hz, 1H), 5.45 (t, J = 14.8 Hz, 2H), 3.93 (qd, J = 14.8, 7.2 Hz, 2H), 3.47-3.35 (m, 2H), 2.11 (t, J = 4.1 Hz, 3H), 1.69-1.56 (m, 5H), 1.43-1.36 (m, 2H), 1.34-1.27 (m, 3H), 0.92-0.84 (m, 3H) | 13C-NMR (101 MHz, ACETONITRILE-D 3) δ 188.5, 158.0, 154.1, 153.5, 144.7, 141.7, 133.3, 133.2, 130.4, 127.9, 102.6, 55.9, 42.5, 40.8, 24.7, 21.1, 13.4, 13.0, 12.9, 11.4 |
| 124 | C₂₂H₂₅ClN₄O₆S | 509.1256 | 1H-NMR (400 MHz, CHLOROFORM-D) δ 8.17 (t, J = 4.1 Hz, 1H), 7.58-7.56 (m, 1H), 5.56 (s, 2H), 3.61 (s, 3H), 3.43 (t, J = 8.0 Hz, 2H), 2.16 (d, J = 4.4 Hz, 3H), 1.73-1.65 (m, 2H), 1.43 (td, J = 15.0, 7.5 Hz, 2H), 0.92 (t, J = 7.3 Hz, 3H), 0.89-0.82 (m, 1H), 0.78-0.72 (m, 2H), 0.41 (dd, J = 8.2, 2.2 Hz, 2H) | 13C-NMR (101 MHz, CHLOROFORM-D) δ 188.8, 159.3, 153.8, 153.3, 152.0, 144.7, 141.9, 134.3, 132.9,130.0,127.9, 103.2, 56.6, 42.4, 32.9, 24.8, 21.6, 13.6, 12.3, 8.5, 6.8 |
| 125 | C₂₃H₂₇ClN₄O₆S | 523.1413 | 1H-NMR (400 MHz, ACETONITRILE-D3) δ 8.14 (d, J = 8.2 Hz, 1H), 7.66 (d, J = 8.2 Hz, 1H), 5.45 (s, 2H), 3.91 (q, J = 7.3 Hz, 2H), 3.41 (t, J = 8.0 Hz, 2H), 2.11 (s, 3H), 1.60-1.54 (m, 2H), 1.43-1.35 (m, 2H), 1.33-1.28 (m, 3H), 0.88 (td, J = 14.9, 7.4 Hz, 4H), 0.60 (s, 2H), 0.34 (d, J = 6.0 Hz, 2H) | 13C-NMR (101 MHz, ACETONITRILE-D 3) δ 188.7, 157.9, 154.0, 153.4, 151.5, 144.9, 141.7, 133.5, 132.9, 130.2,128.2, 103.0, 55.9, 42.5, 41.0, 24.8, 21.1, 13.3, 12.9, 11.5, 8.2, 6.1, 5.9 |
| 126 | C₁₉H₂₁ClN₄O₆S | 469.0943 | 1H-NMR (400 MHz, ACETONITRILE-D3) δ 8.09 (t, J = 4.1 Hz, 1H), 7.67 (d, J = 8.0 Hz, 1H), 7.27 (s, 1H), 5.44 (s, 2H), 3.62 (s, 3H), 2.13 (d, J = 3.6 Hz, 3H), 1.34 (s, 9H) | 13C-NMR (101 MHz, ACETONITRILE-D 3) δ 187.5, 156.9, 153.7, 153.5, 143.6, 139.2, 138.4, 134.6, 134.1, 132.5, 128.4, 103.8, 61.9, 43.3, 32.9, 22.7, 11.5 |
| 127 | C₂₀H₂₃ClN₄O₆S | 483.11 | 1H-NMR (400 MHz, ACETONITRILE-D3) δ 8.11-8.07 (m, 1H), 7.69-7.64 (m, 1H), 7.31-7.26 (m, 1H), 5.44-5.41 (m, 2H), 4.05-3.97 (m, 2H), 2.15-2.11 (m, 3H), 1.38-1.32 (m, 12H) | 13C-NMR (101 MHz, ACETONITRILE-D 3) δ 187.7,156.4, 153.7, 153.5, 143.6, 139.3, 138.4, 134.6, 134.1, 132.5, 128.4, 103.9, 61.9, 43.3, 41.4, 22.7, 13.4, 11.4 |
| 128 | C₂₀H₂₃ClN₄O₆S | 483.11 | 1H-NMR (400 MHz, METHANOL-D4) δ 8.09 (d, J = 8.2 Hz, 1H), 7.57 (d, J = 8.0 Hz, 1H), 5.49 (d, J = 1.4 Hz, 2H), 3.45 (s, 3H), 2.16 (s, 3H), 2.11 (d, J = 2.5 Hz, 2H), 1.37 (s, 9H) | 13C-NMR (101 MHz, CHLOROFORM-D) δ 188.8, 159-3, 153.6, 153.4, 147.4, 144.4, 138.4, 134.6, 134.2, 132.6, 127.1, 102.8, 62.1, 43.2, 32.8, 23.7, 13.6, 12.2 |
| 129 | C₂₁H₂₅ClN₄O₆S | 497.1256 | 1H-NMR (400 MHz, METHANOL-D4) δ 8.09 (d, J = 8.2 Hz, 1H), 7.58 (d, J = 8.2 Hz, 1H), 5.49 (s, 2H), 3.88 (q, J = 7.2 Hz, 2H), 2.16 (t, J = 3.6 Hz, 3H), 2.12 (s, 3H), 1.37-1.34 (m, 9H), 1.30 (t, J = 7.1 Hz, 3H) | 13C-NMR (101 MHz, CHLOROFORM-D) δ 188.9, 158.7, 153.6, 153.4, 147.3, 144.4, 138.3, 134.6, 134.2, 132.6, 127.1, 102.8, 62.0, 43.2, 41.3, 23.7, 14.2, 13.7,12.2 |
| 138 | C₂₂H₂₅ClN₄O₆S | 509.1256 | 1H-NMR (400 MHz, CHLOROFORM-D) δ 8.12-8.10 (m, 1H), 7.57 (d, J = 8.2 Hz, 1H), 5.63-5.34 (m, 2H), 3.61 (s, 3H), 2.13 (t, J = 4.1 Hz, 3H), 1.38 (s, 9H), 0.97-0.90 (m, 1H), 0.67 (s, 2H), 0.41-0.34 (m, 2H) | 13C-NMR (101 MHz, CHLOROFORM-D) δ 188.9, 159.3, 153.6, 153.3, 152.2, 144.7, 138.2, 134.4, 134.3, 132.3, 127.5, 103.2, 62.1, 43.6, 43.3, 32.8, 23.8, 23.7, 12.4, 12.3, 8.4 6.6 |
| 139 | C₂₃H₂₇ClN₄O₆S | 523.1413 | 1H-NMR (400 MHz, METHANOL-D4) δ 8.15-8.04 (m, 1H), 7.65 (d, J = 8.2 Hz, 1H), 5.49 (t, J = 15.0 Hz, 2H), 3.95-3.84 (m, 2H), 2.19-2.10 (m, 3H), 1.74-1.59 (m, 1H), 1.37 (s, 9H), 1.28 (t, J = 7.1 Hz, 3H), 0.83-0.72 (m, 2H), 0.57 (d, J = 53.0 Hz, 2H) | 13C-NMR (101 MHz, CHLOROFORM-D) δ 188.8, 158.6, 153.5, 153.2, 151.8, 144.7, 138.0, 134.3, 134.2, 132.2, 127.4, 103.1, 62.0, 43.2, 41.3, 23.6, 14.0, 12.2, 8.5, 6.5 |
| 141 | C₂₄H₃₀ClN₅O₇S | 568.1627 | 1H-NMR (400 MHz, CHLOROFORM-D) δ 8.07 (d, J = 8.0 Hz, 1H), 7.43 (d, J = 8.0 Hz, 1H), 5.50 (s, 2H), 3.52 (s, 3H), 3.29 (s, 3H), 3.04-3.00 (m, 2H), 2.77 (t, J = 7.6 Hz, 2H), 2.21 (s, 3H), 2.11 (d, J = 2.7 Hz, 3H), 1.43 (td, J = 14.7, 7.2 Hz, 4H), 0.83-0.78 (m, 6H) | 13C-NMR (101 MHz, CHLOROFORM-D) δ 184.3, 152.8, 152.1, 149.4, 149.3, 146.9, 145.4, 142.0, 133.1, 131.4, 127.8, 127.1, 107.7, 49.2, 48.4, 44.3, 41.0, 33.5, 20.8, 20.0, 13.9, 11.3, 10.2, 10.1 |
| 142 | C₂₃H₃₂ClN₃O₇S | 582.1784 | 1H-NMR (400 MHz, CHLOROFORM-D) δ 8.02 (d, J = 8.2 Hz, 1H), 7.42 (d, J = 8.0 Hz, 1H), 5.46 (s, 2H), 3.52 (s, 3H), 3.41 (q, J = 7.4 Hz, 2H), 3.04-3.00 (m, 2H), 2.77 (t, J = 7.7 Hz, 2H), 2.20 (s, 3H), 2.09 (s, 3H), 1.44 (td, J = 14.6, 7.4 Hz, 4H), 1.27-1.24 (m, 3H), 0.84-0.78 (m, 6H) | 13C-NMR (101 MHz, CHLOROFORM-D) δ 185.5, 153.8, 153.3,150.5,150.4, 148.0, 146.4, 141.1, 134.3, 132.8, 130.0, 127.8, 108.8, 51.1, 50.3, 49.5, 42.3, 34.6,21.9,21.1, 15.0, 12.4, 11.2, 11.2, 7.3 |
| 143 | C₂₆H₃₄ClN₃O₇S | 596.194 | 1H-NMR (400 MHz, CHLOROFORM-D) δ 8.03-7.98 (m, 1H), 7.44-7.38 (m, 1H), 5.46-5.39 (m, 2H), 3.67-3.60 (m, 1H), 3.53 (d, J = 8.5 Hz, 3H), 3.04-2.99 (m, 2H), 2.78 (q, J = 7.4 Hz, 2H), 2.25-2.17 (m, 3H), 2.11-2.08 (m, 3H), 1.44 (qd, J = 15.1, 7.6 Hz, 4H), 1.33-1.27 (m, 6H), 0.82 (qd, J = 7.5, 3.9 Hz, 6H) | 13C-NMR (101 MHz, CHLOROFORM-D) δ 185.6,153.8, 153.3, 150.5, 150.4, 148.0, 146.3, 140.5, 134.3, 133.0, 130.5, 127.6, 108.8, 55.8, 50.3, 49.5, 42.6, 34.6, 21.9, 21.1, 15.2, 15.0, 12.4, 11.2 |
| 144 | C₂₆H₃₂ClN₅O₇S | 594.1784 | 1H-NMR (400 MHz, CHLOROFORM-D) δ 8.07 (d, J = 8.0 Hz, 1H), 7.47 (d, J = 8.2 Hz, 1H), 5.50 (s, 2H), 3.50 (s, 3H), 3.28 (s, 3H), 3.05 (t, J = 7.7 Hz, 2H), 2.85 (t, J = 7.6 Hz, 2H), 2.10 (s, 3H), 1.94-1.89 (m, 1H), 1.50-1.42 (m, 4H), 0.86-0.78 (m, 10H) | 13C-NMR (101 MHz, CHLOROFORM-D) δ 185.6, 154.9, 153.8, 153.2, 150.6, 147.9, 146.7, 143.0, 134.3. 132.4, 128.8. 128.4, 109.2, 50.3, 45.5, 42.1, 34.6, 21.9, 21.1, 12.4, 11.3, 11.2, 9.1, 7.9 |
| 149 | C₂₄H₂₈ClN₅O₇S | 566.1471 | 1H-NMR (400 MHz, CHLOROFORM-D) δ 8.09 (d, J = 8.0 Hz, 1H), 7.47 (d, J = 8.0 Hz, 1H), 5.57-5.53 (m, 2H), 3.56 (s, 3H), 3.33 (s, 3H), 3.24-3.17 (m, 2H), 3.06-2.95 (m, 2H), 2.34-2.29 (m, 3H), 2.14 (s, 3H), 1.64-1.49 (m, 8H) | 13C-NMR (101 MHzCHLOROFORM -D) δ 185.3, 153.9, 153.2, 150.6, 150.3, 147.9, 146.2, 143.0, 134.2, 132.3, 128.7, 128.4, 108.7, 48.0, 47.4, 45.4, 42.0, 34,4, 28.5, 27.5, 27.1, 26.7, 15.0, 12.4 |
| 150 | C₂₅H₃₀ClN₅O₇S | 580.1627 | 1H-NMR (400 MHz, CHLOROFORM-D) δ 8.01 (t, J = 4.1 Hz, 1H), 7.42 (d, J = 8.0 Hz, 1H), 5.46 (t, J = 14.4 Hz, 2H), 3.53 (s, 3H), 3.44-3.39 (m, 2H), 3.20 (t, J = 5.9 Hz, 2H), 3.02-2.92 (m, 2H), 2.29 (s, 3H), 2.11 (d, J = 7.4 Hz, 3H), 1.66-1.46 (m, 8H), 1.26 (t, J = 7.4 Hz, 3H) | 13C-NMR (101 MHz, CHLOROFORM-D) δ 185.5, 153.9, 153.3, 150.7, 150.3, 148.0, 146.2, 141.0, 134.3, 132.6, 129.8, 128.0, 108.8, 51.1, 48.0, 47.4, 42.3, 34.4, 28.6, 27.5, 27.2, 26.7, 15.1, 12.4, 7.3 |
| 151 | C₂₆H₃₂ClN₃O₇S | 594.1784 | 1H-NMR (400 MHz, CHLOROFORM-D) δ 7.99 (t, J = 4.0 Hz, 1H), 7.41 (d, J = 8.0 Hz, 1H), 5.43 (t, J = 14.8 Hz, 2H), 3.67-3.60 (m, 1H), 3.52 (s, 3H), 3.27-3.19 (m, 2H), 2.97 (t, J = 5.4 Hz, 2H), 2.29 (s, 3H), 2.08 (d, J = 4.1 Hz, 3H), 1.61-1.46 (m, 8H), 1.30 (d, J = 6.6 Hz, 6H) | 13C-NMR (101 MHz, CHLOROFORM-D) δ 185.5, 153.8, 153.3, 150.6, 150.3, 148.0, 146.1, 140.5, 134.3, 132.8, 130.2, 127.3, 108.8, 55.8, 48.0, 47.4, 42.6, 34.4, 28.6, 27.5, 27.2, 26.7, 15.2, 15.0, 12.4 |
| 152 | C₂₆H₃₀ClN₅O₇S | 592.1627 | 1H-NMR (400 MHz, CHLOROFORM-D) δ 8.06 (d, J = 8.2 Hz, 1H), 7.47 (d, J = 8.2 Hz, 1H), 5.51 (s, 2H), 3.49 (s, 3H), 3.45-3.33 (m, 1H), 3.29 (s, 3H), 3.26-3.21 (m, 2H), 3.02 (t, J = 5.6 Hz, 2H), 2.11 (s, 3H), 1.65-1.47 (m, 8H), 0.87-0.83 (m, 2H), 0.76-0.71 (m, 2H) | 13C-NMR (101 MHz, CHLOROFORM-D) δ 185.5, 155.2, 153.9, 153.2, 150.4, 147.8, 146.5, 142.9, 134.4, 132.2, 128.7, 128.5, 109.1, 48.0, 47.6, 47.5, 45.4, 42.1, 34.5, 28.5, 27.5, 27.2, 26.7, 12.4, 9.1, 8.1 |
| 153 | C₂₇H₃₂ClN₅O₇S | 606.1784 | 1H-NMR (400 MHz, CHLOROFORM-D) δ 8.01 (d, J = 8.0 Hz, 1H), 7.46 (d, J = 8.2 Hz, 1H), 5.46 (s, 2H), 3.49 (s, 3H), 3.41 (q, J = 7.4 Hz, 2H), 3.23 (t, J = 5.9 Hz, 2H), 3.03 (t, J = 5.6 Hz, 2H), 2.09 (s, 4H), 1.61-1.44 (m, 8H), 1.26 (t, J = 7.4 Hz, 3H), 0.87-0.83 (m, 2H), 0.76-0.71 (m, 2H) | 13C-NMR (101 MHz, CHLOROFORM-D) δ 185.7, 155.3, 153.8,153.3,150.4, 146.5, 140.9, 134.4, 132.5, 129.8, 128.2, 109.1, 51.1, 48.0, 47.5, 42.3, 34.5, 28.6, 27.5, 27.2, 26.7, 12.4, 9.1, 8.2, 7.3 |
| 154 | C₂₃H₂₃ClN₆O₇S | 563.111 | 1H-NMR (400 MHz, CHLOROFORM-D) δ 7.93 (td, J = 15.0, 8.2 Hz, 1H), 7.47-7.33 (m, 2H), 5.55-5.31 (m, 2H), 3.86-3.79 (m, 3H), 3.63-3.54 (m, 3H), 3.28-3.18 (m, 3H), 2.49-2.41 (m, 3H), 2.39-2.28 (m, 3H), 2.19-2.10 (m, 3H) | 13C-NMR (101 MHz, CHLOROFORM-D) δ 185.2, 158.1, 153.9, 153.2, 152.5, 151.1, 147.1, 146.2, 142.7, 134.9, 134.0, 132.4, 128.8, 128.1, 108.5, 107.4, 45.3, 41.7, 39.6, 34.8, 15.2, 13.4, 12.4 |
| 155 | C₂₄H₂₅ClN₆O₇S | 577.1267 | 1H-NMR (400 MHz, CHLOROFORM-D) δ 7.90 (d, J = 8.2 Hz, 1H), 7.44 (s, 1H), 7.40 (d, J = 8.2 Hz, 1H), 5.34 (s, 2H), 3.86 (s, 3H), 3.61 (s, 3H), 3.36 (q, J = 7.3 Hz, 2H), 2.47 (s, 3H), 2.39 (s, 3H), 2.16 (s, 3H), 1.26 (t, J = 7.4 Hz, 3H) | 13C-NMR (101 MHz, CHLOROFORM-D) δ 185.4,158.1, 153.8,153.2, 152.6, 151.1, 147.2, 146.1, 140.8, 134.9, 134.1, 132.7, 129.9, 127.8, 108.5, 107.4, 51.1, 42.0, 39.6, 34.8, 15.1, 13.4, 12.4, 7.2 |
| 156 | C₂₅H₂₇ClN₆O₇S | 591.1423 | 1H-NMR (400 MHz, CHLOROFORM-D) δ 7.90-7.79 (m, 1H), 7.42-7.29 (m, 2H), 5.53-5.27 (m, 2H), 3.89-3.74 (m, 3H), 3.63-3.51 (m, 3H), 3.43-3.23 (m, 2H), 2.50-2.40 (m, 3H), 2.38-2.25 (m, 3H), 2.16-2.08 (m, 3H), 1.72-1.57 (m, 2H), 1.03-0.90 (m, 3H) | 13C-NMR (101 MHz, CHLOROFORM-D) δ 185.4,158.1, 153.8, 153.2, 152.5, 151.1, 147.2, 146.1, 141.3, 134.8, 134.0, 132.6, 129.7, 127.8, 108.5, 107.3, 58.1, 41.9, 39.6, 34.8, 16.6, 15.1, 13.4, 12.8, 12.4 |
| 157 | C₂₅H₂₇ClN₆O₇S | 591.1423 | 1H-NMR (400 MHz, CHLOROFORM-D) δ 7.87 (dd, J = 15.1, 6.9 Hz, 1H), 7.43 (s, 1H), 7.41-7.36 (m, 1H), 5.34-5.31 (m, 2H), 3.87 (s, 3H), 3.60 (s, 3H), 3.58-3.49 (m, 1H), 2.47 (s, 3H), 2.39 (s, 3H), 2.15 (t, J = 4.2 Hz, 3H), 1.34-1.25 (m, 6H) | 13C-NMR (101 MHz, CHLOROFORM-D) δ 185.4, 158.1, 153.8, 153.3, 152.6, 151.1, 147.2, 146.0, 140.1, 134.8, 134.1, 132.9, 130.4, 127.5, 108.5, 107.3, 55.9, 53.5, 42.4, 39.6, 34.8, 15.1, 13.3, 12.4 |
| 158 | C₂₂H₂₁ClN₆O₇S | 549.0954 | 1H-NMR (400 MHz, CHLOROFORM-D) δ 7.99 (d, J = 8.2 Hz, 1H), 7.74 (s, IH), 7.63 (s, 1H), 7.43 (d, J = 8.2 Hz, 1H), 5.38 (s, 2H), 3.82 (s, 3H), 3.66 (s, 3H), 3.22 (s, 3H), 2.36 (s, 3H), 2.11 (s, 3H) | 13C-NMR (101 MHz, CHLOROFORM-D) δ 184.7, 158.0, 153.9, 153.2, 152.8, 146.9, 145.2, 143.2, 140.8, 135.4, 134.2, 132.6, 128.8, 128.4, 110.7, 108.0, 45.4, 41.9, 39.6, 35.2, 13.4, 12.4 |
| 159 | C₂₃H₂₃ClN₆O₇S | 563.111 | 1H-NMR (400 MHz, CHLOROFORM-D) δ 7.99 (d, J = 8.2 Hz, 1H), 7.82 (s, 1H), 7.67 (s, 1H), 7.49 (d, J = 8.2 Hz, 1H), 5.41 (s, 2H), 3.89 (s, 3H), 3.72 (s, 3H), 3.40 (q, J = 7.4 Hz, 2H), 2.43 (d, J = 6.6 Hz, 3H), 2.17-2.15 (m, 3H), 1.29 (t, J = 7.4 Hz, 3H) | 13C-NMR (101 MHz, CHLOROFORM-D) δ 184.8, 158.0, 153.8, 153.2, 152.8, 146.9, 145.2, 141.3, 140.8, 135.4, 134.3, 132.9, 129.8, 128.0, 110.7, 107.9, 51.2, 42.2, 39.5, 35.2, 13.4, 12.4, 7.2 |
| 160 | C₂₄H₂₅ClN₆O₇S | 577.1267 | 1H-NMR (400 MHz, CHLOROFORM-D) δ 7.99 (d, J = 8.2 Hz, 1H), 7.83 (s, 1H), 7.66 (s, 1H), 7.48 (d, J = 8.0 Hz, 1H), 5.41 (s, 2H), 3.89 (s, 3H), 3.72 (s, 3H), 3.38-3.34 (m, 2H), 2.44 (s, 3H), 2.17 (d, J = 4.4 Hz, 3H), 1.71 (q, J = 7.8 Hz, 2H), 1.01 (t, J = 7.4 Hz, 3H) | 13C-NMR (101 MHz, CHLOROFORM-D) δ 184.8, 158.0, 153.8, 153.2, 152.8, 146.9, 145.1, 141.8, 140.8, 135.3, 134.3, 132.8, 129.7, 128.1, 110.7, 107.9, 58.2, 42.1, 39.6, 35.2, 16.6, 13.4, 12.9, 12.4 |
| 161 | C₂₄H₂₅ClN₆O₇S | 577.1267 | 1H-NMR (400 MHz, CHLOROFORM-D) δ 7.98-7.87 (m, 1H), 7.83-7.77 (m, 1H), 7.63-7.59 (m, 1H), 7.49-7.41 (m, 1H), 5.40-5.31 (m, 2H), 3.88-3.84 (m, 3H), 3.69 (t, J = 15.0 Hz, 3H), 3.60-3.55 (m, 1H), 2.43-2.36 (m, 3H), 2.16-2.11 (m, 3H), 1.32-1.26 (m, 6H) | 13C-NMR (101 MHz, CHLOROFORM-D) δ 184.9, 158.0, 153.8, 153.3, 152.9, 146.9, 145.0, 140.8, 140.6, 135.3, 134.3, 133.1, 130.4, 127.8, 110.8, 107.8, 55.9, 42.5, 39.6, 35.2, 15.2, 13.4, 12.5 |
| 162 | C₂₅H₂₅ClN₆O₇S | 589.1267 | 1H-NMR (400 MHz, CHLOROFORM-D) δ 7.97 (dd, J = 15.1, 6.9 Hz, 1H), 7.47 (d, J = 4.3 Hz, 1H), 7.46-7.43 (m, 1H), 5.46-5.34 (m, 2H), 3.86 (d, J = 5.2 Hz, 3H), 3.56 (s, 3H), 3.25 (d, J = 4.7 Hz, 3H), 2.47-2.40 (m, 1H), 2.38 (s, 3H), 2.16 (d, J = 4.4 Hz, 3H), 1.03-0.97 (m, 2H), 0.95-0.89 (m, 2H) | 13C-NMR (101 MHz, CHLOROFORM-D) δ 185.4,158.1, 156.0, 153.8, 153.2, 152.5, 146.9, 146.4, 142.7, 135.0, 134.1, 132.4, 128.7, 128.3, 108.8, 107.6, 53.5, 45.3, 41.7, 39.6, 34.8, 13.4, 12.4, 9.1 8.6 |
| 163 | C₂₆H₁₇ClN₆O₇S | 603.1423 | 1H-NMR (400 MHz, ACETONITRILE-D3) δ 7.88 (d, J = 8.2 Hz, 1H), 7.67 (s, 1H), 7.48 (d, J = 8.2 Hz, 1H), 5.30 (s, 2H), 3.77 (s, 3H), 3.49 (s, 3H), 3.31 (d, J = 7.4 Hz, 2H), 2.42-2.50 (1H), 2.27 (s, 3H), 2.13 (d, J = 1.9 Hz, 3H), 1.14 (t, J = 7.3 Hz, 3H), 0.93-0.90 (m, 4H) | |
| 164 | C₂₇H₂₉ClN₆O₇S | 617.158 | 1H-NMR (400 MHz, ACETONITRILE-D3) δ 7.88 (d, J = 8.0 Hz, 1H), 7.67 (s, 1H), 7.48 (d, J = 8.2 Hz, 1H), 5.32 (d, J = 14.8 Hz, 2H), 3.77 (s, 3H), 3.49 (d, J = 4.1 Hz, 3H), 3.31-3.27 (m, 2H), 2.49-2.42 (m, 1H), 2.28 (d, J = 5.2 Hz, 3H), 2.12 (d, J = 4.4 Hz, 3H), 1.63-1.53 (m, 2H), 0.97-0.92 (m, 4H), 0.91 (t, J = 2.3 Hz, 3H) | 13C-NMR (101 MHz, CHLOROFORM-D) δ 185.5, 158.1, 156.0, 153.8, 153.2, 152.6, 146.9, 146.3, 141.2, 134.9, 134.2, 132.6, 129.7, 127.9, 108.9, 107.5, 58.2, 41.9, 39.6, 34.8, 16.6, 13.4, 12.8, 12.4, 9.1, 8.6 |
| 165 | C₂₇H₂₉ClN₆O₇S | 617.158 | 1H-NMR (400 MHz, CHLOROFORM-D) δ 7.93-7.77 (m, 1H), 7.50-7.42 (m, 1H), 7.38 (dd, J = 15.0, 8.1 Hz, 1H), 5.39-5.28 (m, 2H), 3.88-3.80 (m, 3H), 3.60-3.49 (m, 4H), 2.46-2.33 (m, 4H), 2.16-2.09 (m, 3H), 1.33-1.22 (m, 6H), 1.02-0.94 (m, 2H), 0.93-0.82 (m, 2H) | 13C-NMR (101 MHz, CHLOROFORM-D) δ 185.6, 158.1, 156.0, 153.8, 153.3 152.6, 146.9, 146.2 140.0, 134.9, 134.2, 132.8, 130.4, 127.6, 108.9, 107.5, 55,9, 53.6, 42.4, 39.6, 34.9, 15.1, 13.4, 12.5, 9.0, 8.6 |
| 166 | C₂₃H₂₀ClF₃N₆O₇ S | 617.0828 | 1H-NMR (400 MHz, CHLOROFORM-D) δ 7.84 (d, J = 8.0 Hz, 1H), 7.71 (s, 1H), 7.37-7.33 (m, 1H), 5.34 (s, 2H), 3.92 (s, 3H), 3.54 (s, 3H), 3.18 (s, 3H), 2.39 (d, J = 7.6 Hz, 3H), 2.11-2.08 (m, 3H) | 13C-NMR (101 MHz, CHLOROFORM-D) δ 185.6, 158.1, 156.0, 153.8, 153.3, 152.6,146.9,146.2, 140.0, 134.9, 134.2, 132.8, 130.4, 127,6, 108.9, 107.5, 55.9, 53.6, 42.4, 39.6, 34.9, 15.1, 13.4, 12.5, 9.0, 8.6 |
| 167 | C₂₄H₂₂ClF₃N₆O₇ S | 631.0984 | 1H-NMR (400 MHz, CHLOROFORM-D) δ 7.78 (d, J = 8.2 Hz, 1H), 7.69 (s, 1H), 7.33 (d, J = 8.0 Hz, 1H), 5.31 (t, J = 13.9 Hz, 2H), 3.93 (s, 3H), 3.54 (s, 3H), 3.29 (q, J = 7.3 Hz, 2H), 2.40 (s, 3H), 2.10 (d, J = 2.7 Hz, 3H), 1.19 (t, J = 7.4 Hz, 3H) | 13C-NMR (101 MHz, CHLOROFORM-D) δ 185.2, 155.8, 153.8, 153.2, 151.2, 146.2, 145.7, 142.1, 140.9, 137.2, 134.4, 132.8, 129.8, 127.7, 121.2, 108.5, 108.1, 51.1, 42.2, 40.6, 34.8, 15.1, 12.4, 7.2 |
| 168 | C₂₃H₂₄ClF₃N₆O₇ S | 645.1141 | 1H-NMR (400 MHz, CHLOROFORM-D) δ 7.78 (t, J = 4.0 Hz, 1H), 7.67 (s, 1H), 7.32 (d, J = 8.2 Hz, 1H), 5.31 (t, J = 14.0 Hz, 2H), 3.92 (s, 3H), 3.54 (s, 3H), 3.25 (t, J = 8.0 Hz, 2H), 2.40 (s, 3H), 1.73-1.53 (m, 3H), 1.31-1.16 (m, 2H), 0.93 (t, J = 7.4 Hz, 3H) | 13C-NMR (101 MHz, CHLOROFORM-D) δ 185.2,155.8, 153.8, 153.2, 151.2, 146.2, 145.7, 141.4, 137.2, 134.4, 132.7, 129.6, 127.7, 108.5, 108.1, 58.1, 42.1, 40.6, 34.7, 16.7, 15.1, 14.2, 12.8, 12.3 |
| 169 | C₂₅H₂₄ClF₃N₆O₇ S | 645.1141 | 1H-NMR (400 MHz, CHLOROFORM-D) δ 7.75 (d, J = 8.0 Hz, 1H), 7.71 (d, J = 7.4 Hz, 1H), 7.32 (dd, J = 15.1, 6.9 Hz, 1H), 5.35-5.28 (m, 2H), 3.94 (d, J = 8.5 Hz, 3H), 3.54 (s, 3H), 3.51-3.44 (m, 1H), 2.40 (s, 3H), 2.09 (s, 3H), 1.23 (d, J = 6.3 Hz, 6H) | 13C-NMR (101 MHz, CHLOROFORM-D) δ 185.2, 155.8. 153.8, 153.2, 151.2, 146.2, 145.6, 142.1, 14 1.7, 140.3, 137.2, 134.4, 133.0, 130.3, 127.4, 121.3, 118.6, 108.4, 108.0, 55,9, 42.5, 40.6, 34.8, 15.1, 12.4 |
| 170 | C₂₂H₁₈ClF₃N₆O₇ S | 603.0671 | 1H-NMR (400 MHz, CHLOROFORM-D) δ 8.02 (d, J = 8.2 Hz, 1H), 7.96 (s, 1H), 7.79 (s, 1H), 7.49 (d, J = 8.2 Hz, 1H), 5.47 (s, 2H), 4.01 (s, 3H), 3.73 (d, J = 4.1 Hz, 3H), 3.28 (d, J = 2.7 Hz, 3H), 2.17 (d, J = 2.5 Hz, 3H) | 13C-NMR (101 MHz, CHLOROFORM-D) δ 184.5, 155.6, 153.9, 153.2, 146.0, 144.9, 143.3, 142.4, 140.9, 137.7, 134.5, 132.7, 128.7, 128.4, 121.3, 110.6, 108.8, 45.3, 42.1, 40.4, 35.1, 12.4 |
| 171 | C₂₄H₂₂ClF₃N₆O₇ S | 631.0984 | 1H-NMR (400 MHz, CHLOROFORM-D) δ 7.96 (s, 1H), 7.83 (s, 1H), 7.49 (d, J = 8.0 Hz, 1H), 5.44 (s, 2H), 4.14 (d, J = 1.9 Hz, 1H), 4.03 (s, 2H), 4.02 (s, 3H), 3.73 (s, 3H), 3.40 (q, J = 7.4 Hz, 2H), 2.17 (s, 3H), 1.29 (t, J = 7.4 Hz, 3H) | 13C-NMR (101 MHz, CHLOROFORM-D) δ 183.5, 154.4, 152.8, 152.1, 144.9, 143.7, 140.3, 139.7, 136.8, 136.5, 133.4, 132.0, 128.7, 126.9, 117.5, 109.5, 107.5, 50.0, 41.2, 39.4, 39.1, 34.0, 11.3, 6.1 |
| 172 | C24H22ClF3N6 O7S | 631.0984 | 1H-NMR (400 MHz, CHLOROFORM-D) δ 7.90-7.87 (m, 2H), 7.76 (s, 1H), 7.40 (d, J = 8.2 Hz, 1H), 5.37 (s, 2H), 3.95 (s, 3H), 3.66 (s, 3H), 3.30-3.26 (m, 2H), 2.09 (d, J = 3.6 Hz, 3H), 1.67-1.62 (m, 2H), 0.96-0.92 (m, 3H) | 13C-NMR (101 MHz, CHLOROFORM-D) δ 184.6,155.6, 153.8, 153.2, 146.0, 144.8, 141.9, 140.8, 137.6, 134.5, 133.0, 129.8, 129.6, 128.0, 121.3, 110.6, 108.7, 58.2,42.3,40.5, 35.1, 16.6, 12.8, 12.4 |
| 173 | C24H22ClF3NG O7S | 631.0984 | 1H-NMR (400 MHz, CHLOROFORM-D) δ 7.87-7.84 (m, 2H), 7.78 (s, 1H), 7.40-7.38 (m, 1H), 5.33 (s, 2H), 3.95 (s, 3H), 3.66 (s, 3H), 3.55-3.47 (m, 1H), 2.09 (s, 3H), 1.27-1.24 (m, 6H) | 13C-NMR (101 MHz, CHLOROFORM-D) δ 184.6, 155.6, 153.8, 153.3, 145.9, 144.7, 140.8, 140.7, 137.6, 134.5, 133.3, 130.4, 130.3, 127.7, 121.3, 118.6, 110.7, 108.6, 55.9, 42.6, 40.5, 35.2, 15.1, 12.4 |
| 174 | C25H22C1F3N6 O7S | 643.0984 | 1H-NMR (400 MHz, CHLOROFORM-D) δ 7.94-7.90 (m, 1H), 7.79 (s, 1H), 7.46-7.43 (m, 1H), 5.43 (s, 2H), 3.99 (s, 3H), 3.57 (s, 3H), 3.26 (s, 3H), 2.35 (dd, J = 21.3, 4.8 Hz, 1H), 2.17 (s, 3H), 1.00-0.96 (m, 2H), 0.95-0.87 (m, 2H) | 13C-NMR (101 MHz, CHLOROFORM-D) δ 185.2,156.0, 155.8, 153.9, 153.2, 146.0, 145.9, 142.8, 142.1, 141.7, 137.3, 134.4, 132.5, 128.7, 128.2, 121.2, 108.8, 108.3, 45.2, 41.9, 40.5, 34.8, 12.4, 9.1, 8.6 |
| 175 | C26H24C1F3N6 O7S | 657.1141 | 1H-NMR (400 MHz, CHLOROFORM-D) δ 7.84 (dd, J = 8.1, 3.2 Hz, 1H), 7.76 (s, 1H), 7.41 (d, J = 8.2 Hz, 1H), 5.36 (s, 2H), 3.97 (d, J = 4.7 Hz, 3H), 3.54 (s, 3H), 3.34 (q, J = 7.4 Hz, 2H), 2.47-2.29 (m, 1H), 2.14 (d, J = 2.7 Hz, 3H), 1.24 (t, J = 7.3 Hz, 3H), 1.00-0.94 (m, 2H), 0.93-0.87 (m, 2H) | 13C-NMR (101 MHz, CHLOROFORM-D) δ 185.3, 156.1, 155.8, 153.8, 153.2, 145.9, 141.8, 140.9, 137.3, 134.5, 132.8, 129.8, 127.8, 121.3, 118.6, 108.8, 108.3, 51.1, 42.2, 40.5, 34.8, 12.4, 9.1, 8.6, 7.2, 2.0 |
| 176 | C27H26ClF3N6 O7S | 671.1297 | 1H-NMR (400 MHz, ACETONITRILE-D3) δ 7.98 (s, 1H), 7.87 (d, J = 8.0 Hz, 1H), 7.49 (d, J = 8.0 Hz, 1H), 5.32 (s, 2H), 3.93 (s, 3H), 3.53 (d, J = 15.1 Hz, 3H), 3.31 (t, J = 8.0 Hz, 2H), 2.41 (t, J = 6.5 Hz, 1H), 2.12 (s, 3H), 1.59 (q, J = 7.7 Hz, 2H), 0.95 (t, J = 7.4 Hz, 3H), 0.90 (d, J = 6.3 Hz, 4H) | 13C-NMR (101 MHz, ACETONITRILE-D 3) δ 185.3,155.8, 155.3, 154.2, 153.4, 146.1, 145.6, 141.5, 138.0, 133.4, 132.8, 130.1, 127.8, 108.4, 107.6, 89.2, 57.4, 42.1, 39.9, 34.4, 16.4, 12.0, 11.5, 8.6, 8.0 |
| 177 | C27H26ClF3N6 O7S | 671.1297 | 1H-NMR (400 MHz, CHLOROFORM-D) δ 7.82 (dd, J = 15.0, 8.1 Hz, 1H), 7.74 (dd, J = 22.3, 7.1 Hz, 1H), 7.44-7.34 (m, 1H), 5.38-5.29 (m, 2H), 4.04-3.89 (m, 3H), 3.60-3.44 (m, 4H), 2.38 (s, 1H), 2.19-2.07 (m, 3H), 1.26 (dd, J = 21.8, 15.0 Hz, 6H), 1.01-0.92 (m, 2H), 0.91-0.84 (m, 2H) | 13C-NMR (101 MHz, CHLOROFORM-D) δ 185.4, 156.1, 155.8, 153.8, 153.2 145.9.145.8,142.2, 141.8, 140.2, 137.2 134.4, 133.0, 130.3, 127.5, 121.3, 108.8, 108.2, 77.5, 77.2, 76.8, 55.9, 53.6, 42.5, 40.5, 34.8, 15.1, 12.4, 9.0, 8.6 |
| 178 | C24H25CLN6O 7S | 577.1267 | 1H-NMR (400 MHz, CHLOROFORM-D) δ 7.90-7.79 (m, 1H), 7.58-7.50 (m, 1H), 7.39-7.28 (m, 1H), 5.60-5.28 (m, 2H), 4.16-4.04 (m, 2H), 3.59 (t, J = 15.0 Hz, 3H), 3.28-3.13 (m, 3H), 2.53-2.45 (m, 3H), 2.41-2.33 (m, 3H), 2.17-2.10 (m, 3H), 1.43-1.36 (m, 3H) | 13C-NMR (101 MHz, CHLOROFORM-D) δ 185.4, 158.4, 153.8, 153.1, 151.2, 147.2, 146.1, 144.8, 142.5, 140.4, 133.9, 132.5, 128.6, 128.4, 108.5, 106.9, 45.4, 44.8, 41.6, 34.7, 15.2, 14.7, 12.4, 10.1 |
| 179 | C26H29ClN6O 7S | 605.158 | 1H-NMR (400 MHz, CHLOROFORM-D) δ 7.76 (d, J = 8.0 Hz, 1H), 7.54 (s, 1H), 7.28 (d, J = 8.2 Hz, 1H), 5.24 (d, J = 7.4 Hz, 2H), 4.05 (q, J = 7.3 Hz, 2H), 3.54 (d, J = 4.1 Hz, 3H), 3.25 (t, J = 8.0 Hz, 2H), 2.44 (s, 3H), 2.32 (s, 3H), 2.07 (s, 3H), 1.61-1.55 (m, 2H), 1.35 (t, J = 7.3 Hz, 3H), 0.91 (t, J = 7.4 Hz, 3H) | 13C-NMR (101 MHz, CHLOROFORM-D) δ 185.6, 158.3, 153.7, 153.1, 151.2, 147.2, 146.0, 144.7, 141.1, 140.4, 134.0, 132.7, 129.5, 128.1, 108.6,106.9,58.1, 44.8, 41.8, 34.6, 16.6, 15.2, 14.6, 12.8, 12.3, 10.1 |
| 180 | C23H23ClN6O 7S | 563.111 | 1H-NMR (400 MHz, CHLOROFORM-D) δ 7.90 (d, J = 8.2 Hz, 1H), 7.84 (s, 1H), 7.66 (s, 1H), 7.37 (d, J = 8.2 Hz, 1H), 5.36 (s, 2H), 4.07 (q, J = 7.3 Hz, 2H), 3.65 (s, 3H), 3.19 (s, 3H), 2.38 (s, 3H), 2.09 (s, 3H), 1.37 (t, J = 7.3 Hz, 3H) | 13C-NMR (101 MHz, CHLOROFORM-D) δ 184.7, 158.3, 153.8, 153.2, 146.9, 145.3, 144.8, 143.0, 140.8, 140.7, 134.2, 132.7, 128.6, 128.5, 110.9, 107.4, 45.4, 44.8, 41.3, 35.0, 14.8, 12.4, 10.3 |
| 181 | C25H27ClN6O 7S | 591.1423 | 1H-NMR (400 MHz, CHLOROFORM-D) δ 7.94-7.91 (m, 2H), 7.76 (s, 1H), 7.43 (d, J = 8.2 Hz, 1H), 5.38 (s, 2H), 4.14 (q, J = 7.3 Hz, 2H), 3.73 (d, J = 4.1 Hz, 3H), 3.37-3.33 (m, 2H), 2.45 (s, 3H), 2.16 (d, J = 4.7 Hz, 3H), 1.73-1.63 (m, 2H), 1.44 (t, J = 7.3 Hz, 3H), 1.00 (t, J = 7.4 Hz, 3H) | 13C-NMR (101 MHz, CHLOROFORM-D) δ 184.9, 158.3, 153.8, 153.2, 146.9, 145.1, 144.8, 141.6, 140.7, 140.7, 134.2, 132.9, 129.6, 128.2, 110.9, 107.3, 58.1, 44.8,41.9,35.0, 16.6, 14.8, 12.8, 12.4, 10.3 |
| 182 | C26H27ClN6O 7S | 603.1423 | 1H-NMR (400 MHz, CHLOROFORM-D) δ 7.82 (d, J = 8.2 Hz, 1H), 7.51 (s, 1H), 7.32 (d, J = 8.2 Hz, 1H), 5.32 (s, 2H), 4.05 (q, J = 7.3 Hz, 2H), 3.49 (s, 3H), 3.16 (s, 3H), 2.49-2.44 (m, 1H), 2.34 (s, 3H), 2.09 (d, J = 3.8 Hz, 3H), 1.36 (q, J = 7.5 Hz, 3H), 0.96-0.92 (m, 2H), 0.91-0.87 (m, 2H) | 13C-NMR (101 MHz, CHLOROFORM-D) δ 185.5, 158.4 156.2, 153.8, 153.2, 146.9, 146.3, 144.7, 142.5, 140.4, 134.0, 132.5, 128.5, 128.4, 108.9, 107.1, 45.4, 44.8,41.6,34.7, 14.7, 12.3, 10.2, 9.0, 8.7, 2.0 |
| 183 | C28H31ClN6O 7S | 631.1736 | 1H-NMR (400 MHz, CHLOROFORM-D) δ 7.77 (d, J = 8.0 Hz, 1H), 7.55 (s, 1H), 7.31 (d, J = 8.0 Hz, 1H), 5.27 (d, J = 1.6 Hz, 2H), 4.05 (q, J = 7.3 Hz, 2H), 3.50 (s, 3H), 3.25 (t, J = 8.0 Hz, 2H), 2.46-2.42 (m, 1H), 2.33 (s, 3H), 2.07 (t, J = 4.0 Hz, 3H), 1.61-1.56 (m, 2H), 1.35 (t, J = 7.3 Hz, 3H), 0.97-0.86 (m, 7H) | 13C-NMR (101 MHz, CHLOROFORM-D) δ 185.7. 158.4, 153.7, 153.2, 147.0, 146.2, 144.67, 141.0, 140.4. 134.1, 132.7, 129.5, 128.1, 108.9, 107.0, 58.1, 44.8, 41.8, 34.7, 16.6, 14.6, 12.8, 10.1, 9.0, 8.7 |
| 184 | C25H24ClF3N6 O7S | 645.1141 | 1H-NMR (400 MHz, CHLOROFORM-D) δ 7.79 (dd, J = 14.8, 6.9 Hz, 1H), 7.69 (t, J = 15.0 Hz, 1H), 7.33-7.27 (m, 1H), 5.45-5.27 (m, 2H), 4.41-4.31 (m, 2H), 3.56 (s, 3H), 3.29 (td, J = 14.9, 7.5 Hz, 2H), 2.37 (t, J = 15.0 Hz, 3H), 2.11-2.07 (m, 3H), 1.44 (q, J = 7.4 Hz, 3H), 1.17 (t, J = 7.3 Hz, 3H) | 13C-NMR (101 MHz, CHLOROFORM-D) δ 185.3,155.5, 153.8, 153.2, 151.1, 146.5, 145.8, 141.7, 140.9, 134.0, 133.3, 132.9,130.0,127.7, 120.3, 110.1, 108.4, 51.0, 49.3, 41.9, 34.8, 15.4, 15.1, 12.4, 7.2 |
| 185 | C26H26ClF3N6 O7S | 659.1297 | 1H-NMR (400 MHz, CHLOROFORM-D) δ 7.78 (d, J = 8.0 Hz, 1H), 7.72 (s, 1H), 7.29 (d, J = 8.0 Hz, 1H), 5.28 (s, 2H), 4.37 (q, J = 7.2 Hz, 2H), 3.56 (s, 3H), 3.54-3.49 (m, 1H), 2.35 (s, 3H), 2.07 (s, 3H), 1.43 (t, J = 7.3 Hz, 3H), 1.20 (d, J = 6.9 Hz, 6H) | 13C-NMR (101 MHz, CHLOROFORM-D) δ 185.4, 155,5, 153.7, 153.3, 151.0, 146.5, 145.7, 141.8, 140.3, 134.0, 133.2, 133.1, 132.8, 130.5, 127.4, 120.4, 117.6, 110.2, 108.4, 55.6, 49.3, 42.3, 34.8, 15.3, 15.0, 12.4 |
| 186 | C24H22ClF3N6 O7S | 631.0984 | 1H-NMR (400 MHz, CHLOROFORM-D) δ 7.96-7.86 (m, 2H), 7.75-7.65 (m, 1H), 7.46-7.36 (m, 1H), 5.38 (t, J = 15.0 Hz, 2H), 4.44-4.32 (m, 2H), 3.72-3.65 (m, 3H), 3.38-3.29 (m, 2H), 2.13-2.02 (m, 3H), 1.49-1.41 (m, 3H), 1.24-1.17 (m, 3H) | 13C-NMR (101 MHz, CHLOROFORM-D) δ 184.7, 155.4, 153.9, 153.2, 146.2, 144.8, 142.2, 141.4, 140.9, 134.3, 133.1, 129.9, 128.1, 120.4, 110.8, 110.5, 53.6, 51.1, 49.1, 42.2, 35.1, 15.5, 12.4, 7.2 |
| 187 | C25H24C1F3N6 O7S | 645.1141 | 1H-NMR (400 MHz, CHLOROFORM-D) δ 7.90 (d, J = 8.0 Hz, 2H), 7.69 (s, 1H), 7.42-7.39 (m, 1H), 5.35 (s, 2H), 4.38 (td, J = 7.5, 6.8 Hz, 2H), 3.68 (s, 3H), 3.58-3.50 (m, 1H), 2.08 (s, 3H), 1.45 (t, J = 7.3 Hz, 3H), 1.24 (d, J = 6.6 Hz, 6H) | 13C-NMR (101 MHz, CHLOROFORM-D) δ 184.8, 155.5, 153.8, 153.3, 146.2, 144.7, 142.2, 140.9, 140.8, 134.3, 133.3, 132.9, 130.4, 127.8, 120.5, 117.8, 110.9. 110.5, 55.8, 49.1, 42.5, 35.1, 15.5, 15.1, 12.4 |
| 188 | C27H26ClF3N6 O7S | 671.1297 | 1H-NMR (400 MHz, CHLOROFORM-D) δ 7.88 (d, J = 8.0 Hz, 1H), 7.79 (s, 1H), 7.42 (d, J = 8.2 Hz, 1H), 5.41 (s, 2H), 4.43 (q, J = 7.2 Hz, 2H), 3.59 (s, 3H), 3.37 (q, J = 7.3 Hz, 2H), 2.32-2.30 (m, 1H), 2.15 (s, 3H), 1.51 (t, J = 7.3 Hz, 3H), 1.25 (t, J = 7.3 Hz, 3H), 1.00-0.96 (m, 2H), 0.93-0.87 (m, 2H) | 13C-NMR (101 MHz, CHLOROFORM-D) δ 185.5, 155.9, 155.5, 153.8, 153.2, 146.2, 146.1, 141.8, 140.8, 134.1, 133.3, 132.8, 130.0, 127.8, 120.4, 117.7, 110.4, 108.7, 51.0, 49.3, 42.0, 34.8, 15.4, 12.4, 9.1, 8.5, 7.2 |
| 189 | C28H28ClF3N6 O7S | 685.1454 | 1H-NMR (400 MHz, CHLOROFORM-D) δ 7.80 (d, J = 8.0 Hz, 1H), 7.75 (s, 1H), 7.34 (d, J = 8.2 Hz, 1H), 5.30 (s, 2H), 4.37 (td, J = 7.5, 6.9 Hz, 2H), 3.55 (d, J = 6.6 Hz, 1H), 3.52 (d, J = 4.9 Hz, 3H), 2.22 (t, J = 4.8 Hz, 1H), 2.07 (t, J = 4.7 Hz, 3H), 1.43 (t, J = 7.3 Hz, 3H), 1.24-1.16 (m, 6H), 0.92-0.85 (m, 2H), 0.85-0.78 (m, 2H) | 13C-NMR (101 MHz, CHLOROFORM-D) δ 185.6, 155.8, 155.6, 153.7, 153.3, 146.3, 145.9, 141.8, 140.2, 134.1, 133.0, 130.5, 127.6, 120.4 117.7, 116.5, 110.4, 108.8, 55.6, 49.3, 42.3, 34.8, 15.4, 15.1, 12.4, 9.0, 8.5, 2.0 |
| 190 | C27H31ClN6O 7S | 619.1736 | 1H-NMR (400 MHz, CHLOROFORM-D) δ 7.82 (d, J = 8.2 Hz, 1H), 7.35-7.33 (m, 2H), 5.38-5.27 (m, 2H), 3.83-3.76 (m, 5H), 3.31-3.19(m, 3H), 2.39 (s, 3H), 2.09 (s, 3H), 1.32 (t, J = 7.3 Hz, 3H), 1.22-1.16 (m, 9H) | 13C-NMR (101 MHz, CHLOROFORM-D) δ 185.4, 162.0, 158.0, 153.8, 153.2, 151.1, 146.5, 146.2, 140.8, 134.9, 134.1, 132.6, 129.9, 127.7, 108.5, 105.9, 53.6, 51.1, 42.9, 42,0, 39.6, 27.1, 21.5, 15.2, 14.4, 12.4, 7.2 |
| 191 | C25H27ClN6O 7S | 591.1423 | 1H-NMR (400 MHz, CHLOROFORM-D) δ 7.99 (d, J = 8.2 Hz, 1H), 7.71 (s, 1H), 7.64 (s, 1H), 7.45 (d, J = 8.0 Hz, 1H), 5.40 (s, 2H), 3.95 (q, J = 7.3 Hz, 2H), 3.82 (s, 3H), 3.35-3.28 (m, 1H), 3.22 (s, 3H), 2.10 (s, 3H), 1.38 (t, J = 7.3 Hz, 3H), 1.23 (d, J = 7.1 Hz, 6H) | 13C-NMR (101 MHz, CHLOROFORM-D) δ 184.7, 162.3, 157.8, 153.9, 153.2, 146.3, 145.3, 143.2, 141.0, 135.6, 134.3, 132.6, 128.8, 128.4, 110.6, 106.6, 45.4, 43.3, 42.0, 39.6, 27.1, 21.6, 14.5, 12.4 |
| 192 | C30H35ClN6O 7S | 659.2049 | 1H-NMR (400 MHz, CHLOROFORM-D) δ 7.77 (d, J = 8.2 Hz, 1H), 7.56 (s, 1H), 7.33 (d, J = 8.0 Hz, 1H), 5.29 (s, 2H), 4.06 (q, J = 7.3 Hz, 2H), 3.80 (q, J = 7.2 Hz, 2H), 3.25 (t, J = 8.0 Hz, 2H), 2.73 (q, J = 7.5 Hz, 2H), 2.39 (s, 1H), 2.07 (d, J = 4.1 Hz, 3H), 1.63-1.53 (m, 2H), 1.39 (t, J = 7.3 Hz, 3H), 1.29 (t, J = 7.3 Hz, 3H), 1.13-1.06 (m, 3H), 0.96-0.91 (m, 4H), 0.89-0.83 (m, 3H) | 13C-NMR (101 MHz, CHLOROFORM-D) δ 185.8,158.3, 156.0, 153.7, 153.2, 150.2, 146.3, 146.2, 141.0, 140.7, 134.1, 132.6, 129.6, 128.0, 108.8, 106.3, 58.1, 44.5, 42.9, 41.9, 18.0, 16.6, 15.3, 14.5, 13.2, 12.8, 12.4, 9.2, 8.6 |
| 193 | C30H35ClN6O 7S | 659.2049 | 1H-NMR 1H-NMR (400 MHz, CHLOROFORM-D) δ 7.84-7.79 (m, 1H), 7.67-7.59 (m, 1H), 7.41-7.34 (m, 1H), 5.34-5.30 (m, 2H), 4.10 (td, J = 14.8, 7.5 Hz, 2H), 3.87-3.82 (m, 2H), 3.63-3.53 (m, 1H), 2.78 (td, J = 15.0, 7.4 Hz, 2H), 2.46-2.32 (m, 1H), 2.13-2.10 (m, 3H), 1.44 (t, J = 7.3 Hz, 3H), 1.33 (t, J = 7.3 Hz, 3H), 1.26-1.20 (m, 6H), 1.14 (t, J = 7.6 Hz, 3H), 1.01-0.95 (m, 2H), 0.91-0.85 (m, 2H) | 13C-NMR (101 MHz, CHLOROFORM-D) δ 185.8, 158.3, 155.9, 153.6, 153.3, 150.1, 146.2, 140.7, 139.9, 134.1, 132.9, 130.3, 127.8, 108.9, 106.3, 55.6, 44.5, 42.9, 42.3, 18.0, 15.3, 15.1, 14.4, 13.2, 12.4, 9.2, 8.5 |
| 194 | C23H22C12N6 O7S | 597.072 | 1H-NMR (400 MHz, CHLOROFORM-D) δ 7.84 (dd, J = 14.8, 6.9 Hz, 1H), 7.35-7.30 (m, 1H), 5.58-5.27 (m, 2H), 3.78-3.70 (m, 3H), 3.59-3.51 (m, 3H), 3.19 (t, J = 15.0 Hz, 3H), 2.47-2.40 (m, 3H), 2.31-2.24 (m, 3H), 2.12-2.05 (m, 3H) | 13C-NMR (101 MHz, CHLOROFORM-D) δ 185.0, 155.0, 153.9, 153.1, 15 1.5, 145.7, 145.6, 142.8, 135.7, 134.5, 132.6, 128.5, 127.9, 108.4, 104.7, 45.1, 43.6, 41.7, 38.0, 37.7, 34.7, 15,2, 12.4 |
| 195 | C24H24Cl2N6 O7S | 611.0877 | 1H-NMR (400 MHz, CHLOROFORM-D) δ 7.87 (d, J = 8.2 Hz, 1H), 7.39 (d, J = 8.2 Hz, 1H), 5.37 (s, 2H), 3.83 (s, 3H), 3.62 (s, 3H), 3.41-3.36 (m, 2H), 2.52 (d, J = 3.0 Hz, 3H), 2.35 (d, J = 10.2 Hz, 3H), 2.14 (s, 3H), 1.25 (t, J = 7.4 Hz, 3H) | 13C-NMR (101 MHz, CHLOROFORM-D) δ 185.3,157.2, 153.8, 153.2, 152.8, 151.9, 151.3, 146.8, 145.8, 140.9, 134.1, 132.7, 129.8, 127.7, 108.5, 104.6, 51.0, 41.9,37.0,34.7, 15.2, 14.7, 12.4, 7.2 |
| 196 | C25H26Cl2N6 O7S | 625.1033 | 1H-NMR (400 MHz, CHLOROFORM-D) δ 7.79 (dd, J = 14.8, 6.9 Hz, 1H), 7.33-7.28 (m, 1H), 5.45-5.27 (m, 2H), 3.79-3.71 (m, 3H), 3.59-3.51 (m, 3H), 3.29-3.21 (m, 2H), 2.48-2.40 (m, 3H), 2.32-2.25 (m, 3H), 2.11-2.03 (m, 3H), 1.66-1.53 (m, 2H), 0.95-0.88 (m, 3H) | 13C-NMR (101 MHz, CHLOROFORM-D) δ 185.3, 157.2, 153.8, 153.2, 151.9, 151.3, 146.8, 145.8, 141.4, 134.1, 132.6, 129.6, 127.7, 108.5, 104.6, 58.0, 41.9, 37.0, 34.7, 16.7, 15.2, 14.7, 12.8, 12.3 |
| 197 | C25H26Cl2N6 O7S | 625.1033 | 1H-NMR (400 MHz, CHLOROFORM-D) δ 7.78 (d, J = 8.2 Hz, 1H), 7.30 (d, J = 8.2 Hz, 1H), 5.27 (s, 2H), 3.76 (s, 3H), 3.57-3.52 (m, 4H), 2.43 (s, 3H), 2.29 (s, 3H), 2.06 (s, 3H), 1.26-1.21 (m, 6H) | 13C-NMR (101 MHz, CHLOROFORM-D) δ 185.4, 157.3, 153.8, 153.3, 151.9, 151.2, 146.8, 145.7, 140.3, 134.1, 132.9, 132.4, 130.3, 127,5, 108.5, 104.6, 55.6, 42.2, 37.0, 34.7, 15.1, 14.7, 12.4 |
| 198 | C22H20Cl2N6 O7S | 583.0564 | 1H-NMR (400 MHz, CHLOROFORM-D) δ 7.94 (d, J = 8.2 Hz, 1H), 7.81 (s, 1H), 7.41 (d, J = 8.0 Hz, 1H), 5.39 (s, 2H), 3.77 (s, 3H), 3.66 (s, 3H), 3.22 (s, 3H), 2.33 (s, 3H), 2.09 (s, 3H) | 13C-NMR (101 MHz, CHLOROFORM-D) δ 184.5,157.1, 153.9, 153.2, 152.1, 146.5, 145.1, 143.2, 140.7, 134.3, 133.0, 132.6, 128.7, 128.4, 110.8, 105.1, 45.3, 41.9, 36.9, 35.1, 14.8, 12.4 |
| 199 | C23H22Cl2N6O7S | 597.072 | 1H-NMR (400 MHz, CHLOROFORM-D) δ 7.88 (d, J = 8.0 Hz, 1H), 7.83 (s, 1H), 7.39 (d, J = 8.0 Hz, 1H), 5.35 (s, 2H), 3.77 (s, 3H), 3.66 (s, 3H), 3.33 (q, J = 7.3 Hz, 2H), 2.34 (s, 3H), 2.08 (s, 3H), 1.20 (t, J = 7.4 Hz, 3H) | 13C-NMR (101 MHz, CHLOROFORM-D) δ 184.7,157.1, 153.8, 153.2, 152.2, 146.5, 145.0, 141.3, 140.7, 134.3, 132.9, 132.9, 129.8, 128.0, 110.8, 105.1, 51.1, 42.1, 36.9, 35.1, 14.7, 12.4, 7.2 |
| 200 | C24H24Cl2N6 O7S | 611.0877 | 1H-NMR (400 MHz, CHLOROFORM-D) δ 7.87 (d, J = 8.2 Hz, 1H), 7.83 (s, 1H), 7.37 (d, J = 8.2 Hz, 1H), 5.31 (s, 2H), 3.77 (s, 3H), 3.66 (s, 3H), 3.60-3.53 (m, 1H), 2.34 (s, 3H), 2.07 (s, 3H), 1.23 (d, J = 6.9 Hz, 6H) | 13C-NMR (101 MHz, CHLOROFORM-D) δ 184.7, 157.1, 153.8, 153.3, 152.2, 146.5, 144.9, 140.8, 140.7, 134.3, 133.1, 132.8, 130.3, 127.7, 110.8, 105.0, 55.7, 42.4, 36.9, 35.1, 15.1, 14.7, 12.4 |
| 201 | C25H24Cl2N6 O7S | 623.0877 | 1H-NMR (400 MHz, CHLOROFORM-D) δ 7.93 (d, J = 8.0 Hz, 1H), 7.44 (d, J = 8.2 Hz, 1H), 5.42 (s, 2H), 3.82 (s, 3H), 3.57 (d, J = 2^{.}2 Hz, 3H), 3.26 (s, 3H), 2.47 (d, J = 9.6 Hz, 1H), 2.35 (s, 3H), 2.16(s, 3H), 1.01-0.99 (m, 2H), 0.96 (td, J = 5.4, 2.7 Hz, 2H) | 13C-NMR (101 MHz, CHLOROFORM-D) δ 185.3, 157.2, 156.2,153.9,153.2, 15 1.9, 146.5, 146.1, 142.7, 134.1, 132.6, 132.4, 128.7, 128.2, 108.8, 104.8, 45.3, 41.7, 36.9, 34.8, 14.7, 12.4,9.1, 8.7 |
| 202 | C26H26Cl2N6 O7S | 637.1033 | 1H-NMR (400 MHz, CHLOROFORM-D) δ 7.88-7.82 (m, 1H), 7.42-7.36 (m, 1H), 5.53-5.31 (m, 2H), 3.80 (s, 3H), 3.56 (d, J = 8.7 Hz, 3H), 3.36 (td, J = 14.9, 7.5 Hz, 2H), 2.53-2.41 (m, 1H), 2.33 (s, 3H), 2.14-2.11 (m, 3H), 1.22 (td, J = 7.2, 3.1 Hz, 3H), 1.02-0.96 (m, 2H), 0.95-0.86 (m, 2H) | 13C-NMR (101 MHz, CHLOROFORM-D) δ 185.4, 157.3, 156.2, 153.8,153.2, 151.9, 146.5, 146.0, 140.8, 134.2, 132.7, 132.6, 129.8, 127.8, 51.0, 41.9, 36.9, 34.8, 14.7, 12.4, 9.1, 8.7, 7.2 |
| 203 | C27H28Cl2N6 O7S | 651.119 | 1H-NMR (400 MHz, CHLOROFORM-D) δ 7.79 (d, J = 8.2 Hz, 1H), 7.34 (d, J = 8.0 Hz, 1H), 5.28 (s, 2H), 3.76 (s, 3H), 3.55 (t, J = 7.3 Hz, 1H), 3.50 (s, 3H), 2.43-2.39 (m, 1H), 2.29 (s, 3H), 2.06 (s, 3H), 1.22 (dd, J = 14.8, 8.0 Hz, 6H), 0.94-0.90 (m, 2H), 0.89-0.85 (m, 2H) | 13C-NMR (101 MHz, CHLOROFORM-D) δ 185.5, 157.3 153.7, 153.3, 151.9, 146.6, 145.9, 140.3, 134.2, 132.9, 132.5, 130.2, 127.6, 108.9, 104.8,55.6, 42.3, 36.9, 34.7, 15.1, 14.6, 12.4, 9.1, 8.6 |
| 204 | C23H19Cl2F3N 6O7S | 651.0438 | 1H-NMR (400 MHz, CHLOROFORM-D) δ 7.83-7.81 (m, 1H), 7.33 (d, J = 8.2 Hz, 1H), 5.37 (s, 2H), 3.89 (s, 3H), 3.55 (s, 3H), 3.20 (s, 3H), 2.45 (s, 3H), 2.09 (d, J = 7.7 Hz, 3H) | 13C-NMR (101 MHz, CHLOROFORM-D) δ 185.0, 155.0, 153.9, 153.1, 151.5, 145.7, 145.6, 142.8, 135.7, 134.5, 132.6, 128.5, 127.9, 120.9, 108.4, 104.7, 45.1, 41.7, 38.0, 37.7, 34.7, 15.2, 12.4 |
| 205 | C25H23Cl2F3N 6O7S | 679.0751 | 1H-NMR (400 MHz, CHLOROFORM-D) δ 7.76 (d, J = 8.2 Hz, 1H), 7.31 (d, J = 8.2 Hz, 1H), 5.40-5.27 (m, 2H), 3.89 (s, 3H), 3.54 (s, 3H), 3.28 (t, J = 8.0 Hz, 2H), 2.45 (s, 3H), 2.06 (d, J = 4.4 Hz, 3H), 1.67-1.59 (m, 2H), 0.92 (t, J = 7.4 Hz, 3H) | |
| 206 | C25H23C12F3N 6O7S | 679.0751 | 1H-NMR (400 MHz, CHLOROFORM-D) δ 7.76 (t, J = 4.0 Hz, 1H), 7.30 (d, J = 8.0 Hz, 1H), 5.29 (s, 2H), 3.90 (s, 3H), 3.58-3.54 (m, 4H), 2.45 (s, 3H), 2.06 (s, 3H), 1.27-1.18 (m, 6H) | 13C-NMR (101 MHz, CHLOROFORM-D) δ 185.2, 155.0, 153.8, 153.2, 151.4, 145.8, 145.4, 140.4, 135.6,134.5,133.1, 130.1, 127.4, 118.2 108.5, 104.7, 55.5, 42.2, 38.1, 34.7, 15.2, 14.9, 12.4, 2.0 |
| 207 | C22H17Cl2F3N 6O7S | 637.0281 | 1H-NMR (400 MHz, CHLOROFORM-D) δ 7.92 (d, J = 8.2 Hz, 1H), 7.83 (s, 1H), 7.40 (d, J = 8.0 Hz, 1H), 5.42 (s, 2H), 3.90 (s, 3H), 3.66 (s, 3H), 3.22 (s, 3H), 2.09 (s, 3H) | 13C-NMR (101 MHz, CHLOROFORM-D) δ 184.3, 154.7, 153.9, 153.2, 144.8, 143.3. 140.8, 135.9, 134.6, 132.8, 128.6, 121.0, 118.3, 110.7, 105.3, 45.2, 41.9, 37.9, 35.0, 12.3 |
| 208 | C23H19C12F3N 6O7S | 651.0438 | 1H-NMR (400 MHz, CHLOROFORM-D) δ 7.92-7.89 (m, 2H), 7.44 (d, J = 8.2 Hz, 1H), 5.42 (s, 2H), 3.96 (s, 3H), 3.71 (s, 3H), 3.39 (q, J = 7.3 Hz, 2H), 2.13 (s, 3H), 1.25 (t, J = 7.3 Hz, 3H) | 13C-NMR (101 MHz, CHLOROFORM-D) δ 184.4, 154.7, 153.9, 153.2, 144.7, 141.4, 140.7, 135.8, 134.6, 133.1, 129.7, 127.8, 121.0, 118.3, 110.8, 105.2, 53.5, 51.0, 42.1, 37.9, 35.0, 12.4, 7.2 |
| 209 | C24H21Cl2F3N 6O7S | 665.0594 | 1H-NMR (400 MHz, CHLOROFORM-D) δ 7.86 (d, J = 8.2 Hz, 2H), 7.38 (d, J = 8.2 Hz, 1H), 5.37 (s, 2H), 3.91 (s, 3H), 3.66 (s, 3H), 3.31 (t, J = 8.0 Hz, 2H), 2.08 (s, 3H), 1.66-1.59 (m, 2H), 0.93 (t, J = 7.4 Hz, 3H) | 13C-NMR (101 MHz, CHLOROFORM-D) δ 184.5, 154.7, 153.9, 153.2, 145.5, 144.7, 141.8, 140.7, 135.9, 134.6, 133.0, 129.5, 127.8, 121.0, 118.3, 110.7, 105.1. 57.9, 42.0, 38.0, 35.1, 16.7, 12.8, 12.4 |
| 210 | C24H21Cl2F3N 6O7S | 665.0594 | 1H-NMR (400 MHz, CHLOROFORM-D) δ 7.83-7.89 (2H), 7.36-7.40 (1H), 5.30-5.36 (2H), 3.90-3.93 (3H), 3.64-3.67 (3H), 3.53-3.61 (1H), 2.05-2.09 (3H), 1.21-1.26 (6H) | 13C-NMR (101 MHz, CHLOROFORM-D) δ 184.5,154.7, 153.8, 153.2, 145.5, 144.6, 141.6, 140.8, 140.7, 135.8, 134.6, 133.3, 130.1, 127.6, 121.0, 118.3, 110.8, 105.1, 55.6, 42.4, 38.0, 35.1, 15.1, 12.4 |
| 211 | C26H23Cl2F3N 6O7S | 691.0751 | 1H-NMR (400 MHz, CHLOROFORM-D) δ 7.78 (d, J = 8.2 Hz, 1H), 7.35 (d, J = 8.2 Hz, 1H), 5.34 (s, 2H), 3.89 (s, 3H), 3.50 (s, 3H), 3.33 (q, J = 7.4 Hz, 2H), 2.46 (t, J = 4.9 Hz, 1H), 2.07 (s, 3H), 1.21-1.16 (m, 3H), 0.96-0.87 (m, 4H) | 13C-NMR (101 MHz, CHLOROFORM-D) δ 185.2, 156.4, 155.0, 153.8, 153.2, 145.7, 145.5, 140.8, 135.6, 134.6, 132.9, 129.6, 127.7, 120.9, 108.7, 104.9, 50.9, 42.0, 38,0, 37.7, 34.7, 12.4, 9.1, 8.8, 7.3 |
| 212 | C27H25Cl2F3N 6O7S | 705.0907 | 1H-NMR (400 MHz, CHLOROFORM-D) δ 7.84 (dd, J = 8.1, 3.2 Hz, 1H), 7.44-7.38 (m, 1H), 5.51-5.19 (m, 2H), 3.97 (s, 3H), 3.67-3.61 (m, 1H), 3.58 (d, J = 7.4 Hz, 3H), 2.57-2.46 (m, 1H), 2.13 (d, J = 4.4 Hz, 3H), 1.32 (dd, J = 16.8, 6.6 Hz, 6H), 1.02-0.96 (m, 4H) | 13C-NMR (101 MHz, CHLOROFORM-D) δ 185.3, 156.4, 155.0, 153.8, 153.2, 145.6, 145.5, 141.4, 141.0, 140.4, 135.5, 134.6,133.1,130.1, 127.5. 120.9, 118.2, 108.8, 104.9, 55.5, 42.3, 38.0, 34.7, 15.1, 12.4, 9.1, 8.7 |
| 213 | C22H19C13N6 O7S | 617.0174 | 1H-NMR (400 MHz, DMF-D7) δ 7.84 (s, 1H), 7.51 (d, J = 8.2 Hz, 1H), 5.26 (d, J = 21.4 Hz, 2H), 3.71 (s, 3H), 3.54 (d, J = 7.4 Hz, 3H), 3.25 (s, 3H), 2.19 (d, J = 7.7 Hz, 3H), 2.02 (s, 3H) | |
| 214 | C23H21C13N6 O7S | 631.0331 | 1H-NMR (400 MHz, CHLOROFORM-D) δ 7.90-7.79 (m, 1H), 7.36 (td, J = 15.0, 8.0 Hz, 1H), 5.64-5.28 (m, 2H), 3.85 (t, J = 15.0 Hz, 3H), 3.63-3.61 (m, 3H), 3.46-3.31 (m, 2H), 2.53 (q, J = 15.0 Hz, 3H), 2.16-2.08 (m, 3H), 1.23 (t, J = 7.3 Hz, 3H) | 13C-NMR (101 MHz, CHLOROFORM-D) δ 185.2,155.5, 153.9, 153.2, 151.4, 146.2, 145.7, 140.8, 140.7, 134.5, 134.3, 132.9, 129.7, 127.7, 108.3, 104.1, 50.9, 41.9, 37.8, 34.8, 15.2, 12.5, 7.2 |
| 215 | C24H23C13N6 O7S | 645.0487 | 1H-NMR (400 MHz, CHLOROFORM-D) δ 7.79 (d, J = 8.0 Hz, 1H), 7.30 (d, J = 8.0 Hz, 1H), 5.34 (s, 2H), 3.80 (d, J = 4.4 Hz, 3H), 3.60-3.55 (m, 4H), 2.46 (s, 3H), 2.06 (d, J = 4.1 Hz, 3H), 1.28-1.19 (m, 6H) | 13C-NMR (101 MHz, CHLOROFORM-D) δ 185.3, 155.4, 153.8, 153.2, 151.4, 146.2, 145.5, 140.8, 140.3, 134.5, 134.2, 133.1,130.2,127.5, 108.4, 104.1, 55.5, 42.3, 37.7, 34.8, 15.2, 12.4 |
| 218 | C22H20Cl2N6 O7S | 583.0564 | 1H-NMR (400 MHz, CHLOROFORM-D) δ 7.86 (d, J = 8.0 Hz, 1H), 7.54 (s, 1H), 7.33 (d, J = 8.0 Hz, 1H), 5.40 (s, 2H), 3.82 (d, J = 3.0 Hz, 3H), 3.55 (s, 3H), 3.18 (d, J = 2.7 Hz, 3H), 2.42 (s, 3H), 2.10 (t, J = 4.0 Hz, 3H) | |
| 219 | C23H22Cl2N6 O7S | 597.072 | 1H-NMR (400 MHz, CHLOROFORM-D) δ 7.82-7.80 (m, 1H), 7.54 (s, 1H), 7.32 (d, J = 8.0 Hz, 1H), 5.36 (s, 2H), 3.84 (s, 3H), 3.55 (s, 3H), 3.30 (q, J = 7.3 Hz, 2H), 2.42 (s, 3H), 2.09 (d, J = 3.0 Hz, 3H), 1.20 (t, J = 7.4 Hz, 3H) | 13C-NMR (101 MHz, CHLOROFORM-D) δ 185.3, 156.3, 153.8, 153.2, 151.2, 146.5, 145.9, 141.2, 140.9, 136.4, 134.4, 132.9, 129.9, 127.7, 108.4, 107.1, 51.1, 42.1, 40.7, 34.9, 15.2, 12.4, 7.2 |
| 220 | C24H24C12N6 O7S | 611.0877 | 1H-NMR (400 MHz, CHLOROFORM-D) δ 7.78 (d, J = 8.0 Hz, 1H), 7.53 (s, 1H), 7.31 (d, J = 8.2 Hz, 1H), 5.32 (s, 2H), 3.84 (s, 3H), 3.54 (s, 3H), 3.49 (t, J = 6.6 Hz, 1H), 2.41 (s, 3H), 2.09 (s, 3H), 1.23 (d, J = 6.6 Hz, 6H) | 13C-NMR (101 MHz, CHLOROFORM-D) δ 185.3,156.3, 153.8, 153.2, 151.2, 146.5, 145.8, 141.3, 140.2, 136.3, 134.4, 133.1, 130.4, 127.4, 108.5, 107.1, 55.9, 42.5, 40.6, 34.9, 15.1, 12.4 |
| 221 | C24H22Cl2N6 O7S | 609.072 | 1H-NMR (400 MHz, CHLOROFORM-D) δ 7.85 (dd, J = 15.1, 6.9 Hz, 1H), 7.67-7.57 (m, 1H), 7.39-7.29 (m, 1H), 5.32 (d, J = 36.6 Hz, 2H), 3.82 (t, J = 15.1 Hz, 3H), 3.51 (t, J = 15.0 Hz, 3H), 3.20 (q, J = 14.9 Hz, 3H), 2.49-2.42 (m, 1H), 2.13-2.05 (m, 3H), 0.98-0.85 (m, 4H) | 13C-NMR (101 MHz, CHLOROFORM-D) δ 185.3, 156.3, 156.3, 153.8, 153.2, 146.3, 146.1, 142.7, 141.1, 134.1, 133.1, 132.5, 128.7, 128.3, 108.7, 106.7, 45.3, 41.8, 37.3, 34.8, 12.4, 9.1. 8.7 |
| 222 | C26B26Cl2N6 O7S | 637.1033 | 1H-NMR (400 MHz, CHLOROFORM-D) δ 7.79 (d, J = 8.0 Hz, 1H), 7.67 (s, 1H), 7.32 (d, J = 8.0 Hz, 1H), 5.31 (d, J = 21.7 Hz, 2H), 3.83 *(s,* 3H), 3.58-3.53 (m, 1H), 3.50 (s, 3H), 2.43-2.40 (m, 1H), 2.07 (s, 3H), 1.21 (d, J = 6.6 Hz. 6H), 0.94-0.92 (m, 2H), 0.89 (dt, J = 8.2, 2.6 Hz, 2H) | 13C-NMR (101 MHz, CHLOROFORM-D) δ 185.5, 156.3, 156.1, 153.7, 153.2, 146.3, 146.0, 141.3, 140.2, 136.3, 134.5, 133.0, 130.3, 127.6, 108.8, 107.3, 55.9, 42.6, 40.6, 34.9, 15.1, 12.4, 9.0, 8.6 |
| 223 | C23H23ClN60 7S | 563.111 | 1H-NMR (400 MHz, CHLOROFORM-D) δ 8.03 (d, J = *8.0* Hz, 1H), 7.42 (d, J = 8.2 Hz, 1H), 6.38 (s, 1H), 5.37 (s, 2H), 4.05 (s, 3H), 3.65 (s, 3H), 3.24 (s, 3H), 2.38 (s, 3H), 2.29 (s, 3H), 2.17 (s, 3H) | 13C-NMR (101 MHz, CHLOROFORM-D) δ 185.2, 154.9, 153.9, 153.2, 150.9, 148.0, 146.3, 145.9, 143.0, 133.9, 132.7, 128.8, 128.4, 128.2, 111.8, 108.4, 45.4, 41.6,39.3,34.9, 15.0, 13.3, 12.4 |
| 224 | C24H25ClN6O 7S | 577.1267 | 1H-NMR (400 MHz, CHLOROFORM-D) δ 7.98 (d, J = 8.0 Hz, 1H), 7.41 (d, J = 8.0 Hz, 1H), 6.40 (s, 1H), 5.34 (s, 2H), 4.05 (s, 3H), 3.65 (s, 3H), 3.37 (q, J = 7.4 Hz, 2H), 2.36 (s, 3H), 2.30 (s, 3H), 2.16 (s, 3H), 1.26 (t, J = 7.3 Hz, 3H) | 13C-NMR (101 MHz, CHLOROFORM-D) δ 185.4, 154.9, 153.8, 153.2, 150.8, 148.1, 146.3, 145.9, 141.1, 133.9, 133.0, 129.9, 128.4, 127.8, 111.8, 108.5, 51.1, 41.8, 39.3, 34.9, 15.0, 13.3, 12.4, 7.1 |
| 225 | C25H27ClN6O 7S | 591.1423 | 1H-NMR (400 MHz, CHLOROFORM-D) δ 7.99-7.94 (m, 1H), 7.43-7.38 (m, 1H), 6.43 (t, J = 15.0 Hz, 1H), 5.36-5.27 (m, 2H), 4.06 (s, 3H), 3.65 (s, 3H), 3.63-3.56 (m, 1H), 2.34 (s, 3H), 2.31 (s, 3H), 2.15-2.13 (m, 3H), 1.32-1.26 (m, 6H) | 13C-NMR (101 CHLOROFORM-D) δ 185.5, 154.9, 153.8, 153.3, 150.7, 148.1, 146.4,145.7, 140.5, 133.9, 133.2, 130.4, 128.4, 127.5, 111.9, 111.3, 108.5, 55.8, 42.2, 39.2, 34.8, 15.2, 14.9, 13.3, 12.4 |
| 226 | C27H29ClN6O 7S | 617.158 | 1H-NMR (400 MHz, CHLOROFORM-D) Õ 7.98 (dd, J = 14.8, 6.9 Hz, 1H), 7.48-7.43 (m, 1H), 6.48 (t, J = 15.0 Hz, 1H), 5.36 (d, J = 14.8 Hz, 2H), 4.09-4.05 (m, 3H), 3.62-3.61 (m, 4H), 2.31 (s, 3H), 2.15-2.12 (m, 3H), 2.05-1.98 (m, 1H), 1.30 (d, J = 6.6 Hz, 6H), 0.97-0.92 (m, 2H), 0.83-0.78 (m, 2H) | 13C-NMR (101 MHz, CHLOROFORM-D) δ 185.7, 162.1, 153.7, 153.3, 148.1, 146.2, 146.0. 140.4, 134.1, 133.1. 130.4, 128.7, 127.7, 112.0, 111.4, 108.9, 55.8, 42.3, 39.3, 34.8, 15.2, 13.3, 12.4, 9.0, 8.3 9.0, |
| 227 | C23H23ClN6O 7S | 563.111 | 1H-NMR (400 MHz, CHLOROFORM-D) δ 7.88 (d, J = 8.2 Hz, 1H), 7.38 (d, J = 8.0 Hz, 1H), 6.34 (d, J = 0.5 Hz, 1H), 5.40 (s, 2H), 3.85 (s, 3H), 3.63 (d, J = 9.6 Hz, 3H), 3.21 (s, 3H), 2.56 (s, 3H), 2.29 (d, J = 0.5 Hz, 3H), 2.15 (s, 3H) | 13C-NMR (101 MHz, CHLOROFORM-D) δ 185.3, 157.5, 153.8, 153.2, 151.5, 147.1, 145.8, 142.1, 141.7, 136.9, 134.0, 132.4, 129.0, 128.1, 109.3, 108.2, 45.4, 41.5, 37.6, 34.8, 15.3, 12.4, 11.3 |
| 228 | C24H25ClN6O 7S | 577.1267 | 1H-NMR (400 MHz, CHLOROFORM-D) δ 7.75 (d, J = 8.0 Hz, 1H), 7.30 (d, J = 8.0 Hz, 1H), 6.28 (d, J = 0.5 Hz, 1H), 5.28 (d, J = 6.0 Hz, 2H), 3.78 (s, 3H), 3.55 (s, 3H), 3.26 (q, J = 7.4 Hz, 2H), 2.49 (s, 3H), 2.23 (d, J = 0.5 Hz, 3H), 2.06 (d, J = 4.1 Hz, 3H), 1.15 (t, J = 7.4 Hz, 3H) | 13C-NMR (101 MHz, CHLOROFORM-D) δ 185.4,157.5, 153.8, 153.2, 151.4, 147.1, 145.7, 141.8, 140.1, 136.9, 134.0, 132.6, 130.1, 127.7, 109.3, 108.2, 51.0, 41.7, 37.6, 34.8, 15.3, 12.4, 11.3, 7.2 |
| 229 | C25H27ClN6O 7S | 591.1423 | 1H-NMR (400 MHz, CHLOROFORM-D) δ 7.84-7.78 (m, 1H), 7.36 (dd, J = 15.1, 6.9 Hz, 1H), 6.35 (t, J = 14.8 Hz, 1H), 5.45-5.27 (m, 2H), 3.86 (s, 3H), 3.62 (s, 3H), 3.60-3.55 (m, 1H), 2.58-2.51 (m, 3H), 2.32-2.31 (m, 3H), 2.13 (d, J = 4.1 Hz, 3H), 1.24 (t, J = 7.0 Hz, 6H) | 13C-NMR (101 MHz, CHLOROFORM-D) δ 185.5, 157.6, 153.7, 153.3, 151.4, 147.1, 145.6, 141.8, 139.5, 136.9, 134.0, 132.8, 130.6, 127.5, 109.2, 108.2, 55.5, 42.1, 37.6, 34.8, 15.3, 15.2, 12.4, 11.4 |
| 231 | C22H19ClN4O 8S | 535.0685 | 1H-NMR (400 MHz, CHLOROFORM-D) δ 7.88 (d, J = 8.0 Hz, 1H), 7.58 (q, J = 0.8 Hz, 1H), 7.34 (d, J = 8.2 Hz, 1H), 7.05-7.04 (m, 1H), 6.51 (q, J = 1.7 Hz, 1H), 5.35 (s, 2H), 3.58 (s, 3H), 3.17 (s, 3H), 2.39 (s, 3H), 2.11 (d, J = 5.2 Hz, 3H) | 13C-NMR (101 MHz, CHLOROFORM-D) δ 185.2, 153.9, 153.2,153.2,151.2, 149.3, 146.3, 145.9, 142.8, 140.5, 134.0, 132.4, 128.9, 128.1, 122.3, 113.1, 108.4, 77.5, 77.1, 76.8, 45.4,41.6,35.0, 15.1, 12.4 |
| 233 | C23H21ClN6O 8S | 577.0903 | 1H-NMR (400 MHz, CHLOROFORM-D) δ 7.82 (d, J = 8.0 Hz, 1H), 7.59 (q, J = 0.8 Hz, 1H), 7.33 (d, J = 8.2 Hz, 1H), 7.06 (d, J = 3.6 Hz, 1H), 6.51 (q, J = 1.7 Hz, 1H), 5.31 (s, 2H), 3.58 (s, 3H), 3.32-3.27 (m, 2H), 2.38 (s, 3H), 2.09 (d, J = 5.5 Hz, 3H), 1.19 (q, J = 7.4 Hz, 3H) | 13C-NMR (101 MHz, CHLOROFORM-D) δ 185.3, 153.8, 153.3, 153.2, 151.1, 149.4, 146.3, 145.9, 140.9, 140.5, 134.1, 132.7, 129.9, 127.7, 122.3, 113.2, 108.4, 51.1, 41.9, 35.0, 15-1, 12.4, 7.2 |
| 234 | C24H23ClN4O 8S | 563.0998 | 1H-NMR (400 MHz, CHLOROFORM-D) δ 7.88 (d, J = 8.2 Hz, 1H), 7.67 (q, J = 0.8 Hz, 1H), 7.39 (d, J = 8.0 Hz, 1H). 7.14-7.13 (m, 1H), 6.59 (q, J = 1.7 Hz, IH), 5.36 (s, 2H), 3.65 (s, 3H), 3.60 (t, J = 6.7 Hz, 1H), 2.44 (s, 3H), 2.17 (d, J = 14.8 Hz, 3H), 1.30-1.26 (m, 6H) | 13C-NMR (101 MHz, CHLOROFORM-D) δ 185.4, 153.8, 153.3, 151.0, 149.4, 148.7, 146.3, 145.8, 140.5, 140.2, 134.1, 132.9, 130.4, 127.4, 122.3, 121.8, 113.2, 112.8, 108.5, 55.8, 42.3, 35.0, 15.1, 12.4 |
| 235 | C24H21ClN4O 8S | 561.0841 | 1H-NMR (400 MHz, CHLOROFORM-D) δ 7.97 (d, J = 8.2 Hz, 1H), 7.65 (q, J = 0.8 Hz, 1H), 7.45 (d, J = 8.2 Hz, 1H), 7.14 (d, J = 3.6 Hz, 1H), 6.58 (q, J = 1.7 Hz, 1H), 5.43 (s, 2H), 3.61-3.59 (m, 3H), 3.25 (s, 3H), 2.37 (s, 1H), 2.19-2.16 (m, 3H), 1.01-0.97 (m, 2H), 0.93-0.89 (m, 2H) | 13C-NMR (101 MHz, CHLOROFORM-D) δ 185.4, 156.0, 153.8, 153.2, 149.2, 1458.6, 146.1, 142.7, 140.7, 134.1, 132.4, 128.8, 128.2, 122.2, 121.8, 113.1, 112.8, 108.8, 45.5, 41.7, 35.0, 12.4, 9.1, 8.6 |
| 236 | C25H23ClN4O 8S | 575.0998 | 1H-NMR (400 MHz, ACETONITRILE-D3) δ 7.94 (d, J = 8.2 Hz, 1H), 7.79 (q, J = 0.8 Hz, 1H), 7.53 (d, J = 8.0 Hz, 1H), 7.24 (dd, J = 3.6, 0.5 Hz, 1H). 6.62 (q, J = 1.7 Hz, 1H), 5.32 (s, 2H), 3.54 (d, J = 5.5 Hz, 3H), 3.31 (q, J = 7.3 Hz, 2H), 2.18 (d, J = 2.7 Hz, 1H), 2.13 (s, 3H), 1.14 (q, J = 7.5 Hz, 3H), 0.87-0.85 (m, 2H), 0.84-0.79 (m, 2H) | |
| 237 | C25H25ClN4O 8S | 577.1154 | 1H-NMR (400 MHz, CHLOROFORM-D) δ 7.81 (d, J = 8.2 Hz, 1H), 7.59 (q, J = 0.8 Hz, 1H), 7.32 (d, J = 8.0 Hz, 1H), 7.07 (dd, J = 3.7, 0.7 Hz, 1H), 6.51 (q, J = 1.8 Hz, 1H), 5.29 (t, J = 14.8 Hz, 2H), 3.85 (q, J = 7.2 Hz, 2H), 3.56-3.49 (m, 1H), 2.37 (d, J = 7.4 Hz, 3H), 2.07 (d, J = 4.1 Hz, 3H), 1.37-1.33 (m, 3H), 1.21 (dd, J = 15.0, 8.1 Hz, 6H) | 13C-NMR (101 MHz, CHLOROFORM-D) δ 185.5, 153.7, 153.5, 153.3, 151.1, 149.3, 145.8, 145.6, 140.6, 140.2, 134.2, 132.9, 130.4, 127.5, 122.2, 113.1, 108.4, 55.8, 43.1, 42.3, 15.2, 14.4, 12.4 |
| 238 | C22H19ClN4O 7S2 | 551.0456 | 1H-NMR (400 MHz, CHLOROFORM-D) δ 7.84 (d, J = 8.0 Hz, 1H), 7.67 (dd, J = 4.9, 1.1 Hz, 1H), 7.55 (dd, J = 3.8, 1.1 Hz, 1H), 7.33 (d, J = 8.2 Hz, 1H), 7.08 (dd, J = 4.9, 3.8 Hz, 1H), 5.27 (s, 2H), 3.58 (s, 3H), 3.12 (s, 3H), 2.43 (s, 3H), 2.09 (s, 3H) | 13C-NMR (101 MHz, CHLOROFORM-D) δ 185.3, 157.2, 153.8, 153.2, 151.3, 146.6, 145.9, 142.6, 136.6, 136.6, 133.9, 132.5, 129.0, 128.9, 128.1, 128.1, 108.4, 45.4, 41.6, 34.9, 15.2, 12.4 |
| 239 | C23H21ClN4O 7S2 | 565,0613 | 1H-NMR (400 MHz, CHLOROFORM-D) δ 7.79 (d, J = 8.0 Hz, 1H), 7.68 (dd, J = 5.1, 1.2 Hz, 1H), 7.56 (dd, J = 3.8, 1.4 Hz, 1H), 7.32 (d, J = 8.2 Hz, 1H), 7.08 (dd, J = 4.9, 3.8 Hz, 1H), 5.24 (s, 2H), 3.58 (s, 3H), 3.25 (q, J = 7.3 Hz, 2H), 2.42 (s, 3H), 2.08 (s, 3H), 1.15 (t, J = 7.3 Hz, 3H) | 13C-NMR (101 MHz, CHLOROFORM-D) δ 185.4, 157.2, 153.8 153.3, 151.2, 146.7, 145.9, 140.7, 136.7, 136.6, 134.0, 132.8, 130.1, 128.9, 128.0, 127.7, 108.5, 51.1, 41.8, 34.9, 15.2, 12.4, 7.1 |
| 240 | C24H23ClN4O 7S2 | 579.0769 | 1H-NMR (400 MHz, CHLOROFORM-D) δ 7.84 (d, J = 8.0 Hz, 1H), 7.75 (dd, J = 4.9, 1.4 Hz, 1H), 7.63 (dd, J = 3.8, 1.1 Hz, 1H), 7.38 (d, J = 8.2 Hz, 1H), 7.16 (dd, J = 4.9, 3.8 Hz, 1H), 5.29 (d, J = 8.0 Hz, 2H), 3.66 (d, J = 5.5 Hz, 3H), 3.61-3.56 (m, 1H), 2.48 (s, 3H), 2.14 (s, 3H), 1.26 (d, J = 6.9 Hz, 6H) | 13C-NMR (101 MHz, CHLOROFORM-D) δ 185.5, 157.2, 156.7, 153.7, 153.3, 151.1, 146.7, 145.8, 140.1, 136.7, 136.6, 135.4, 134.0, 133.0, 130.5, 128.8, 128.0, 127.4, 108.5, 55.7, 42.2, 35.0, 15.1, 12.4 |
| 241 | C24H21ClN4O 7S2 | 577.0613 | 1H-NMR (400 MHz, CHLOROFORM-D) δ 7.85 (d, J = 8.2 Hz, 1H), 7.66 (dd, J = 4.9, 1.1 Hz, 1H), 7.56 (dd, J = 3.8, 1.1 Hz, 1H), 7.37 (d, J = 8.0 Hz, 1H), 7.07 (dd, J = 4.9, 3.9 Hz, 1H), 5.33-5.25 (m, 2H), 3.53 (s, 3H), 3.12 (s, 3H), 2.43-2.34 (m, 1H), 2.09 (s, 3H), 0.93 (td, J = 5.3, 3.2 Hz, 2H), 0.90-0.85 (m, 2H) | 13C-NMR (101 MHz, CHLOROFORM-D) δ 185.4, 157.2, 156.1, 153.8, 153.2, 146.4, 146.2, 142.6, 136.5, 136.4, 134.0, 132.5, 128.9, 128.8, 128.3, 128.2, 108.8, 45.4, 41.7, 34.9, 12.3, 9.1, 8.6 |
| 242 | C25H23ClN4O 7S2 | 591.0769 | 1H-NMR (400 MHz, CHLOROFORM-D) δ 7.80 (t, J = 4.0 Hz, 1H), 7.68-7.65 (m, 1H), 7.57 (td, J = 3.9, 1.2 Hz, 1H), 7.37-7.35 (m, 1H), 7.09-7.07 (m, 1H), 5.29-5.26 (m, 2H), 3.53 (s, 3H), 3.25 (q, J = 7.3 Hz, 2H), 2.38-2.31 (m, 1H), 2.07 (t, J = 4.0 Hz, 3H), 1.16 (t, J = 7.4 Hz, 3H), 0.95-0.90 (m, 2H), 0.88-0.83 (m, 2H) | 13C-NMR (101 MHz, CHLOROFORM-D) δ 185.5, 157.2, 156.0, 153.7, 153.3, 146.4, 146.1, 140.8, 136.5, 136.5, 134.1, 132.8, 130.0, 128.8, 128.3, 127.9, 108.9, 51.1,41.9,34.9, 12.4, 9.1, 8.6, 7.1 |
| 243 | C26H25ClN4O 7S2 | 605.0926 | 1H-NMR (400 MHz, CHLOROFORM-D) δ 7.80-7.77 (m, 1H), 7.68-7.66 (m, 1H), 7.58 (dd, J = 3.8, 1.1 Hz, 1H), 7.35 (dd, J = 8.1, 2.6 Hz, 1H), 7.09 (dd, J = 4.9, 3.8 Hz, 1H), 5.21 (d, J = 14.8 Hz, 2H), 3.53 (d, J = 2.5 Hz, 3H), 3.52-3.47 (m, 1H), 2.36-2.29 (m, 1H), 2.07-2.06 (m, 3H), 1.20-1.16 (m, 6H), 0.95-0.90 (m, 2H), 0.88-0.82 (m, 2H) | 13C-NMR (101 MHz, CHLOROFORM-D) δ 185.6, 157.2, 156.0,153.7,153.3, 146.4, 146.0, 140.1, 136.5, 134.1, 132.9, 130.5, 128.9, 128.2, 127.6, 108.9, 55.7, 42.3, 34.9, 15.1, 12.4, 9.0, 8.6 |
| 244 | C22H19ClN4O 8S | 535.0685 | 1H-NMR (400 MHz, CHLOROFORM-D) δ 7.91 (d, J = 8.2 Hz, 1H), 7.83 (q, J = 0.7 Hz, 1H), 7.41 (t, J = 1.6 Hz, 1H), 7.33 (d, J = 8.0 Hz, 1H), 6.45 (q, J = 0.9 Hz, 1H), 5.35 (s, 2H), 3.56 (s, 3H), 3.16 (s, 3H), 2.37 (s, 3H), 2.11 (s, 3H) | 13C-NMR (101 MHz, CHLOROFORM-D) δ 184.1, 156.8, 152.8, 152.1, 150.0, 148.5, 145.5, 144.9, 144.2, 141.7, 132.8, 131.4, 127.8, 127.0, 114.4, 108.1, 107.3, 44.2, 40.7, 33.8, 14.0, 11.3 |
| 246 | C24H23ClN4O 8S | 563.0998 | 1H-NMR (400 MHz, CHLOROFORM-D) δ 7.87-7.86 (m, 1H), 7.85-7.83 (m, 1H), 7.42 (t, J = 1.8 Hz, 1H), 7.31 (d, J = 8.2 Hz, 1H), 6.45 (q, J = 0.9 Hz, 1H), 5.27 (s, 2H), 3.56 (s, 3H), 3.54-3.48 (m, 1H), 2.34 (s, 3H), 2.09 (d, J = 3.6 Hz, 3H), 1.22 (dd, J = 15.0, 8.1 Hz, 6H) | 13C-NMR (101 MHz, CHLOROFORM-D) δ 185.4,157.9, 153.8, 153.3, 150.9, 149.7, 146.7, 145.9, 145.4, 140.4, 134.0, 133.0, 130.5, 127.5, 115.5, 109.1, 108.5, 55.8, 42,4, 34.8, 15.1, 15.0, 12.4 |
| 247 | C24H21ClN4O 8S | 561.0841 | 1H-NMR (400 MHz, CHLOROFORM-D) δ 7.92 (d, J = 8.2 Hz, 1H), 7.86 (q, J = 0.7 Hz, 1H), 7.41 (t, J = 1.6 Hz, 1H), 7.37 (d, J = 8.0 Hz, 1H), 6.48 (q, J = 0.8 Hz, 1H), 5.36 (s, 2H), 3.51 (s, 3H), 3.16(s, 3H), 2.25-2.21 (m, 1H), 2.11 (s, 3H), 0.92-0.88 (m, 2H), 0.83-0.79 (m, 2H) | 13C-NMR (101 MHz, CHLOROFORM-D) δ 185.3, 157.9, 155.8, 153.8, 153.2, 149.6, 146.4, 146.3, 145.2, 142.9, 134.1, 132.4, 128.9, 128.2, 115.7, 109.3, 108.8, 45.3, 41.9, 34.9, 12.3, 9.0, 8.5 |
| 248 | C25H23ClN4O 8S | 575.0998 | 1H-NMR (400 MHz, CHLOROFORM-D) δ 7.92-7.83 (m, 2H), 7.43-7.40 (m, 1H), 7.39-7.34 (m, 1H), 6.48 (td, J = 15.0, 1.6 Hz, 1H), 5.36-5.27 (m, 2H), 3.52 (t, J = 15.0 Hz, 3H), 3.30 (td, J = 15.0, 7.6 Hz, 2H), 2.25-2.17 (m, 1H), 2.13-2.05 (m, 3H), 1.22-1.15 (m, 3H), 0.95-0.88 (m, 2H), 0.85-0.76 (m, 2H) | 13C-NMR (101 MHz, CHLOROFORM-D) δ 185.5, 157.9, 155.8, 153.8, 153.3, 149.7, 146.5, 146.2, 140.9, 132.8, 130.0, 127.9, 115.7, 109.2, 108.8, 51.1,43.5,42.1, 34.9, 12.4, 9.1, 8.5, 7.1 |
| 249 | C26H25ClN4O 8S | 589.1154 | 1H-NMR (400 MHz, ACETONITRILE-D3) δ 8.09 (t, J = 1.1 Hz, 1H), 7.92 (d, J = 8.0 Hz, 1H), 7.57 (t, J = 1.8 Hz, 1H), 7.51 (d, J = 8.2 Hz, 1H), 6.63 (q, J = 0.9 Hz, 1H), 5.30 (s, 2H), 3.53 (t, J = 4.1 Hz, 3H), 2.22 (t, J = 5.1 Hz, 1H), 2.13-2.09 (m, 3H), 1.26-1.23 (m, 1H), 1.21-1.14 (m, 6H), 0.88-0.85 (m, 2H), 0.85-0.80 (m, 2H) | 13C-NMR (101 MHz, CHLOROFORM-D) δ 185.6, 157.9, 155.7, 153.7, 153.3, 149.7, 146.5, 146.1, 145.3, 144.7, 140.3, 134.1, 132.9, 130.5, 127.6, 115.7, 110.0, 109.2, 108.8, 55.8, 42.4, 34.9, 15.1, 12.4, 9.0, 8.5 |
| 250 | C22H19ClN4O 7S2 | 551.0456 | 1H-NMR (400 MHz, CHLOROFORM-D) δ 7.93 (q, J = 1.4 Hz, 1H), 7.85 (dd, J = 14.8, 6.9 Hz, 1H), 7.35-7.29 (m, 2H), 7.17-7.14 (m, 1H), 5.25-5.20 (m, 2H), 3.57 (s, 3H), 3.12 (s, 3H), 2.42 (s, 3H), 2.10 (s, 3H) | 13C-NMR (101 MHz, CHLOROFORM-D) δ 184.1, 156.3, 152.7, 152.1, 150.1, 145.9, 144.9, 141.5, 135.0, 132.7, 131.3, 127.8,127.4,127.0, 126.9, 126.2, 107.2, 44.3, 40.4, 33.8, 14.1, 11.3 |

The compound of Formula (I-3) of the present application can be prepared by the following process:

| | | | | |
|---|---|---|---|---|
| 251 | C24H23ClN4O 7S2 | 579.0769 | 1H-NMR (400 MHz, CHLOROFORM-D) δ 8.02 (q, J = 1.4 Hz, 1H), 7.84 (d, J = 8.0 Hz, 1H), 7.42-7.38 (m, 2H), 7.23 (dd, J = 5.1, 1.2 Hz, 1H), 5.27 (s, 2H), 3.65 (d, J = 5.8 Hz, 3H), 3.55 (t, J = 6.9 Hz, 1H), 2.46 (s, 3H), 2.16 (d, J = 8.8 Hz, 3H), 1.38-1.33 (m, 1H), 1.29-1.26 (m, 6H) | 13C-NMR(101 MHz, CHLOROFORM-D) δ 185.5, 157.6, 157.5, 153.7, 153.3, 151.1,147.0, 145.9, 140.1, 136.2, 133.9, 132.9, 132.3, 130.5, 128.5, 128.3, 127.5, 127.4, 108.4, 55.7, 42.2,34.9, 15.1, 12.4 |
| 252 | C24H21ClN4O 7S2 | 577.0613 | 1H-NMR (400 MHz, CHLOROFORM-D) δ 8.00 (q, J = 1.4 Hz, 1H), 7.91 (dd, J = 14.8, 6.9 Hz, 1H), 7.44-7.39 (m, 1H), 7.38-7.35 (m, 1H), 7.23-7.21 (m, 1H), 5.33 (s, 2H), 3.58 (s, 3H), 3.18 (s, 3H), 2.49-2.37 (m, 1H), 2.14 (d, J = 4.4 Hz, 3H), 1.02-0.97 (m, 2H), 0.95-0.89 (m, 2H) | 13C-NMR (101 MHz, CHLOROFORM-D) δ 185.4,157.4, 156.1, 153.8, 153.2, 146.7, 146.3, 142.6, 136.1, 134.0, 132.4, 128.9, 128.7, 128.2, 128.0, 127.4, 108.7, 45.4, 41.6, 34.9, 12.4, 9.1, 8.7 |
| 253 | C25H23ClN4O 7S2 | 591.0769 | 1H-NMR (400 MHz, CHLOROFORM-D) δ 8.03 (q, J = 1.4 Hz, 1H), 7.88 (d, J = 8.2 Hz, 1H), 7.43-7.41 (m, 1H), 7.39 (q, J = 2.7 Hz, 1H), 7.26-7.24 (m, 1H), 5.31 (s, 2H), 3.60-3.56 (m, 3H), 3.33 (q, J = 7.4 Hz, 2H), 2.39 (s, 1H), 2.17-2.11 (m, 3H), 1.27-1.20 (m, 3H), 1.02-0.98 (m, 2H), 0.96-0.90 (m, 2H) | 13C-NMR (101 MHz, CHLOROFORM-D) δ184.4,156.4, 154.9, 152.7, 152.2, 145.7, 145.1, 139.6, 135.0, 132.9, 131.6, 128.8, 127.6, 126.9, 126.8, 126.3, 107.7, 50.1, 40.8, 33.8, 11.3, 7.9, 7.5, 6.0 |
| 254 | C26H25ClN4O 7S2 | 605.0926 | 1H-NMR (400 MHz, CHLOROFORM-D) δ 8.04 (q, J = 1.5 Hz, 1H), 7.86 (d, J = 8.2 Hz, 1H), 7.43-7.38 (m, 2H), 7.26 (dd, J = 5.1, 1.2 Hz, 1H), 5.29 (s, 2H), 3.61-3.58 (m, 3H), 2.37 (s, 1H), 2.15 (d, J = 5.5 Hz, 3H), 1.33 (t, J = 6.2 Hz, 1H), 1.30-1.21 (m, 6H), 1.01-0.98 (m, 2H), 0.95-0.89 (m, 2H) | 13C-NMR (101 MHz, CHLOROFORM-D) δ 184.5, 156.4, 154.8, 152.6, 152.2, 145.7, 145.0, 138.9, 135.0, 134.6, 132.9, 13 1.8, 129.3, 127.6, 127.2, 126.9, 126.5, 126.3, 107.7, 54.7, 41.2, 33.8, 14.0, 11.3, 7.9, 7.5 |

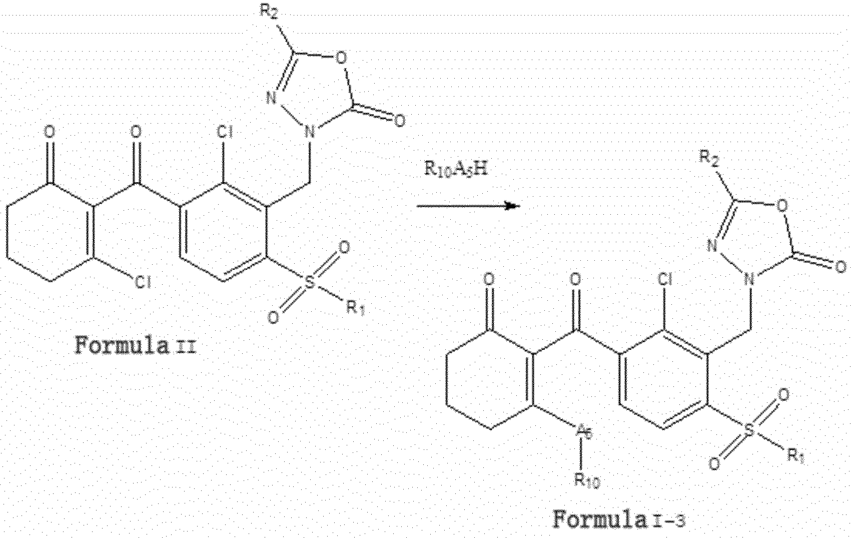

In the above reaction formula, a compound of Formula (II) and R₁₀A₅H are reacted in the presence of a base in a suitable solvent at a temperature from -10 °C to the boiling point for 0.5 to 24 hours to obtain the compound of Formula (1-3). Among them, the suitable solvent is selected from acetonitrile, DMF, HMPA, dimethyl sulfoxide, tetrahydrofuran, and the like. The suitable base is selected from sodium hydrogen, sodium carbonate, potassium carbonate, triethylamine, and the like.

The compound of Formula (II) can be prepared by the following process:

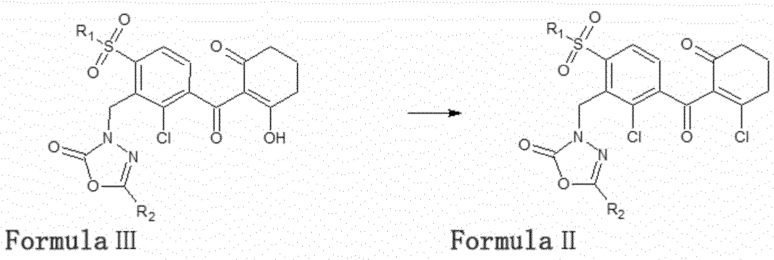

In the above reaction formula, a compound of Formula (III) is reacted with a suitable chlorinating agent at a temperature from 0 °C to the boiling point for 0.5 to 4 hours to obtain the compound of Formula (II). Among them, the suitable chlorinating agent is selected from thionyl chloride, oxalyl chloride, phosphorus trichloride, and the like.

The compound of Formula (III) can be prepared by the following process:

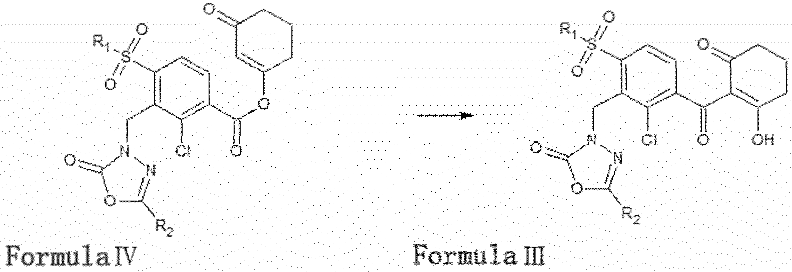

In the above reaction formula, a compound of Formula (IV) is reacted under the action of a base and a catalyst in a suitable solvent at a temperature from -10 °C to the boiling point for 0.5 to 24 hours to obtain the compound of Formula (III). The suitable solvent is selected from dichloromethane, 1,2-dichloroethane, chloroform, carbon tetrachloride, benzene, toluene, ethyl acetate, acetonitrile, tetrahydrofuran, dioxane, N,N-dimethylformamide, dimethyl sulfoxide, and the like. The suitable base is selected from sodium carbonate, potassium carbonate, triethylamine, and the like. The suitable catalyst is selected from sodium carbonate, potassium carbonate, acetone cyanohydrin, and the like.

The compound of Formula (IV) can be prepared by the following process:

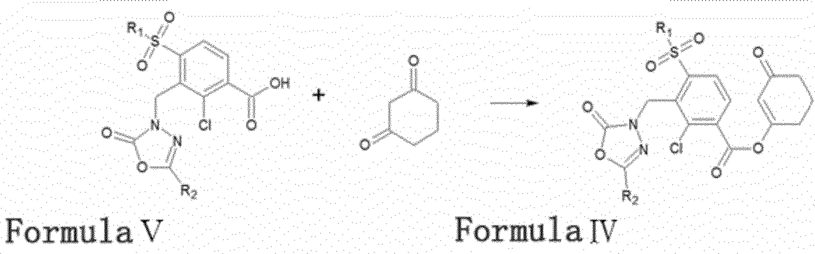

In the above reaction formula, a compound of Formula (V) is first reacted with an acyl halide reagent in a suitable solvent at a temperature from -10 °C to the boiling point of the solvent for 0.5 to 24 hours to obtain the corresponding acyl chloride. The acyl halide reagent is selected from oxalyl chloride, thionyl chloride, phosphorus oxychloride, phosphorus trichloride, or solid phosgene; the suitable solvent is selected from dichloromethane, 1,2-dichloroethane, chloroform, carbon tetrachloride, benzene, toluene, acetonitrile, tetrahydrofuran, dioxane, and the like. The prepared corresponding acyl chloride is reacted with 1,3-cyclohexanedione in the presence of a suitable base in the same or different solvent at a temperature from -10 °C to the boiling point of the corresponding solvent for 0.5 to 24 hours to obtain the compound of Formula (IV). The suitable base is selected from triethylamine, pyridine, sodium carbonate, potassium carbonate, sodium methanol, potassium tert-butanol, and the like.

The compound of Formula (V) can be prepared by the following process:

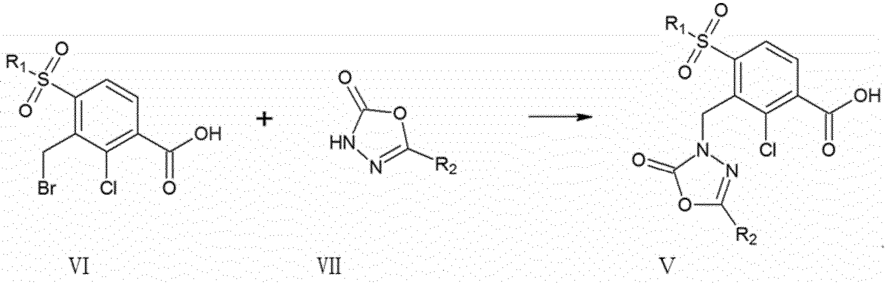

A compound of Formula (VI) (see Synthesis of Herbicide Tembotrione in Corn Field, Agrochemicals, 2017, 56 (5): 326-327, 379) and a compound of Formula (VII) (commercially available product) are reacted in the presence of a suitable base in a suitable solvent at a temperature from 0 °C to the boiling point of the solvent for 0.5 to 24 hours to obtain the compound of Formula (V). The suitable solvent is selected from acetonitrile, N,N-dimethylformamide, dimethyl sulfoxide, methanol, ethanol, isopropyl alcohol, and the like. The suitable base is selected from sodium hydroxide, potassium hydroxide, sodium carbonate, potassium carbonate, sodium methoxide, potassium tert-butoxide, and the like.

A compound of Formula (I-2) can be prepared by the following process:

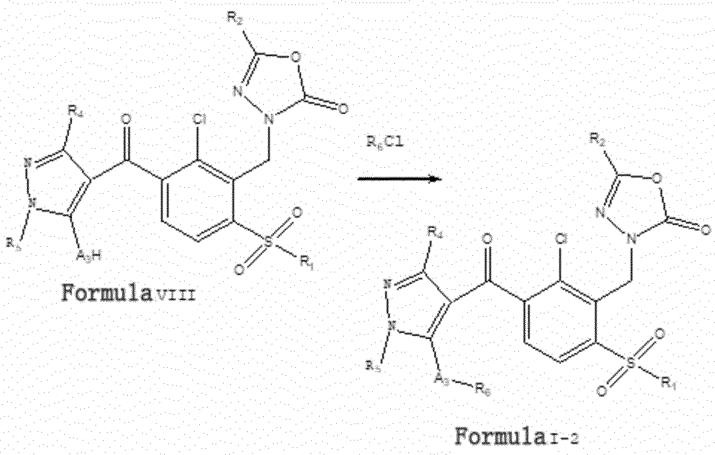

In the above reaction formula, a compound of Formula (VIII) and R₆Cl are reacted under the action of a base in a suitable solvent for 0.5 to 24 hours to obtain the compound of Formula (I-2). The suitable solvent is selected from dichloromethane, 1,2-dichloroethane, chloroform, carbon tetrachloride, benzene, toluene, acetonitrile, tetrahydrofuran, dioxane, and the like. The suitable base is selected from sodium carbonate, potassium carbonate, triethylamine, and the like.

Taking A₃=O as an example, the compound of Formula (VIII) can be prepared by the following process:

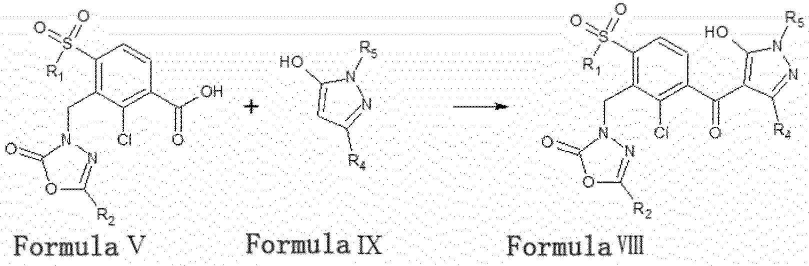

The compound of Formula (VIII) is prepared by the compound of Formula (V) and the compound of Formula (IX) under conditions similar to those for preparing the compound of Formula (III) by the compound of Formula (IV), which will not be repeated herein.

The compound of Formula (I-1) and the compound of Formula (III) are tautomers depending on the environmental conditions, such as keto-enol interconversion due to solvent, PH, etc.

The herbicidal compositions, oxadiazolone compounds and use thereof provided herein are further described below with reference to examples.

### Example 1: Synthesis of Compound 1

### (1) Synthesis of 2-chloro-3-[(5-methyl-2-oxo-1,3,4-oxadiazol-3(2H)yl) methyl)]-4-methylsulfonylbenzoic acid

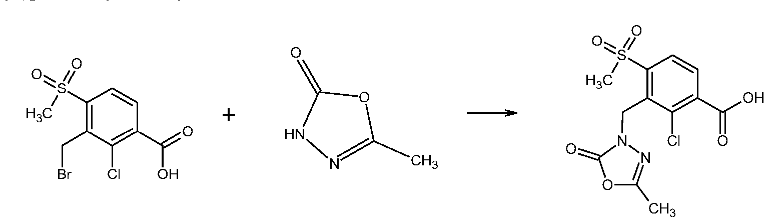

3-bromomethyl-2-chloro-4-methylsulfonylbenzoic acid (6.5 g, 20 mmol), 5-methyl-1,3,4-oxadiazol-2 (3H)-one (2 g, 20 mmol), potassium carbonate (4.1 g, 30 mmol) and acetonitrile (60 mL) were added to a reaction flask. The mixture was heated under reflux for 3 hours, then the solvent was evaporated under reduced pressure. Water (30 mL) was added therein, and then the pH of the resultant solution was adjusted to 2-3 with 10% dilute hydrochloric acid under stirring, as a result, white crystals slowly precipitated. The white crystals were subjected to suction filtration and dried, affording 4.8 g of a white solid in 70% yield.

### (2) 3-oxocyclohex-1-enyl 2-chloro-3-[(5-methyl-2-oxo-1,3,4-oxadiazol-3(2H)-yl)methyl)]-4-methylsulfonylbenzoate

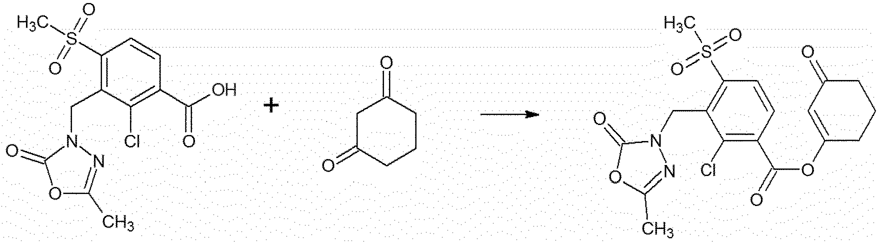

2-chloro-3-[(5-methyl-2-oxo-1,3,4-oxadiazol-3(2H)-yl)methyl)]-4-methylsulfonylbenzoic acid (6.9 g, 20 mmol) and toluene (50 mL) were added to a reaction flask, followed by slow addition of sulfoxide chloride (14.3 g, 120 mmol). The mixture was heated under reflux for 4 hours, evaporated under reduced pressure to remove all the solvent and excess thionyl chloride. Dichloromethane (60 mL) and 1,3-cyclohexanedione (2.3 g, 20.5 mmol) were added, followed by dropwise addition of triethylamine (3.2 g, 32 mmol). The mixture was stirred at room temperature for 1 hour, and then water (50 mL) was added. The aqueous phase was extracted twice with dichloromethane (30 mL × 2). The combined oil phase was evaporated to remove all the solvent, affording 8.3 g of a pale yellow solid, which was used directly in the next step.

### (3) Synthesis of Compound 1 (2-{2-chloro-3-[(5-methyl-2-oxo-1,3,4-oxadiazol-3(2H)yl)methyl)]-4-methylsulfonylbenzoyl}-1,3 -cyclohexanedione)

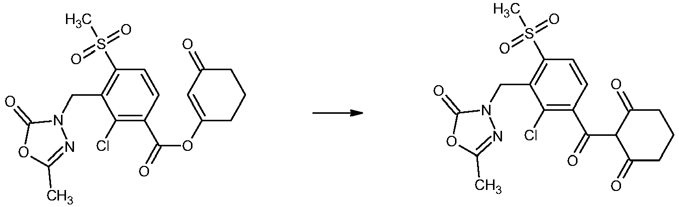

Dichloromethane (60 mL), triethylamine (2.4 g, 24 mmol), and 2 drops of acetone cyanohydrin were added to a reaction flask, and 8.3 g of the solid obtained from the previous step was added therein. The mixture was stirred at room temperature overnight, followed by addition of water (60 mL) with stirring for another half an hour, and the pH of the resultant solution was adjusted to 7-8 with 10% dilute hydrochloric acid. The aqueous phase was then extracted twice with ethyl acetate (60 mL × 2). The combined organic phase was washed with saturated brine (60 mL), dried over anhydrous magnesium sulfate, and evaporated under reduced pressure to remove all the solvent, affording 8.1 g of a pale yellow solid with a purity of 97%.

### Example 2: Synthesis of Compound 53

### (1) 3-{2-chloro-3-[(2-chloro-6-oxocyclohex-1-enyl)carbonyl]-6-(methylsulfonyl)benzyl}-5-methyl-1, 3, 4-oxadiazol-2(3H)-one

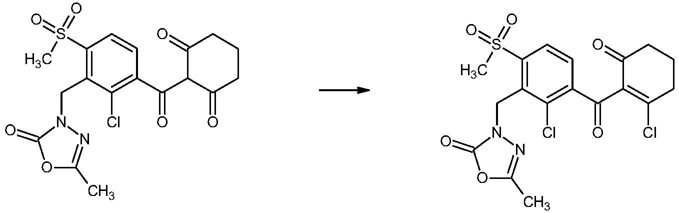

2-{2-chloro-3-[(5-methyl-2-oxo-1,3,4-oxadiazol-3(2H)yl)methyl]-4-methylsulfonylbenzoyl}-1,3-c yclohexanedione (8.8 g, 20 mmol) and dichloromethane (50 mL) were added to a reaction flask, followed by slow addition of thionyl chloride (4.7 g, 40 mmol). The mixture was stirred at room temperature for 4 hours, and then evaporated under reduced pressure to remove all the solvent and excess thionyl chloride, affording 8.2 g of a yellow solid with a purity of 96%.

### (2) Synthesis of Compound 53 (3-{2-chloro-3-[(2-benzylthio-6-oxocyclohex-1-enyl)carbonyl]-6-(methylsulfonyl)benzyl}-5-meth yl-1,3,4-oxadiazol-2 (3H)-one)

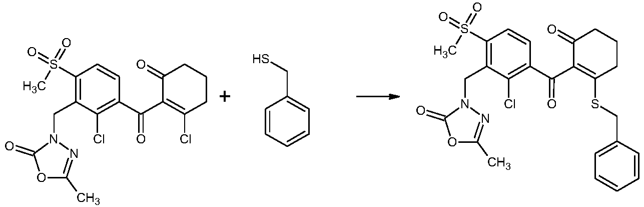

3-{2-chloro-3-[(2-chloro-6-oxocyclohex-1-enyl)carbonyl]-6-(methylsulfonyl)benzyl}-5-methyl-1, 3,4-oxadiazol-2(3H)-one (6 g, 13 mmol), 30 g HMPA and 1.6 g benzyl mercaptan were added to 100 mL four-necked flask. The mixture was stirred well and cooled down to 0 °C. 0.38 g sodium hydrogen was slowly added to the four-necked flask in batches until the sample was determined to show that the reaction of the raw materials was completed. The pH of the resulting solution was adjusted to 3 with acid water. The solution was extracted with ethyl acetate. The ethyl acetate phase was collected and subjected to rotary evaporation under reduced pressure to give the crude product, which was then treated with dichloromethane and sodium bicarbonate aqueous solution with stirring overnight. The dichloromethane phase was collected and subjected to rotary evaporation to give 5.6 g of a yellow solid with a purity of 90%.

### Example 3: Synthesis of Compound 100

### (1) Synthesis of 2-chloro-3-[(5-methyl-2-oxo-1,3,4-oxadiazol-3(2H)-yl)methyl)]-4-isopropylsulfonylbenzoic acid

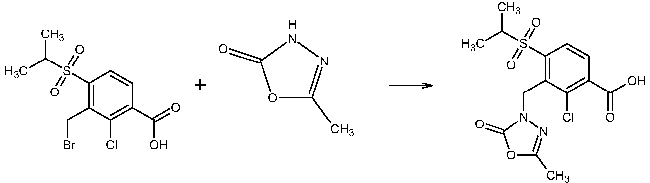

3-bromomethyl-2-chloro-4-isopropylsulfonylbenzoic acid (14.2 g, 40 mmol), 5-methyl-1,3,4-oxadiazol-2(3H)-one (4 g, 40 mmol), 30% sodium hydroxide solution (8 g, 60 mmol), and ethanol (100 mL) were added to a reaction flask. The mixture was heated under reflux for 3 hours, then the solvent was evaporated under reduced pressure. Water (50 mL) was added to the residue, and then the pH of the resultant solution was adjusted to 2-3 with 10% dilute hydrochloric acid under stirring, as a result, white crystals slowly precipitated. The white crystals were subjected to suction filtration and dried, affording 12 g of a white solid in 80% yield.

### (2) Synthesis of 1,3-dimethylpyrazol-5-yl 2-chloro-3-[(5-methyl-2-oxo-1,3,4-oxadiazol-3(2H)-yl)methyl)]-4-isopropylsulfonylbenzoate

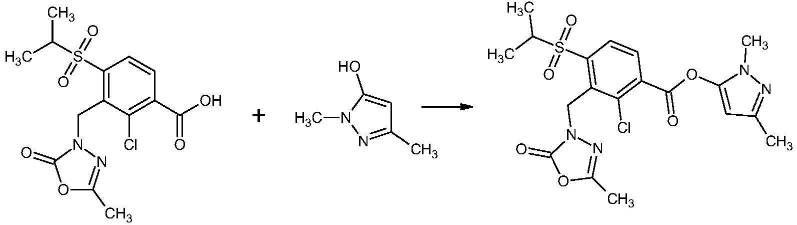

2-chloro-3-[(5-methyl-2-oxo-1,3,4-oxadiazol-3(2H)-yl)methyl)]-4-isopropylsulfonylbenzoic acid (7.5 g, 20 mmol) and dichloroethane (50 ml) were added to a reaction flask, followed by slow addition of oxalyl chloride (7.6 g, 60 mmol). The mixture was heated under reflux for 4 hours, and evaporated under reduced pressure to remove all the solvent and excess oxalyl chloride. Dichloroethane (60 mL) and 1,3-dimethyl-5-hydroxypyrazole (2.3 g, 20.5 mmol) were added, followed by dropwise addition of triethylamine (3.2 g, 32 mmol). The mixture was stirred at room temperature for 1 hour, and then water (50 mL) was added. The aqueous phase was extracted twice with dichloroethane (30 mL × 2). The combined oil phase was evaporated to remove all the solvent, and the residue was separated by column chromatography to give 6.1 g of a pale yellow solid in 65% yield.

### (3) Synthesis of Compound 100 (1,3-dimethyl-4-{2-chloro-3-[(5-methyl-2-oxo-1,3,4-oxadiazol-3(2H)yl)methyl]-4-isopropylsulfon ylbenzoyl}-5-hydroxypyrazole)

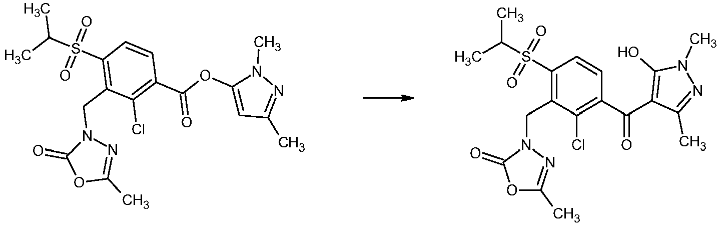

1,3-dimethylpyrazol-5-yl 2-chloro-3-[(5-methyl-2-oxo-1,3,4-oxadiazol-3(2H)-yl)methyl)]-4-isopropylsulfonylbenzoate (6.1 g, 13 mmol), dichloromethane (100 mL), triethylamine (1.9 g, 19 mmol), and acetone cyanohydrin (2 ml) were added to a reaction flask. The reaction was conducted at room temperature for 12 hours, and then water (60 mL) was added to the reaction solution for extraction. After stirring for half an hour, the pH of the solution was adjusted to 2-3 with 10% dilute hydrochloric acid. The aqueous phase was extracted twice with ethyl acetate (100 mL × 2). The combined organic phase was washed with saturated brine (60 mL), dried over anhydrous magnesium sulfate, and evaporated under reduced pressure to remove all the solvent, affording 5.8 g of a pale yellow solid in 96% yield.

### Example 4: Synthesis of Compound 171

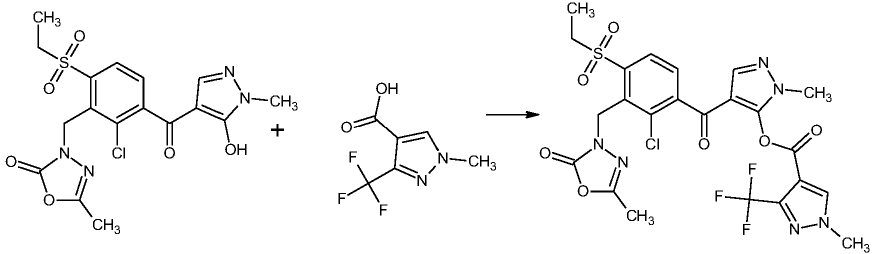

1-methyl-3-trifluoromethyl-4-pyrazolecarboxylic acid (4.5 g, 23 mmol) and dichloroethane (80 mL) were added to a reaction flask, followed by slow addition of sulfoxide chloride (7.1 g, 60 mmol). The mixture was heated under reflux for 4 hours and then evaporated under reduced pressure to remove all the solvent and excess sulfoxide chloride. Dichloroethane (80 mL), and 3-{2-chloro-3-[(1-methyl-5-hydroxy-1H-pyrazol-4-yl)carbonyl]-6-ethylsulfonylbenzyl}-5-methyl-1,3,4-oxadiazol-2(3H)-one (7.2 g, 15.8 mmol) were added, followed by dropwise addition of triethylamine (3 g, 30 mmol). The mixture was stirred at room temperature for 1 hour, and then water (50 mL) was added. The aqueous phase was extracted twice with dichloroethane (30 mL × 2). The combined oil phase was washed with saturated brine (60 mL), dried over anhydrous magnesium sulfate, and evaporated under reduced pressure to remove all the solvent, and then the residue was separated by column chromatography to give 10.1 g of a solid in 70% yield.

### Example 5: Test of herbicidal activity

Seeds of broadleaf weeds (abutilon, Solanum nigrum) or gramineous weeds (crabgrass, barnyard grass) were sown into nutrient soil in plastic cups, respectively, and then covered with soil, compacted, watered, and further cultivated in a greenhouse by conventional methods. The stems and leaves of the weeds were sprayed when the weeds reached the 2- to 3-leaf stage. The test compounds of the present application were dissolved in acetone respectively, followed by addition of Tween 80. The solution was diluted with a certain amount of water to a certain solubility, and then sprayed onto the plants using a spray tower. The test was repeated three times. After drying naturally in the shade, the plants were put in a greenhouse and managed according to conventional methods. The growth and development of weeds were observed, and the control effect of the test agent on weeds was checked visually periodically, which was expressed by 0-100%, with "0" representing no control effect, and "100%" representing complete killing or severe inhibition.

The experimental results showed that the compounds of Formula (I) generally showed higher control effect on broadleaf weeds and gramineous weeds. Some of the test compounds, such as Compounds 1, 5, 6, 11, 16, 32, 45, 46, 47, 49, 51, 53, 58, 154, 166, 194, 215, 223, 224, 227, 228, 231, 233, 238, 244, 246, and 250 showed better control effect on crabgrass at a dose of 300 g a.i/ha, which was greater than 80%. For example, Compounds 1, 5, 32, 56, 58, 59, 68, 72, 82, 96, 100, 149, 150, 152, 153, 154, 166, 194, 201, 215, 223, 224, 227, 228, 233, and 238 showed better control effect on barnyard grass at a dose of 300 g a.i/ha, which was greater than 80%. For example, Compounds 1, 5, 48, 53, 58, 68, 72, 96, 100, 150, 152, 154, 166, 171, 178, 180, 182, 185, 187, 190, 201, 215, 223, 224, 225, 226, 227, 228, 230, 231, 233, 235, 236, 238, 240, 241, 242, 243, 244, 245, 246, 248, 249, 250, 251, 252, 253, and 254 showed better control effect on abutilon and *Solanum nigrum* at a dose of 300 g a.i/ha, which was greater than 90%.

According to the above test method, Compounds 1, 32, 49, 166, 194, 201, 204, 218, 224, 228 and Control compounds A, B, and C were selected for herbicidal activity test at the same time, and the data of activity against abutilon, *Solanum nigrum,* crabgrass, and barnyard grass are shown in Table 5, which shows the post-seedling weed test results of the compounds provided in the examples herein.

**Table 5 Post-seedling weed test results for the compounds provided in the examples herein**

| Test agent | Dose (g a.i/ha) | Abutilon (%) | *Solanum nigrum* (%) | Crabgrass (%) | Barnyard grass (%) |
|---|---|---|---|---|---|
| 1 | 300 | 100 | 100 | 95 | 98 |
| | 150 | 98 | 95 | 85 | 90 |
| | 75 | 90 | 90 | 75 | 80 |
| | 37.5 | 65 | 60 | 50 | 55 |
| 32 | 300 | 100 | 100 | 90 | 95 |
| | 150 | 98 | 95 | 85 | 90 |
| | 75 | 90 | 90 | 75 | 80 |
| | 37.5 | 65 | 60 | 50 | 55 |
| 49 | 300 | 100 | 100 | 90 | 92 |
| | 150 | 95 | 90 | 85 | 88 |
| | 75 | 90 | 80 | 75 | 70 |
| | 37.5 | 65 | 60 | 50 | 55 |
| 166 | 300 | 95 | 93 | 85 | 90 |
| | 150 | 92 | 90 | 80 | 85 |
| | 75 | 88 | 85 | 75 | 80 |
| | 37.5 | 65 | 60 | 50 | 55 |
| 194 | 300 | 97 | 96 | 85 | 88 |
| | 150 | 92 | 90 | 82 | 85 |
| | 75 | 85 | 80 | 75 | 80 |
| | 37.5 | 65 | 60 | 50 | 55 |
| 201 | 300 | 98 | 100 | 90 | 100 |
| | 150 | 92 | 95 | 80 | 95 |
| | 75 | 80 | 75 | 65 | 75 |
| | 37.5 | 55 | 55 | 45 | 55 |
| 204 | 300 | 98 | 97 | 88 | 90 |
| | 150 | 90 | 90 | 80 | 85 |
| | 75 | 80 | 70 | 64 | 70 |
| | 37.5 | 55 | 52 | 45 | 50 |
| 218 | 300 | 95 | 95 | 85 | 90 |
| | 150 | 90 | 90 | 80 | 85 |
| | 75 | 80 | 70 | 65 | 70 |
| | 37.5 | 55 | 50 | 45 | 50 |
| 224 | 300 | 92 | 93 | 84 | 88 |
| | 150 | 90 | 90 | 80 | 85 |
| | 75 | 80 | 70 | 65 | 70 |
| | 37.5 | 55 | 50 | 45 | 50 |
| 228 | 300 | 95 | 95 | 100 | 100 |
| | 150 | 90 | 90 | 95 | 95 |
| | 75 | 80 | 70 | 75 | 80 |
| | 37.5 | 55 | 50 | 55 | 60 |
| Control compound A | 300 | 100 | 95 | 50 | 60 |
| | 150 | 98 | 90 | 45 | 50 |
| | 75 | 80 | 70 | 25 | 30 |
| | 37.5 | 50 | 50 | 5 | 10 |
| Control compound B | 300 | 85 | 70 | 45 | 55 |
| | 150 | 75 | 65 | 40 | 45 |
| | 75 | 55 | 50 | 15 | 25 |
| | 37.5 | 45 | 40 | 5 | 10 |
| Control compound C | 300 | 90 | 85 | 70 | 75 |
| | 150 | 70 | 65 | 65 | 70 |
| | 75 | 55 | 50 | 55 | 60 |
| | 37.5 | 45 | 40 | 20 | 35 |

In the table, Control compound A was

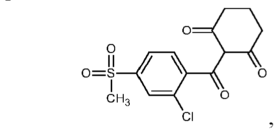

Control compound B was 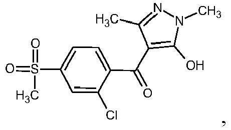 and
Control compound C was

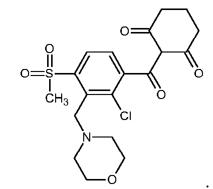

As can be seen from Table 5, Compounds 1, 32, 49, 166, 194, 201, 204, 218, 224, and 228 provided herein had better herbicidal activities against abutilon, *Solanum nigrum,* crabgrass, barnyard grass and the like at different doses compared with Control compounds A, B and C.

### Example 6: Safety testing on corn and rice

The seeds of corn and rice were sown into nutrient soil in plastic cups, respectively, then covered with soil, compacted, watered, and further cultivated in a greenhouse by conventional methods. The stems and leaves of the crops were sprayed when the crops reached the 2- to 3-leaf stage. The test compounds of the present application were dissolved in acetone, followed by addition of Tween 80. The solution was then diluted with a certain amount of water to a certain solubility, and sprayed onto the plants using a spray tower. The test was repeated three times. After drying naturally in the shade, the plants were put in a greenhouse and managed according to conventional methods. The growth and development of corn and rice were observed, and the safety of the test agent on corn and rice was checked visually periodically. A safety grading standard was represented by 0-100%, with "0" representing no damage to crops, and "100%" representing complete killing or severe inhibition of crops. Some test results are shown in Table 6, which shows the safety test results on corn and rice for the compounds provided in the examples herein.

**Table 6 Safety test results on corn and rice for the compounds provided in the examples herein**

| Test agent | Dose (g a.i/ha) | Corn (%) | Rice (%) |
|---|---|---|---|
| 6 | 300 | 5 | 10 |
| | 150 | 0 | 5 |
| | 75 | 0 | 0 |
| | 37.5 | 0 | 0 |
| 58 | 300 | 0 | 5 |
| | 150 | 0 | 0 |
| | 75 | 0 | 0 |
| | 37.5 | 0 | 0 |
| 100 | 300 | 0 | 2 |
| | 150 | 0 | 0 |
| | 75 | 0 | 0 |
| | 37.5 | 0 | 0 |
| 201 | 300 | 0 | 2 |
| | 150 | 0 | 0 |
| | 75 | 0 | 0 |
| | 37.5 | 0 | 0 |
| 224 | 300 | 2 | 5 |
| | 150 | 0 | 2 |
| | 75 | 0 | 0 |
| | 37.5 | 0 | 0 |
| 228 | 300 | 3 | 5 |
| | 150 | 0 | 2 |
| | 75 | 0 | 0 |
| | 37.5 | 0 | 0 |
| 233 | 300 | 2 | 5 |
| | 150 | 0 | 2 |
| | 75 | 0 | 0 |
| | 37.5 | 0 | 0 |
| Control compound A | 300 | 5 | 60 |
| | 150 | 0 | 45 |
| | 75 | 0 | 25 |
| | 37.5 | 0 | 10 |
| Control compound B | 300 | 2 | 55 |
| | 150 | 0 | 40 |
| | 75 | 0 | 20 |
| | 37.5 | 0 | 10 |
| Control compound C | 300 | 5 | 85 |
| | 150 | 2 | 75 |
| | 75 | 0 | 55 |
| | 37.5 | 0 | 30 |

### Control compound A:

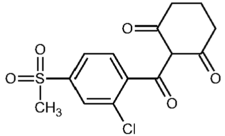

### Control compound B:

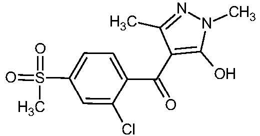

### Control compound C:

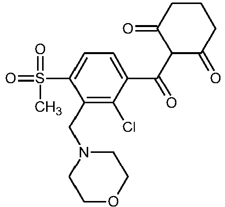

As can be seen from Table 6, Compounds 6, 58, 100, 201, 224, 228, and 233 provided herein showed no significant inhibitory effect on both corn and rice at different doses as compared to Control compounds A, B, and C.

Finally, it should be noted that the above embodiments are only used to illustrate the technical solutions of the present invention and not to limit it; although the present invention has been described in detail with reference to the foregoing embodiments, it will be understood by one of ordinary skill in the art that the technical solutions described in the foregoing embodiments can still be modified or some technical features can be equivalently substituted; however, these modifications or substitutions do not make the essence of the corresponding technical solutions departing from the spirit and scope of the present invention.

### Industrial Applicability

In the present application, an oxadiazolone group of Formula (a) is provided as a substituting group on the position between the sulfonyl group and Cl on the benzene ring of a benzoyl compound to obtain an oxadiazolone compound, which not only exhibits unexpectedly high herbicidal activity against various weeds, but also is safe for various crops such as corn and rice. The experimental results show that the compound of the present application exhibits good herbicidal activity and can effectively control weeds (such as barnyard grass, crabgrass, and abutilon) at a low dose to achieve an excellent herbicidal effect, in the meanwhile, it demonstrates higher safety for crops.

## Claims

1. An oxadiazolone compound of Formula (I) or tautomer thereof: 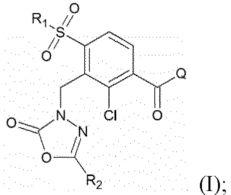
in Formula (I), R₁ and R₂ are independently selected from the group consisting of unsubstituted or substituted C₁-C₆ alkyl and C₃-C₆ cycloalkyl, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy;
Q is selected from a structure of Q1, Q2 or Q3: 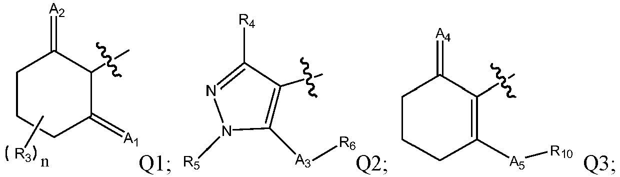
wherein, A₁, A₂, A₃, A₄, and A₅ are independently selected from O and S;
R₃ and R₁₀ are independently selected from the group consisting of hydrogen, unsubstituted or substituted C₁-C₆ alkyl, C₃-C₆ cycloalkyl, C₁-C₆ alkoxy, phenyl, and benzyl, wherein the substituents are 1 to 3 groups independently selected from the group consisting of halogen, nitro, C₁-C₆ alkyl, C₁-C₆ haloalkyl, C₁-C₆ alkoxy, C₁-C₆ haloalkoxy, phenyl, and halophenyl;
n is an integer from 1 to 6;
R₄ is selected from the group consisting of hydrogen, halogen, unsubstituted or substituted C₁-C₆ alkyl, C₃-C₆ cycloalkyl, and C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy;
R₅ is selected from the group consisting of hydrogen, unsubstituted or substituted C₁-C₆ alkyl, and C₃-C₆ cycloalkyl, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy;
R₆ is selected from the group consisting of hydrogen, a structure of Formula (1), unsubstituted or substituted C₁-C₆ alkyl, C₃-C₆ cycloalkyl, and C₃-C₈ heterocycloalkyl, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy; 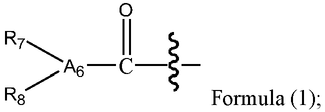
in Formula (1), A₆ is selected from the group consisting of N and CR₉, R₉ is selected from the group consisting of hydrogen, unsubstituted or substituted C₁-C₆ alkyl, C₃-C₆ cycloalkyl, and C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy;
R₇ and R₈ are independently selected from the group consisting of hydrogen, unsubstituted or substituted C₁-C₆ alkyl, C₃-C₆ cycloalkyl, and C₁-C₆ alkoxy, wherein the substituents are 1 to 3 groups independently selected from the group consisting of halogen, nitro, C₁-C₆ alkyl, C₁-C₆ haloalkyl, C₁-C₆ alkoxy, and C₁-C₆ haloalkoxy; R₇ and R₈ are not both H;
or, R₇ and R₈ together with A₆ to which they are attached form an unsubstituted or substituted C₃-C₈ ring comprising 0-3 heteroatoms, wherein the substituents are 1 to 3 groups independently selected from the group consisting of halogen, nitro, C₁-C₆ alkyl, C₃-C₆ cycloalkyl, C₁-C₆ haloalkyl, C₁-C₆ alkoxy, and C₁-C₆ haloalkoxy; the heteroatoms are selected from the group consisting of oxygen, sulfur, and nitrogen.

2. The oxadiazolone compound or tautomer thereof of claim 1, **characterized in that** Q is selected from a structure of Q1-1: 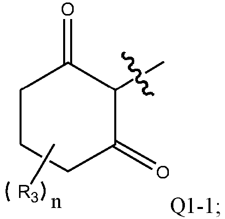
R₃ is selected from the group consisting of H, C₁-C₄ alkyl, and C₁-C₄ haloalkyl;
R₁ and R₂ are independently selected from the group consisting of C₁-C₄ alkyl, C₃-C₆ cycloalkyl, and C₁-C₄ haloalkyl;
or,
Q is selected from a structure of Q3-1: 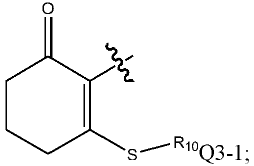
R₁₀ is selected from the group consisting of H, C₁-C₄ alkyl, C₁-C₄ haloalkyl, phenyl, halophenyl, phenyl-substituted C₁-C₄ alkyl, and halophenyl-substituted C₁-C₄ alkyl;
R₁ and R₂ are independently selected from the group consisting of C₁-C₄ alkyl, C₃-C₆ cycloalkyl, and C₁-C₄ haloalkyl;
or,
Q is selected from a structure of Q2-1: 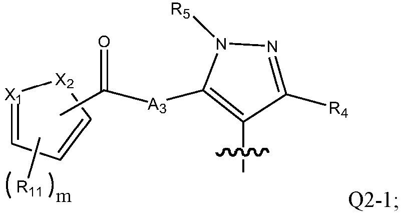
X₁ is selected from C or N, X₂ is selected from C, N, O, or S, and X₁ and X₂ are not both C;
R₁₁ is selected from the group consisting of H, halogen, C₁-C₄ alkyl, C₃-C₆ cycloalkyl, and C₁-C₄ haloalkyl;
m is an integer from 1 to 3;
R₄ is selected from the group consisting of hydrogen, halogen, C₁-C₄ alkyl, C₁-C₄ haloalkyl, C₃-C₆ cycloalkyl, C₃-C₆ halocycloalkyl, C₁-C₄ alkoxy, and C₁-C₄ haloalkoxy;
R₅ is selected from the group consisting of hydrogen, C₁-C₄ alkyl, and C₁-C₄ haloalkyl;
R₁ and R₂ are independently selected from the group consisting of C₁-C₄ alkyl, C₃-C₆ cycloalkyl, and C₁-C₄ haloalky.

3. The oxadiazolone compound or tautomer thereof of claim 2, **characterized in that** R₁ and R₂ are independently selected from the group consisting of -CH₃, -CH₂CH₃, -CH₂CH₂CH₃, -CH(CH₃)₂, -C(CH₃)₃, -CH₂CH₂CH₂CH₃, -CF₃, -CH₂CF₃, -CH₂CH₂CF₃, -CH(CF₃)₂, -C(CF₃)₃, -CHCH₃CF₃, -C(CH₃)₂CF₃, and CCH₂(CF₃)₂.

4. The oxadiazolone compound or tautomer thereof of claim 3, **characterized in that** R₁₀ is selected from the group consisting of H, -CH₃, -CH₂CH₃, -CH₂CH₂CH₃, -CH(CH₃)₂, -C(CH₃)₃, -CH₂CH₂CH₂CH₃, phenyl, chlorophenyl, dichlorophenyl, C₆H₅CH₂-, C₆H₄ClCH₂-, and C₆H₃Cl₂CH₂-;
R₁₁ is selected from the group consisting of H, CH₃, CF₃, -CH₂CH₃, cyclopropyl, and Cl.

5. The oxadiazolone compound or tautomer thereof of claim 1, **characterized in that** R₆ is selected from hydrogen or a structure of Formula (1): 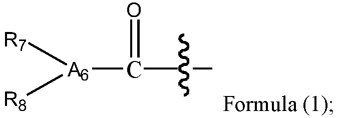
in Formula (1), A₆ is selected from N, R₇ and R₈ are independently selected from the group consisting of hydrogen, C₁-C₄ alkyl, C₁-C₄ haloalkyl, C₃-C₆ cycloalkyl, C₁-C₄ alkoxy, and C₁-C₄ haloalkoxy; R₇ and R₈ are not both H;
or, A₆ is selected from N or C, R₇ and R₈ together with A₆ to which they are attached form an unsubstituted or substituted C₃-C₈ ring comprising 1-3 heteroatoms, wherein the substituents are 1 to 3 groups independently selected from the group consisting of halogen, nitro, C₁-C₄ alkyl, C₁-C₄ haloalkyl, C₁-C₄ alkoxy, and C₁-C₄ haloalkoxy; the heteroatoms are selected from the group consisting of oxygen, sulfur, and nitrogen.

6. The oxadiazolone compound or tautomer thereof of claim 5, **characterized in that** R₆ is selected from the group consisting of the following structures: 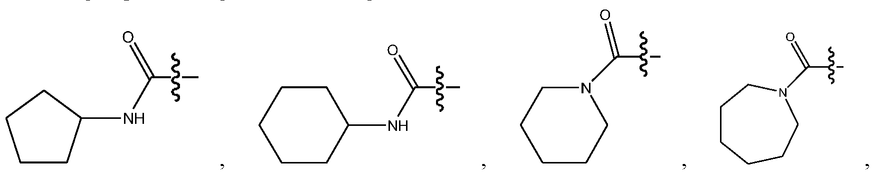 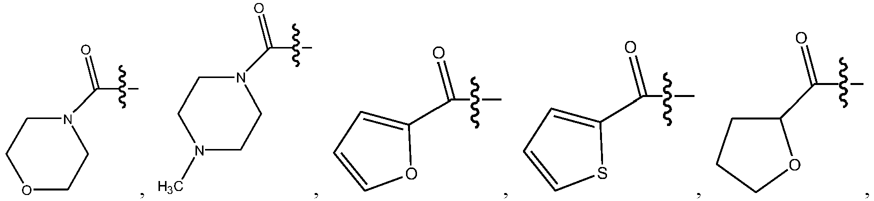 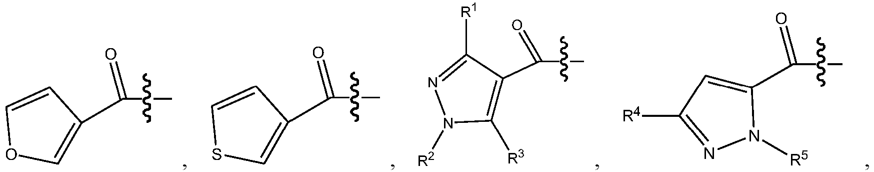 or 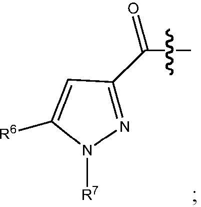
wherein R¹, R³, R⁴, and R⁶ are independently selected from the group consisting of hydrogen, halogen, C₁-C₄ alkyl, C₁-C₄ haloalkyl, and C₃-C₆ cycloalkyl;
R², R⁵, and R⁷ are independently selected from the group consisting of hydrogen, C₁-C₄ alkyl, C₁-C₄ haloalkyl, and C₃-C₆ cycloalkyl.

7. The oxadiazolone compound or tautomer thereof of claim 1, **characterized in that** the compound is selected from the group consisting of the following compounds:
Compound 1: Q is selected from the structure of Q1-1, R₁=CH₃, R₂=CH₃, R₃=H;
Compound 2: Q is selected from the structure of Q1-1, R₁=CH₃, R₂=CH₂CH₃, R₃=H;
Compound 3: Q is selected from the structure of Q1-1, R₁=CH₃, R₂=CH₂CH₂CH₃, R₃=H;
Compound 4: Q is selected from the structure of Q1-1, R₁=CH₃, R₂=C(CH₃)₃, R₃=H;
Compound 5: Q is selected from the structure of Q1-1, R₁=CH₃, R₂=CF₃, R₃=H;
Compound 6: Q is selected from the structure of Q1-1, R₁=CH₂CH₃, R₂=CH₃, R₃=H;
Compound 7: Q is selected from the structure of Q1-1, R₁=CH₂CH₃, R₂= CH₂CH₃, R₃=H;
Compound 8: Q is selected from the structure of Q1-1, R₁=CH₂CH₃, R₂=CH₂CH₂CH₃, R₃=H;
Compound 9: Q is selected from the structure of Q1-1, R₁=CH₂CH₃, R₂=C(CH₃)₃, R₃=H;
Compound 10: Q is selected from the structure of Q1-1, R₁=CH₂CH₃, R₂=CF₃, R₃=H;
Compound 11: Q is selected from the structure of Q1-1, R₁=CH₂CH₂CH₃, R₂=CH₃, R₃=H;
Compound 12: Q is selected from the structure of Q1-1, R₁=CH₂CH₂CH₃, R₂=CH₂CH₃, R₃=H;
Compound 13: Q is selected from the structure of Q1-1, R₁= CH₂CH₂CH₃, R₂=CH₂CH₂CH₃, R₃=H;
Compound 14: Q is selected from the structure of Q1-1, R₁=CH₂CH₂CH₃, R₂=C(CH₃)₃, R₃=H;
Compound 15: Q is selected from the structure of Q1-1, R₁=CH₂CH₂CH₃, R₂=CF₃, R₃=H;
Compound 16: Q is selected from the structure of Q1-1, R₁=CH(CH₃)₂, R₂=CH₃, R₃=H;
Compound 17: Q is selected from the structure of Q1-1, R₁=CH(CH₃)₂, R₂=CH₂CH₃, R₃=H;
Compound 18: Q is selected from the structure of Q1-1, R₁=CH(CH₃)₂, R₂=CH₂CH₂CH₃, R₃=H;
Compound 19: Q is selected from the structure of Q1-1, R₁=CH(CH₃)₂, R₂=C(CH₃)₃, R₃=H;
Compound 20: Q is selected from the structure of Q1-1, R₁=CH(CH₃)₂, R₂=CF₃, R₃=H;
Compound 21: Q is selected from the structure of Q1-1, R₁=CH₂CH₂CH₂CH₃, R₂=CH₃, R₃=H;
Compound 22: Q is selected from the structure of Q1-1, R₁=CH₂CH₂CH₂CH₃, R₂=CH₂CH₃, R₃=H;
Compound 23: Q is selected from the structure of Q1-1, R₁=CH₂CH₂CH₂CH₃, R₂=CH₂CH₂CH₃, R₃=H;
Compound 24: Q is selected from the structure of Q1-1, R₁=CH₂CH₂CH₂CH₃, R₂=C(CH₃)₃, R₃=H;
Compound 25: Q is selected from the structure of Q1-1, R₁=CH₂CH₂CH₂CH₃, R₂=CF₃, R₃=H;
Compound 26: Q is selected from the structure of Q1-1, R₁=C(CH₃)₃, R₂=CH₃, R₃=H;
Compound 27: Q is selected from the structure of Q1-1, R₁=C(CH₃)₃, R₂=CH₂CH₃, R₃=H;
Compound 28: Q is selected from the structure of Q1-1, R₁=C(CH₃)₃, R₂=CH₂CH₂CH₃, R₃=H;
Compound 29: Q is selected from the structure of Q1-1, R₁=C(CH₃)₃, R₂=C(CH₃)₃, R₃=H;
Compound 30: Q is selected from the structure of Q1-1, R₁=C(CH₃)₃, R₂=CF₃, R₃=H;
Compound 31: Q is selected from the structure of Q3, R₁=CH₃, R₂=CH₃, A₄=O, A₅=O, R₁₀=CH₃;
Compound 32: Q is selected from the structure of Q3, R₁=CH₃, R₂=CH₃, A₄=O, A₅=O, R₁₀= CH₂CH₃;
Compound 33: Q is selected from the structure of Q3, R₁=CH₃, R₂=CH₃, A₄=O, A₅=O, R₁₀= CH₂CH₂CH₃;
Compound 34: Q is selected from the structure of Q3, R₁=CH₃, R₂=CH₃, A₄=O, A₅=O, R₁₀= CH(CH₃)₂;
Compound 35: Q is selected from the structure of Q3, R₁=CH₃, R₂=CH₃, A₄=O, A₅=O, R₁₀= CH₂CH₂CH₂CH₃;
Compound 36: Q is selected from the structure of Q3, R₁=CH₃, R₂=CH₃, A₄=O, A₅=O, R₁₀=C(CH₃)₃;
Compound 37: Q is selected from the structure of Q3, R₁=CH₃, R₂=CH₃, A₄=O, A₅=O, 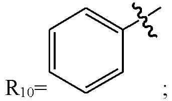
Compound 38: Q is selected from the structure of Q3, R₁=CH₃, R₂=CH₃, A₄=O, A₅=O, 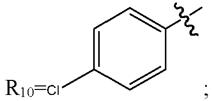
Compound 39: Q is selected from the structure of Q3, R₁=CH₃, R₂=CH₃, A₄=O, A₅=O, R₁₀= 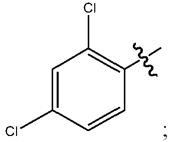
Compound 40: Q is selected from the structure of Q3, R₁=CH₃, R₂=CH₃, A₄=O, A₅=O, 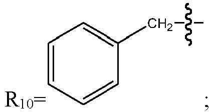
Compound 41: Q is selected from the structure of Q3, R₁=CH₃, R₂=CH₃, A₄=O, A₅=O, 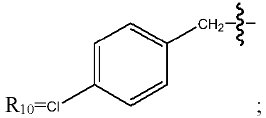
Compound 42: Q is selected from the structure of Q3, R₁=CH₃, R₂=CH₃, A₄=O, A₅=O, R₁₀= 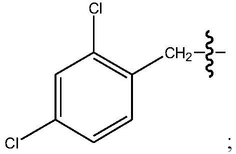
Compound 43: Q is selected from the structure of Q3, R₁=CH₃, R₂=CH₃, A₄=O, A₅=O, R₁₀= 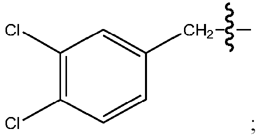
Compound 44: Q is selected from the structure of Q3, R₁=CH₃, R₂=CH₃, A₄=O, A₅=S, R₁₀= CH₃;
Compound 45: Q is selected from the structure of Q3, R₁=CH₃, R₂=CH₃, A₄=O, A₅=S, R₁₀= CH₂CH₃ ;
Compound 46: Q is selected from the structure of Q3, R₁=CH₃, R₂=CH₃, A₄=O, A₅=S, R₁₀= CH₂CH₂CH₃ ;
Compound 47: Q is selected from the structure of Q3, R₁=CH₃, R₂=CH₃, A₄=O, A₅=S, R₁₀= CH(CH₃)₂;
Compound 48: Q is selected from the structure of Q3, R₁=CH₃, R₂=CH₃, A₄=O, A₅=S, R₁₀= CH₂CH₂CH₂CH₃;
Compound 49: Q is selected from the structure of Q3, R₁=CH₃, R₂=CH₃, A₄=O, A₅=S, R₁₀= C(CH₃)₃;
Compound 50: Q is selected from the structure of Q3, R₁=CH₃, R₂=CH₃, A₄=O, A₅=S, 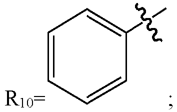
Compound 51: Q is selected from the structure of Q3, R₁=CH₃, R₂=CH₃, A₄=O, A₅=S, 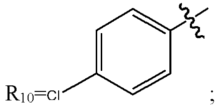
Compound 52: Q is selected from the structure of Q3, R₁=CH₃, R₂=CH₃, A₄=O, A₅=S, R₁₀= 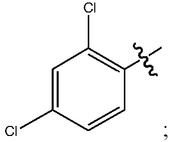
Compound 53: Q is selected from the structure of Q3, R₁=CH₃, R₂=CH₃, A₄=O, A₅=S, 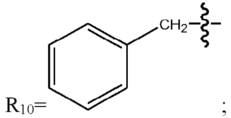
Compound 54: Q is selected from the structure of Q3, R₁=CH₃, R₂=CH₃, A₄=O, A₅=S, 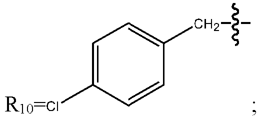
Compound 55: Q is selected from the structure of Q3, R₁=CH₃, R₂=CH₃, A₄=O, A₅=S, R₁₀= 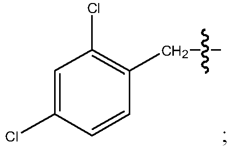
Compound 56: Q is selected from the structure of Q2, A₃=O, R₁=CH₃, R₂=CH₃, R₄=H, R₅=CH₃, R₆=H;
Compound 57: Q is selected from the structure of Q2, A₃=O, R₁=CH₃, R₂=CH₃, R₄=H, R₅=CH₂CH₃, R₆=H;
Compound 58: Q is selected from the structure of Q2, A₃=O, R₁=CH₃, R₂=CH₃, R₄=CH₃, R₅=CH₃, R₆=H;
Compound 59: Q is selected from the structure of Q2, A₃=O, R₁=CH₃, R₂=CH₃, R₄=CH₃, R₅=CH₂CH₃, R₆=H;
Compound 60: Q is selected from the structure of Q2, A₃=O, R₁=CH₃, R₂=CH₃, R₄= CH₂CH₃, R₅=CH₃, R₆=H;
Compound 61: Q is selected from the structure of Q2, A₃=O, R₁=CH₃, R₂=CH₃, R₄=CH₂CH₃, R₅=CH₂CH₃, R₆=H;
Compound 62: Q is selected from the structure of Q2, A₃=O, R₁=CH₃, R₂=CH₃, R₄=CH₂CH₂CH₃, R₅=CH₃, R₆=H;
Compound 63: Q is selected from the structure of Q2, A₃=O, R₁=CH₃, R₂=CH₃, R₄=CH₂CH₂CH₃, R₅=CH₂CH₃, R₆=H;
Compound 64: Q is selected from the structure of Q2, A₃=O, R₁=CH₃, R₂=CH₃, R₄=CH(CH₃)₂, R₅=CH₃, R₆=H;
Compound 65: Q is selected from the structure of Q2, A₃=O, R₁=CH₃, R₂=CH₃, R₄=CH(CH₃)₂, R₅=CH₂CH₃, R₆=H;
Compound 66: Q is selected from the structure of Q2, A₃=O, R₁=CH₃, R₂=CH₃, R₄=CF₃, R₅=CH₃, R₆=H;
Compound 67: Q is selected from the structure of Q2, A₃=O, R₁=CH₃, R₂=CH₃, R₄=CF₃, R₅=CH₂CH₃, R₆=H;
Compound 68: Q is selected from the structure of Q2, A₃=O, R₁=CH₃, R₂=CH₃, R₄= ◁, R₅=CH₃, R₆=H;
Compound 69: Q is selected from the structure of Q2, A₃=O, R₁=CH₃, R₂=CH₃, R₄= ◁, R₅=CH₂CH₃, R₆=H;
Compound 70: Q is selected from the structure of Q2, A₃=O, R₁=CH₂CH₃, R₂=CH₃, R₄=H, R₅=CH₃, R₆=H;
Compound 71: Q is selected from the structure of Q2, A₃=O, R₁= CH₂CH₃, R₂=CH₃, R₄=H, R₅=CH₂CH₃, R₆=H;
Compound 72: Q is selected from the structure of Q2, A₃=O, R₁= CH₂CH₃, R₂=CH₃, R₄=CH₃, R₅=CH₃, R₆=H;
Compound 73: Q is selected from the structure of Q2, A₃=O, R₁= CH₂CH₃, R₂=CH₃, R₄=CH₃, R₅=CH₂CH₃, R₆=H;
Compound 74: Q is selected from the structure of Q2, A₃=O, R₁=CH₂CH₃, R₂=CH₃, R₄= CH₂CH₃, R₅=CH₃, R₆=H;
Compound 75: Q is selected from the structure of Q2, A₃=O, R₁=CH₂CH₃, R₂=CH₃, R₄=CH₂CH₃, R₅=CH₂CH₃, R₆=H;
Compound 76: Q is selected from the structure of Q2, A₃=O, R₁=CH₂CH₃, R₂=CH₃, R₄=CH₂CH₂CH₃, R₅=CH₃, R₆=H;
Compound 77: Q is selected from the structure of Q2, A₃=O, R₁=CH₂CH₃, R₂=CH₃, R₄=CH₂CH₂CH₃, R₅=CH₂CH₃, R₆=H;
Compound 78: Q is selected from the structure of Q2, A₃=O, R₁=CH₂CH₃, R₂=CH₃, R₄=CH(CH₃)₂, R₅=CH₃, R₆=H;
Compound 79: Q is selected from the structure of Q2, A₃=O, R₁=CH₂CH₃, R₂=CH₃, R₄=CH(CH₃)₂, R₅=CH₂CH₃, R₆=H;
Compound 80: Q is selected from the structure of Q2, A₃=O, R₁=CH₂CH₃, R₂=CH₃, R₄=CF₃, R₅=CH₃, R₆=H;
Compound 81: Q is selected from the structure of Q2, A₃=O, R₁=CH₂CH₃, R₂=CH₃, R₄=CF₃, R₅=CH₂CH₃, R₆=H;
Compound 82: Q is selected from the structure of Q2, A₃=O, R₁=CH₂CH₃, R₂=CH₃, R₄= ◁, R₅=CH₃, R₆=H;
Compound 83: Q is selected from the structure of Q2, A₃=O, R₁=CH₂CH₃, R₂=CH₃, R₄= ◁ , R₅=CH₂CH₃, R₆=H;
Compound 84: Q is selected from the structure of Q2, A₃=O, R₁=CH₂CH₂CH₃, R₂=CH₃, R₄=H, R₅=CH₃, R₆=H;
Compound 85: Q is selected from the structure of Q2, A₃=O, R₁=CH₂CH₂CH₃, R₂=CH₃, R₄=H, R₅=CH₂CH₃, R₆=H;
Compound 86: Q is selected from the structure of Q2, A₃=O, R₁=CH₂CH₂CH₃, R₂=CH₃, R₄=CH₃, R₅=CH₃, R₆=H;
Compound 87: Q is selected from the structure of Q2, A₃=O, R₁=CH₂CH₂CH₃, R₂=CH₃, R₄=CH₃, R₅=CH₂CH₃, R₆=H;
Compound 88: Q is selected from the structure of Q2, A₃=O, R₁=CH₂CH₂CH₃, R₂=CH₃, R₄= CH₂CH₃, R₅=CH₃, R₆=H;
Compound 89: Q is selected from the structure of Q2, A₃=O, R₁=CH₂CH₂CH₃, R₂=CH₃, R₄=CH₂CH₃, R₅=CH₂CH₃, R₆=H;
Compound 90: Q is selected from the structure of Q2, A₃=O, R₁=CH₂CH₂CH₃, R₂=CH₃, R₄=CH₂CH₂CH₃, R₅=CH₃, R₆=H;
Compound 91: Q is selected from the structure of Q2, A₃=O, R₁=CH₂CH₂CH₃, R₂=CH₃, R₄=CH₂CH₂CH₃, R₅=CH₂CH₃, R₆=H;
Compound 92: Q is selected from the structure of Q2, A₃=O, R₁=CH₂CH₂CH₃, R₂=CH₃, R₄=CH(CH₃)₂, R₅=CH₃, R₆=H;
Compound 93: Q is selected from the structure of Q2, A₃=O, R₁=CH₂CH₂CH₃, R₂=CH₃, R₄=CH(CH₃)₂, R₅=CH₂CH₃, R₆=H;
Compound 94: Q is selected from the structure of Q2, A₃=O, R₁=CH₂CH₂CH₃, R₂=CH₃, R₄=CF₃, R₅=CH₃, R₆=H;
Compound 95: Q is selected from the structure of Q2, A₃=O, R₁=CH₂CH₂CH₃, R₂=CH₃, R₄=CF₃, R₅=CH₂CH₃, R₆=H;
Compound 96: Q is selected from the structure of Q2, A₃=O, R₁=CH₂CH₂CH₃, R₂=CH₃, R₄= ◁, R₅=CH₃, R₆=H;
Compound 97: Q is selected from the structure of Q2, A₃=O, R₁=CH₂CH₂CH₃, R₂=CH₃, R₄= ◁, R₅=CH₂CH₃, R₆=H;
Compound 98: Q is selected from the structure of Q2, A₃=O, R₁=CH(CH₃)₂, R₂=CH₃, R₄=H, R₅=CH₃, R₆=H;
Compound 99: Q is selected from the structure of Q2, A₃=O, R₁=CH(CH₃)₂, R₂=CH₃, R₄=H, R₅=CH₂CH₃, R₆=H;
Compound 100: Q is selected from the structure of Q2, A₃=O, R₁=CH(CH₃)₂, R₂=CH₃, R₄=CH₃, R₅=CH₃, R₆=H;
Compound 101: Q is selected from the structure of Q2, A₃=O, R₁=CH(CH₃)₂, R₂=CH₃, R₄=CH₃, R₅=CH₂CH₃, R₆=H;
Compound 102: Q is selected from the structure of Q2, A₃=O, R₁=CH(CH₃)₂, R₂=CH₃, R₄= CH₂CH₃, R₅=CH₃, R₆=H;
Compound 103: Q is selected from the structure of Q2, A₃=O, R₁=CH(CH₃)₂, R₂=CH₃, R₄=CH₂CH₃, R₅=CH₂CH₃, R₆=H;
Compound 104: Q is selected from the structure of Q2, A₃=O, R₁=CH(CH₃)₂, R₂=CH₃, R₄=CH₂CH₂CH₃, R₅=CH₃, R₆=H;
Compound 105: Q is selected from the structure of Q2, A₃=O, R₁=CH(CH₃)₂, R₂=CH₃, R₄=CH₂CH₂CH₃, R₅=CH₂CH₃, R₆=H;
Compound 106: Q is selected from the structure of Q2, A₃=O, R₁=CH(CH₃)₂, R₂=CH₃, R₄=CH(CH₃)₂, R₅=CH₃, R₆=H;
Compound 107: Q is selected from the structure of Q2, A₃=O, R₁=CH(CH₃)₂, R₂=CH₃, R₄=CH(CH₃)₂, R₅=CH₂CH₃, R₆=H;
Compound 108: Q is selected from the structure of Q2, A₃=O, R₁=CH(CH₃)₂, R₂=CH₃, R₄=CF₃, R₅=CH₃, R₆=H;
Compound 109: Q is selected from the structure of Q2, A₃=O, R₁=CH(CH₃)₂, R₂=CH₃, R₄=CF₃, R₅=CH₂CH₃, R₆=H;
Compound 110: Q is selected from the structure of Q2, A₃=O, R₁=CH(CH₃)₂, R₂=CH₃, R₄= ◁ , R₅=CH₃, R₆=H;
Compound 111: Q is selected from the structure of Q2, A₃=O, R₁=CH(CH₃)₂, R₂=CH₃, R₄= ◁ , R₅=CH₂CH₃, R₆=H;
Compound 112: Q is selected from the structure of Q2, A₃=O, R₁=CH₂CH₂CH₂CH₃, R₂=CH₃, R₄=H, R₅=CH₃, R₆=H;
Compound 113: Q is selected from the structure of Q2, A₃=O, R₁=CH₂CH₂CH₂CH₃, R₂=CH₃, R₄=H, R₅=CH₂CH₃, R₆=H;
Compound 114: Q is selected from the structure of Q2, A₃=O, R₁=CH₂CH₂CH₂CH₃, R₂=CH₃, R₄=CH₃, R₅=CH₃, R₆=H;
Compound 115: Q is selected from the structure of Q2, A₃=O, R₁=CH₂CH₂CH₂CH₃, R₂=CH₃, R₄=CH₃, R₅=CH₂CH₃, R₆=H;
Compound 116: Q is selected from the structure of Q2, A₃=O, R₁=CH₂CH₂CH₂CH₃, R₂=CH₃, R₄= CH₂CH₃, R₅=CH₃, R₆=H;
Compound 117: Q is selected from the structure of Q2, A₃=O, R₁=CH₂CH₂CH₂CH₃, R₂=CH₃, R₄=CH₂CH₃, R₅=CH₂CH₃, R₆=H;
Compound 118: Q is selected from the structure of Q2, A₃=O, R₁=CH₂CH₂CH₂CH₃, R₂=CH₃, R₄=CH₂CH₂CH₃, R₅=CH₃, R₆=H;
Compound 119: Q is selected from the structure of Q2, A₃=O, R₁=CH₂CH₂CH₂CH₃, R₂=CH₃, R₄=CH₂CH₂CH₃, R₅=CH₂CH₃, R₆=H;
Compound 120: Q is selected from the structure of Q2, A₃=O, R₁= CH₂CH₂CH₂CH₃, R₂=CH₃, R₄=CH(CH₃)₂, R₅=CH₃, R₆=H;
Compound 121: Q is selected from the structure of Q2, A₃=O, R₁= CH₂CH₂CH₂CH₃, R₂=CH₃, R₄=CH(CH₃)₂, R₅=CH₂CH₃, R₆=H;
Compound 122: Q is selected from the structure of Q2, A₃=O, R₁=CH₂CH₂CH₂CH₃, R₂=CH₃, R₄=CF₃, R₅=CH₃, R₆=H;
Compound 123: Q is selected from the structure of Q2, A₃=O, R₁= CH₂CH₂CH₂CH₃, R₂=CH₃, R₄=CF₃, R₅=CH₂CH₃, R₆=H;
Compound 124: Q is selected from the structure of Q2, A₃=O, R₁=CH₂CH₂CH₂CH₃, R₂=CH₃, R₄= 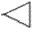 , R₅=CH₃, R₆=H;
Compound 125: Q is selected from the structure of Q2, A₃=O, R₁=CH₂CH₂CH₂CH₃, R₂=CH₃, R₄= 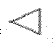 , R₅=CH₂CH₃, R₆=H;
Compound 126: Q is selected from the structure of Q2, A₃=O, R₁=C(CH₃)₃, R₂=CH₃, R₄=H, R₅=CH₃, R₆=H;
Compound 127: Q is selected from the structure of Q2, A₃=O, R₁= C(CH₃)₃, R₂=CH₃, R₄=H, R₅=CH₂CH₃, R₆=H;
Compound 128: Q is selected from the structure of Q2, A₃=O, R₁= C(CH₃)₃, R₂=CH₃, R₄=CH₃, R₅=CH₃, R₆=H;
Compound 129: Q is selected from the structure of Q2, A₃=O, R₁= C(CH₃)₃, R₂=CH₃, R₄=CH₃, R₅=CH₂CH₃, R₆=H;
Compound 130: Q is selected from the structure of Q2, A₃=O, R₁=C(CH₃)₃, R₂=CH₃, R₄= CH₂CH₃, R₅=CH₃, R₆=H;
Compound 131: Q is selected from the structure of Q2, A₃=O, R₁=C(CH₃)₃, R₂=CH₃, R₄=CH₂CH₃, R₅=CH₂CH₃, R₆=H;
Compound 132: Q is selected from the structure of Q2, A₃=O, R₁=C(CH₃)₃, R₂=CH₃, R₄=CH₂CH₂CH₃, R₅=CH₃, R₆=H;
Compound 133: Q is selected from the structure of Q2, A₃=O, R₁=C(CH₃)₃, R₂=CH₃, R₄=CH₂CH₂CH₃, R₅=CH₂CH₃, R₆=H;
Compound 134: Q is selected from the structure of Q2, A₃=O, R₁= C(CH₃)₃, R₂=CH₃, R₄=CH(CH₃)₂, R₅=CH₃, R₆=H;
Compound 135: Q is selected from the structure of Q2, A₃=O, R₁= C(CH₃)₃, R₂=CH₃, R₄=CH(CH₃)₂, R₅=CH₂CH₃, R₆=H;
Compound 136: Q is selected from the structure of Q2, A₃=O, R₁= C(CH₃)₃, R₂=CH₃, R₄=CF₃, R₅=CH₃, R₆=H;
Compound 137: Q is selected from the structure of Q2, A₃=O, R₁= C(CH₃)₃, R₂=CH₃, R₄=CF₃, R₅=CH₂CH₃, R₆=H;
Compound 138: Q is selected from the structure of Q2, A₃=O, R₁= C(CH₃)₃, R₂=CH₃, R₄= 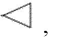 R₅=CH₃, R₆=H;
Compound 139: Q is selected from the structure of Q2, A₃=O, R₁= C(CH₃)₃, R₂=CH₃, R₄= 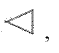 R₅=CH₂CH₃, R₆=H;
Compound 140: Q is selected from the structure of Q2, A₃=S, R₁=CH₃, R₂=CH₃, R₄=CH₃, R₅=CH₃, R₆=(CH₃CH₂)₂NCO;
Compound 141: Q is selected from the structure of Q2, A₃=O, R₁=CH₃, R₂=CH₃, R₄=CH₃, R₅=CH₃, R₆=(CH₃CH₂CH₂)₂NCO;
Compound 142: Q is selected from the structure of Q2, A₃=O, R₁=CH₂CH₃, R₂=CH₃, R₄=CH₃, R₅=CH₃, R₆=(CH₃CH₂CH₂)₂NCO;
Compound 143: Q is selected from the structure of Q2, A₃=O, R₁=CH(CH₃)₂, R₂=CH₃, R₄=CH₃, R₅=CH₃, R₆=(CH₃CH₂CH₂)₂NCO;
Compound 144: Q is selected from the structure of Q2, A₃=O, R₁=CH₃, R₂=CH₃, R₄= 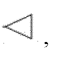 R₅=CH₃, R₆=(CH₃CH₂CH₂)₂NCO;
Compound 145: Q is selected from the structure of Q2, A₃=S, R₁=CH₃, R₂=CH₃, R₄=CH₃, R₅=CH₃, R₆=(CH₃OCH₂CH₂)₂NCO;
Compound 146: Q is selected from the structure of Q2, A₃=S, R₁=CH₃, R₂=CH₃, R₄=CH₃, R₅=CH₃, R₆= 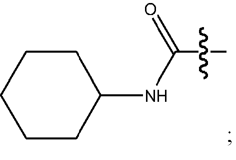
Compound 147: Q is selected from the structure of Q2, A₃=S, R₁=CH₃, R₂=CH₃, R₄=CH₃, R₅=CH₃, R₆= 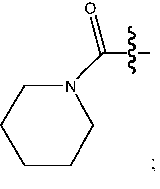
Compound 148: Q is selected from the structure of Q2, A₃=S, R₁=CH₃, R₂=CH₃, R₄=CH₃, R₅=CH₃, R₆= 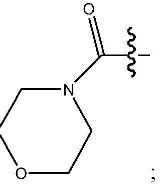
Compound 149: Q is selected from the structure of Q2, A₃=O, R₁=CH₃, R₂=CH₃, R₄=CH₃, R₅=CH₃, R₆= 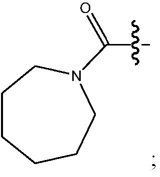
Compound 150: Q is selected from the structure of Q2, A₃=O, R₁=CH₂CH₃, R₂=CH₃, R₄=CH₃, R₅=CH₃, R₆= 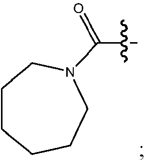
Compound 151: Q is selected from the structure of Q2, A₃=O, R₁=CH(CH₃)₂, R₂=CH₃, R₄=CH₃, R₅=CH₃, R₆= 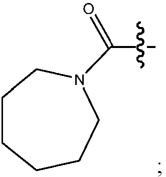
Compound 152: Q is selected from the structure of Q2, A₃=O, R₁=CH₃, R₂=CH₃, R₄= 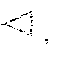 R₅=CH₃, R₆= 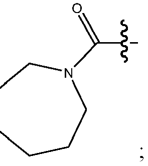
Compound 153: Q is selected from the structure of Q2, A₃=O, R₁=CH₂CH₃, R₂=CH₃, 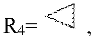 R₅=CH₃, 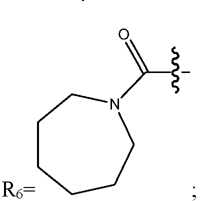
Compound 154: Q is selected from the structure of Q2, A₃=O, R₁=CH₃, R₂=CH₃, R₄=CH₃, R₅=CH₃, 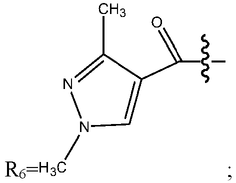
Compound 155: Q is selected from the structure of Q2, A₃=O, R₁=CH₂CH₃, R₂=CH₃, R₄=CH₃, R₅=CH₃, 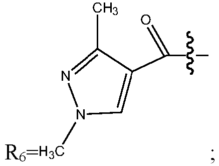
Compound 156: Q is selected from the structure of Q2, A₃=O, R₁=CH₂CH₂CH₃, R₂=CH₃, R₄=CH₃, R₅=CH₃, 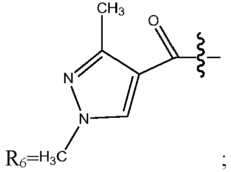
Compound 157: Q is selected from the structure of Q2, A₃=O, R₁=CH(CH₃)₂, R₂=CH₃, R₄=CH₃, R₅=CH₃, 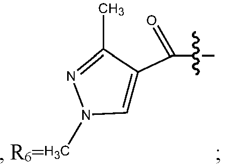
Compound 158: Q is selected from the structure of Q2, A₃=O, R₁=CH₃, R₂=CH₃, R₄=H, R₅=CH₃, 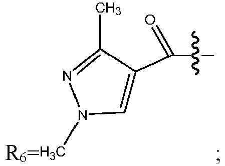
Compound 159: Q is selected from the structure of Q2, A₃=O, R₁=CH₂CH₃, R₂=CH₃, R₄=H, R₅=CH₃, 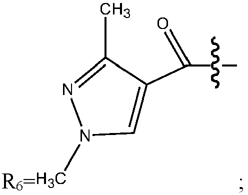
Compound 160: Q is selected from the structure of Q2, A₃=O, R₁=CH₂CH₂CH₃, R₂=CH₃, R₄=H, R₅=CH₃, 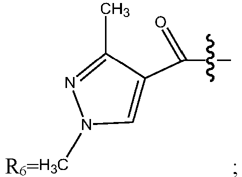
Compound 161: Q is selected from the structure of Q2, A₃=O, R₁=CH(CH₃)₂, R₂=CH₃, R₄=H, R₅=CH₃, 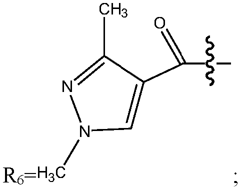
Compound 162: Q is selected from the structure of Q2, A₃=O, R₁=CH₃, R₂=CH₃, 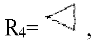 R₅=CH₃, 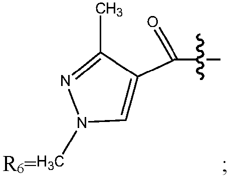
Compound 163: Q is selected from the structure of Q2, A₃=O, R₁=CH₂CH₃, R₂=CH₃, 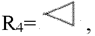 R₅=CH₃, 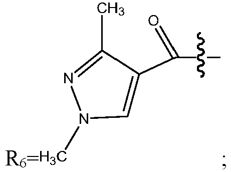
Compound 164: Q is selected from the structure of Q2, A₃=O, R₁=CH₂CH₂CH₃, R₂=CH₃, 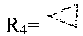 , R₅=CH₃, 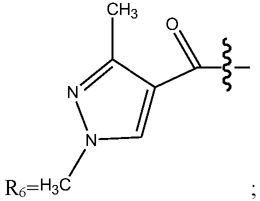
Compound 165: Q is selected from the structure of Q2, A₃=O, R₁=CH(CH₃)₂, R₂=CH₃, 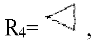 R₅=CH₃, 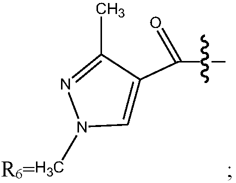
Compound 166: Q is selected from the structure of Q2, A₃=O, R₁=CH₃, R₂=CH₃, R₄= CH₃, R₅=CH₃, 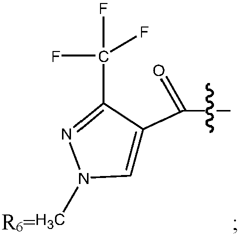
Compound 167: Q is selected from the structure of Q2, A₃=O, R₁=CH₂CH₃, R₂=CH₃, R₄=CH₃, R₅=CH₃, 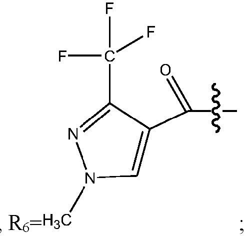
Compound 168: Q is selected from the structure of Q2, A₃=O, R₁=CH₂CH₂CH₃, R₂=CH₃, R₄=CH₃, R₅=CH₃, 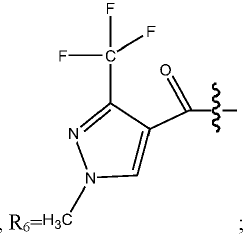
Compound 169: Q is selected from the structure of Q2, A₃=O, R₁=CH(CH₃)₂, R₂=CH₃, R₄=CH₃, R₅=CH₃, 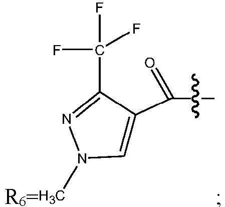
Compound 170: Q is selected from the structure of Q2, A₃=O, R₁=CH₃, R₂=CH₃, R₄=H, R₅=CH₃, 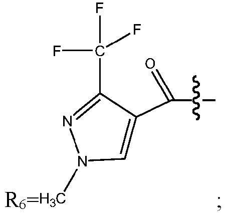
Compound 171: Q is selected from the structure of Q2, A₃=O, R₁=CH₂CH₃, R₂=CH₃, R₄=H, R₅=CH₃, 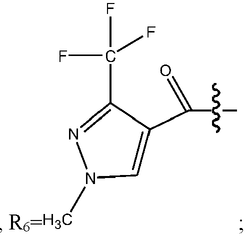
Compound 172: Q is selected from the structure of Q2, A₃=O, R₁=CH₂CH₂CH₃, R₂=CH₃, R₄=H, R₅=CH₃, 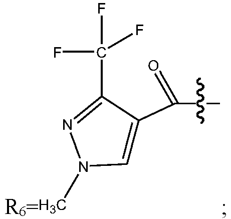
Compound 173: Q is selected from the structure of Q2, A₃=O, R₁=CH(CH₃)₂, R₂=CH₃, R₄=H, R₅=CH₃, 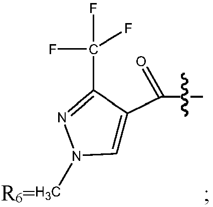
Compound 174: Q is selected from the structure of Q2, A₃=O, R₁=CH₃, R₂=CH₃, 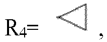 R₅=CH₃, 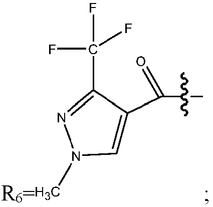
Compound 175: Q is selected from the structure of Q2, A₃=O, R₁=CH₂CH₃, R₂=CH₃, 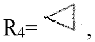 R₅=CH₃, 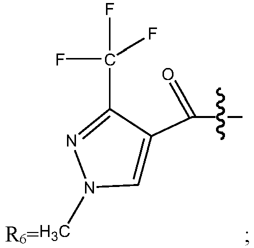
Compound 176: Q is selected from the structure of Q2, A₃=O, R₁=CH₂CH₂CH₃, R₂=CH₃, 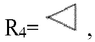 R₅=CH₃, 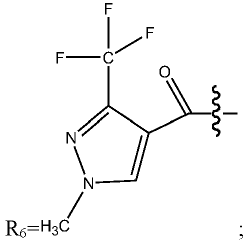
Compound 177: Q is selected from the structure of Q2, A₃=O, R₁=CH(CH₃)₂, R₂=CH₃, 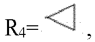 R₅=CH₃, 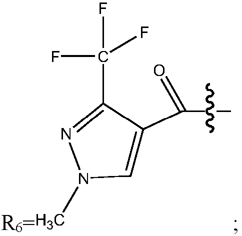
Compound 178: Q is selected from the structure of Q2, A₃=O, R₁=CH₃, R₂=CH₃, R₄=CH₃, R₅=CH₃, 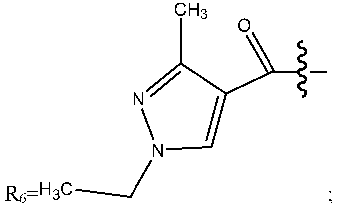
Compound 179: Q is selected from the structure of Q2, A₃=O, R₁=CH₂CH₂CH₃, R₂=CH₃, R₄=CH₃, R₅=CH₃, 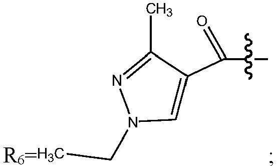
Compound 180: Q is selected from the structure of Q2, A₃=O, R₁=CH₃, R₂=CH₃, R₄=H, R₅=CH₃, 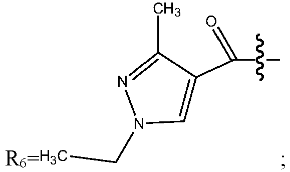
Compound 181: Q is selected from the structure of Q2, A₃=O, R₁=CH₂CH₂CH₃, R₂=CH₃, R₄=H, R₅=CH₃, 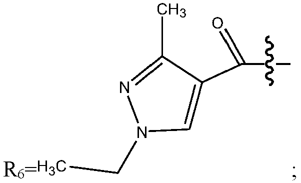
Compound 182: Q is selected from the structure of Q2, A₃=O, R₁=CH₃, R₂=CH₃, 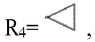 R₅=CH₃, 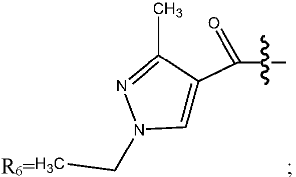
Compound 183: Q is selected from the structure of Q2, A₃=O, R₁=CH₂CH₂CH₃, R₂=CH₃, 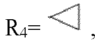 R₅=CH₃, 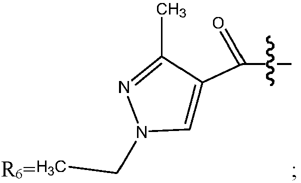
Compound 184: Q is selected from the structure of Q2, A₃=O, R₁=CH₂CH₃, R₂=CH₃, R₄=CH₃, R₅=CH₃, 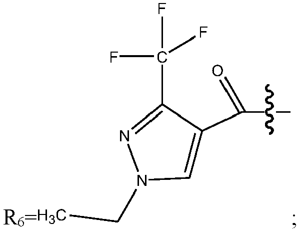
Compound 185: Q is selected from the structure of Q2, A₃=O, R₁=CH(CH₃)₂, R₂=CH₃, R₄=CH₃, R₅=CH₃, 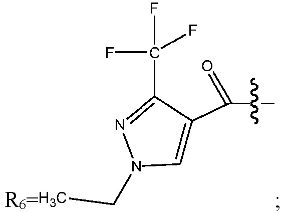
Compound 186: Q is selected from the structure of Q2, A₃=O, R₁=CH₂CH₃, R₂=CH₃, R₄=H, R₅=CH₃, 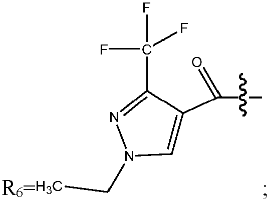
Compound 187: Q is selected from the structure of Q2, A₃=O, R₁=CH(CH₃)₂, R₂=CH₃, R₄=H, R₅=CH₃, 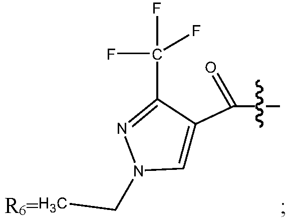
Compound 188: Q is selected from the structure of Q2, A₃=O, R₁=CH₂CH₃, R₂=CH₃, 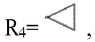 R₅=CH₃, 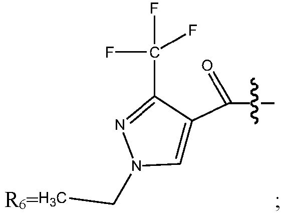
Compound 189: Q is selected from the structure of Q2, A₃=O, R₁=CH(CH₃)₂, R₂=CH₃, 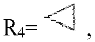 R₅=CH₃, 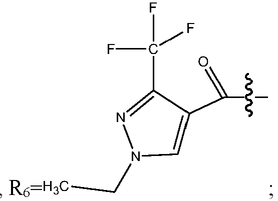
Compound 190: Q is selected from the structure of Q2, A₃=O, R₁=CH₂CH₃, R₂=CH₃, R₄=CH₃, R₅=CH₂CH₃, 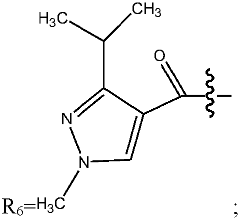
Compound 191: Q is selected from the structure of Q2, A₃=O, R₁=CH₃, R₂=CH₃, R₄=H, R₅=CH₂CH₃, 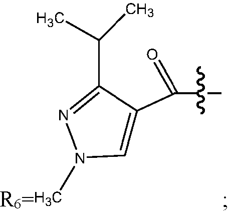
Compound 192: Q is selected from the structure of Q2, A₃=O, R₁=CH₂CH₂CH₃, R₂=CH₃, 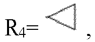 R₅=CH₂CH₃, 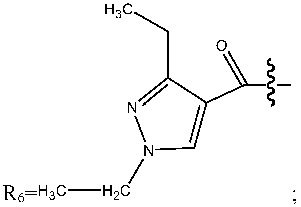
Compound 193: Q is selected from the structure of Q2, A₃=O, R₁=CH(CH₃)₂, R₂=CH₃, 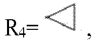 R₅=CH₂CH₃, 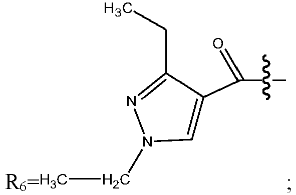
Compound 194: Q is selected from the structure of Q2, A₃=O, R₁=CH₃, R₂=CH₃, R₄=CH₃, R₅=CH₃, 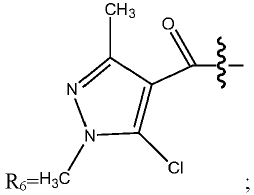
Compound 195: Q is selected from the structure of Q2, A₃=O, R₁=CH₂CH₃, R₂=CH₃, R₄=CH₃, R₅=CH₃, 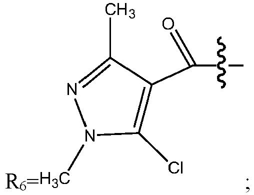
Compound 196: Q is selected from the structure of Q2, A₃=O, R₁=CH₂CH₂CH₃, R₂=CH₃, R₄=CH₃, R₅=CH₃, 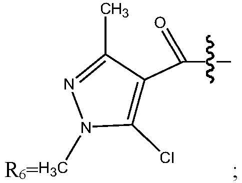
Compound 197: Q is selected from the structure of Q2, A₃=O, R₁=CH(CH₃)₂, R₂=CH₃, R₄=CH₃, R₅=CH₃, 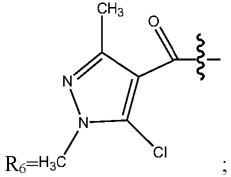
Compound 198: Q is selected from the structure of Q2, A₃=O, R₁=CH₃, R₂=CH₃, R₄=H, R₅=CH₃, 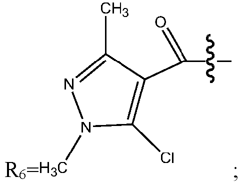
Compound 199: Q is selected from the structure of Q2, A₃=O, R₁=CH₂CH₃, R₂=CH₃, R₄=H, R₅=CH₃, 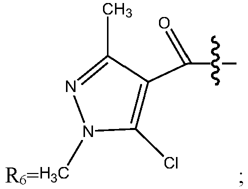
Compound 200: Q is selected from the structure of Q2, A₃=O, R₁=CH(CH₃)₂, R₂=CH₃, R₄=H, R₅=CH₃, 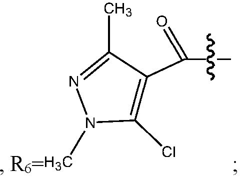
Compound 201: Q is selected from the structure of Q2, A₃=O, R₁=CH₃, R₂=CH₃, 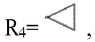 R₅=CH₃, 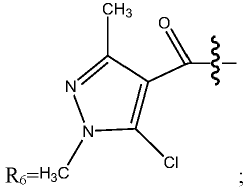
Compound 202: Q is selected from the structure of Q2, A₃=O, R₁=CH₂CH₃, R₂=CH₃, 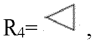 R₅=CH₃, 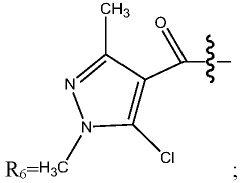
Compound 203: Q is selected from the structure of Q2, A₃=O, R₁=CH(CH₃)₂, R₂=CH₃, 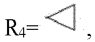 R₅=CH₃, 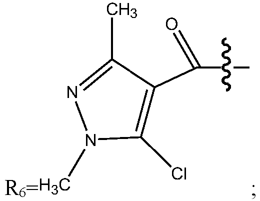
Compound 204: Q is selected from the structure of Q2, A₃=O, R₁=CH₃, R₂=CH₃, R₄=CH₃, R₅=CH₃, 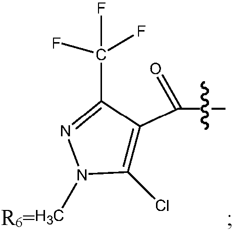
Compound 205: Q is selected from the structure of Q2, A₃=O, R₁=CH₂CH₃, R₂=CH₃, R₄=CH₃, R₅=CH₃, 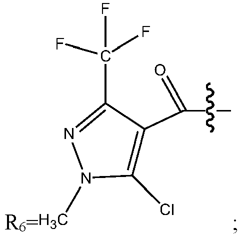
Compound 206: Q is selected from the structure of Q2, A₃=O, R₁=CH(CH₃)₂, R₂=CH₃, R₄=CH₃, R₅=CH₃, 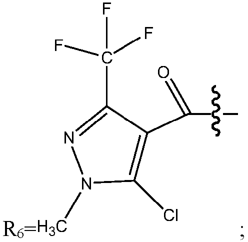
Compound 207: Q is selected from the structure of Q2, A₃=O, R₁=CH₃, R₂=CH₃, R₄=H, R₅=CH₃, 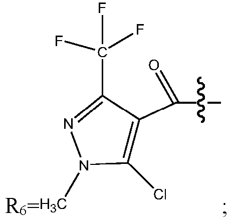
Compound 208: Q is selected from the structure of Q2, A₃=O, R₁=CH₂CH₃, R₂=CH₃, R₄=H, R₅=CH₃, 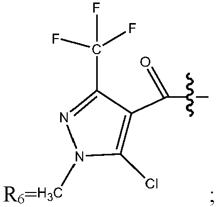
Compound 209: Q is selected from the structure of Q2, A₃=O, R₁=CH₂CH₂CH₃, R₂=CH₃, R₄=H, R₅=CH₃, 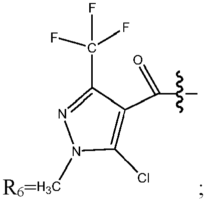
Compound 210: Q is selected from the structure of Q2, A₃=O, R₁=CH(CH₃)₂, R₂=CH₃, R₄=H, R₅=CH₃, 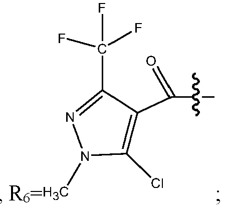
Compound 211: Q is selected from the structure of Q2, A₃=O, R₁=CH₂CH₃, R₂=CH₃, 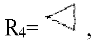 R₅=CH₃, 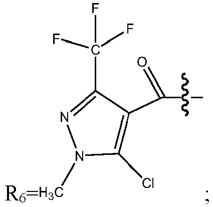
Compound 212: Q is selected from the structure of Q2, A₃=O, R₁=CH(CH₃)₂, R₂=CH₃, 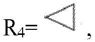 R₅=CH₃, 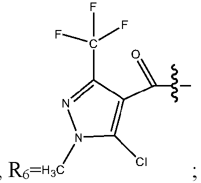
Compound 213: Q is selected from the structure of Q2, A₃=O, R₁=CH₃, R₂=CH₃, R₄=CH₃, R₅=CH₃, 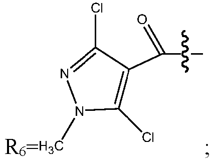
Compound 214: Q is selected from the structure of Q2, A₃=O, R₁=CH₂CH₃, R₂=CH₃, R₄=CH₃, R₅=CH₃, 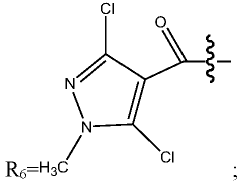
Compound 215: Q is selected from the structure of Q2, A₃=O, R₁=CH(CH₃)₂, R₂=CH₃, R₄=CH₃, R₅=CH₃, 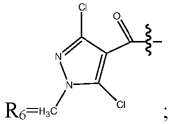
Compound 216: Q is selected from the structure of Q2, A₃=O, R₁=CH₃, R₂=CH₃, 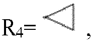 R₅=CH₃, 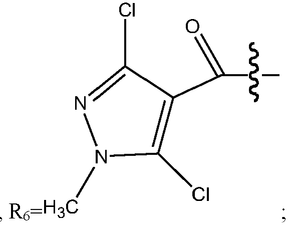
Compound 217: Q is selected from the structure of Q2, A₃=O, R₁=CH(CH₃)₂, R₂=CH₃, 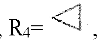 R₅=CH₃, 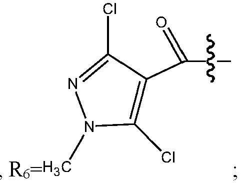
Compound 218: Q is selected from the structure of Q2, A₃=O, R₁=CH₃, R₂=CH₃, R₄=CH₃, R₅=CH₃, 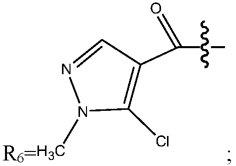
Compound 219: Q is selected from the structure of Q2, A₃=O, R₁=CH₂CH₃, R₂=CH₃, R₄=CH₃, R₅=CH₃, 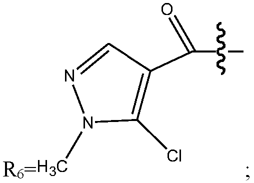
Compound 220: Q is selected from the structure of Q2, A₃=O, R₁=CH(CH₃)₂, R₂=CH₃, R₄=CH₃, R₅=CH₃, 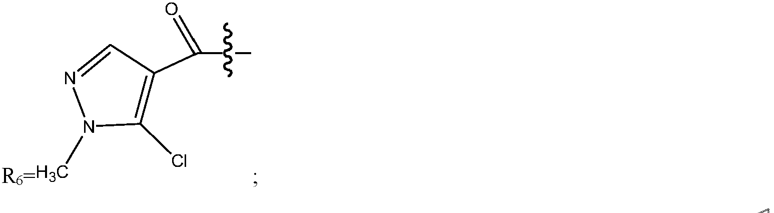
Compound 221: Q is selected from the structure of Q2, A₃=O, R₁=CH₃, R₂=CH₃, 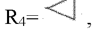 R₅=CH₃, 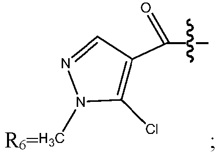
Compound 222: Q is selected from the structure of Q2, A₃=O, R₁=CH(CH₃)₂, R₂=CH₃, 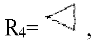 R₅=CH₃, 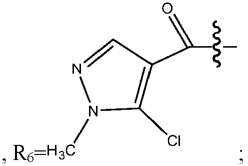
Compound 223: Q is selected from the structure of Q2, A₃=O, R₁=CH₃, R₂=CH₃, R₄=CH₃, R₅=CH₃, 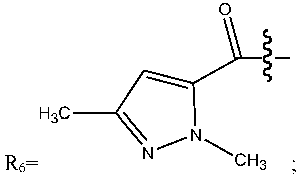
Compound 224: Q is selected from the structure of Q2, A₃=O, R₁=CH₂CH₃, R₂=CH₃, R₄=CH₃, R₅=CH₃, 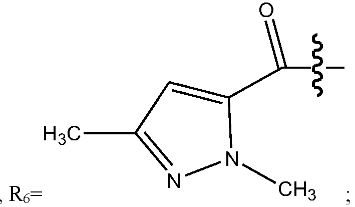
Compound 225: Q is selected from the structure of Q2, A₃=O, R₁=CH(CH₃)₂, R₂=CH₃, R₄=CH₃, R₅=CH₃, 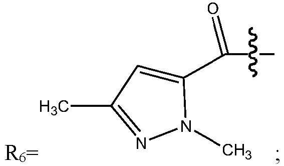
Compound 226: Q is selected from the structure of Q2, A₃=O, R₁=CH(CH₃)₂, R₂=CH₃, 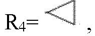 R₅=CH₃, 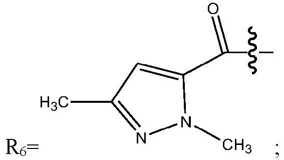
Compound 227: Q is selected from the structure of Q2, A₃=O, R₁=CH₃, R₂=CH₃, R₄=CH₃, R₅=CH₃, 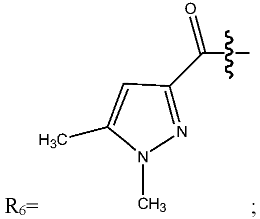
Compound 228: Q is selected from the structure of Q2, A₃=O, R₁=CH₂CH₃, R₂=CH₃, R₄=CH₃, R₅=CH₃, 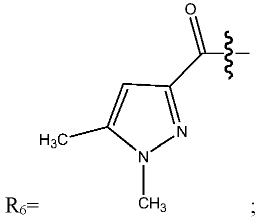
Compound 229: Q is selected from the structure of Q2, A₃=O, R₁=CH(CH₃)₂, R₂=CH₃, R₄=CH₃, R₅=CH₃, 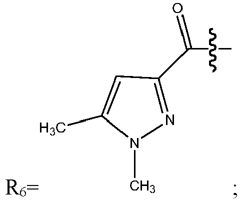
Compound 230: Q is selected from the structure of Q2, A₃=O, R₁=CH(CH₃)₂, R₂=CH₃, 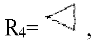 R₅=CH₃, 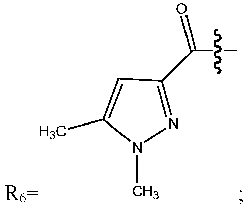
Compound 231: Q is selected from the structure of Q2, A₃=O, R₁=CH₃, R₂=CH₃, R₄=CH₃, R₅=CH₃, 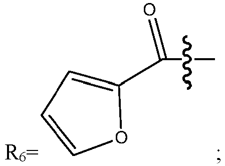
Compound 232: Q is selected from the structure of Q2, A₃=S, R₁=CH₃, R₂=CH₃, R₄=CH₃, R₅=CH₃, 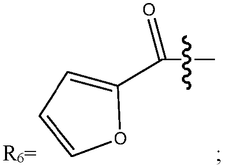
Compound 233: Q is selected from the structure of Q2, A₃=O, R₁=CH₂CH₃, R₂=CH₃, R₄=CH₃, R₅=CH₃, 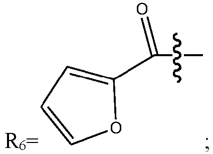
Compound 234: Q is selected from the structure of Q2, A₃=O, R₁=CH(CH₃)₂, R₂=CH₃, R₄=CH₃, R₅=CH₃, 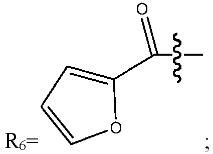
Compound 235: Q is selected from the structure of Q2, A₃=O, R₁=CH₃, R₂=CH₃, R₄= 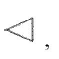 R₅=CH₃, 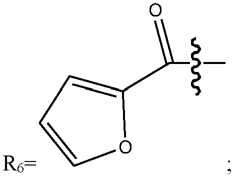
Compound 236: Q is selected from the structure of Q2, A₃=O, R₁=CH₂CH₃, R₂=CH₃, R₄= 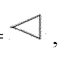 R₅=CH₃, 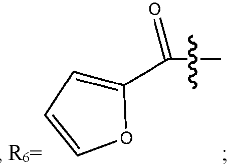
Compound 237: Q is selected from the structure of Q2, A₃=O, R₁=CH(CH₃)₂, R₂=CH₃, 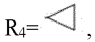 R₅=CH₃, 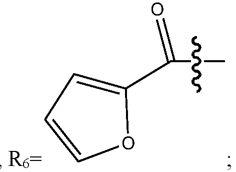
Compound 238: Q is selected from the structure of Q2, A₃=O, R₁=CH₃, R₂=CH₃, R₄=CH₃, R₅=CH₃, 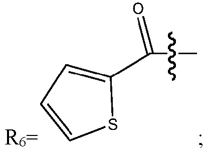
Compound 239: Q is selected from the structure of Q2, A₃=O, R₁=CH₂CH₃, R₂=CH₃, R₄=CH₃, R₅=CH₃, 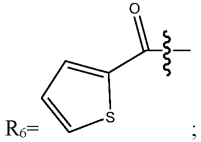
Compound 240: Q is selected from the structure of Q2, A₃=O, R₁=CH(CH₃)₂, R₂=CH₃, R₄=CH₃, R₅=CH₃, 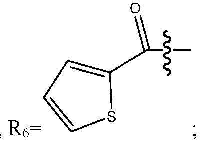
Compound 241: Q is selected from the structure of Q2, A₃=O, R₁=CH₃, R₂=CH₃, 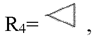 R₅=CH₃, 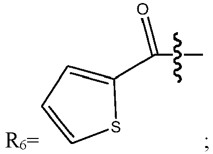
Compound 242: Q is selected from the structure of Q2, A₃=O, R₁=CH₂CH₃, R₂=CH₃, 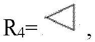 R₅=CH₃, 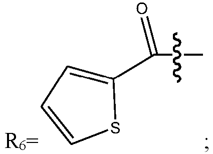
Compound 243: Q is selected from the structure of Q2, A₃=O, R₁=CH(CH₃)₂, R₂=CH₃, 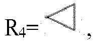 R₅=CH₃, 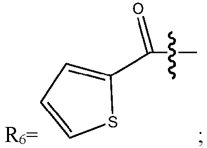
Compound 244: Q is selected from the structure of Q2, A₃=O, R₁=CH₃, R₂=CH₃, R₄=CH₃, R₅=CH₃, 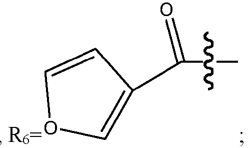
Compound 245: Q is selected from the structure of Q2, A₃=S, R₁=CH₃, R₂=CH₃, R₄=CH₃, R₅=CH₃, 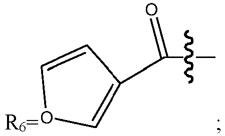
Compound 246: Q is selected from the structure of Q2, A₃=O, R₁=CH(CH₃)₂, R₂=CH₃, R₄=CH₃, R₅=CH₃, 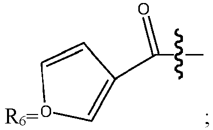
Compound 247: Q is selected from the structure of Q2, A₃=O, R₁=CH₃, R₂=CH₃, 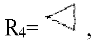 R₅=CH₃, 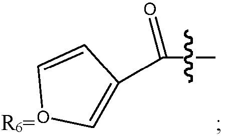
Compound 248: Q is selected from the structure of Q2, A₃=O, R₁=CH₂CH₃, R₂=CH₃, 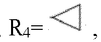 R₅=CH₃, 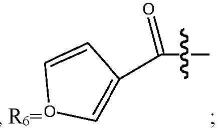
Compound 249: Q is selected from the structure of Q2, A₃=O, R₁=CH(CH₃)₂, R₂=CH₃, 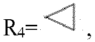 R₅=CH₃, 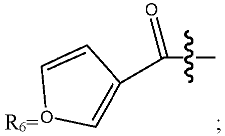
Compound 250: Q is selected from the structure of Q2, A₃=O, R₁=CH₃, R₂=CH₃, R₄=CH₃, R₅=CH₃, 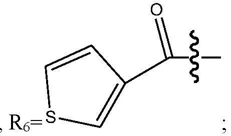
Compound 251: Q is selected from the structure of Q2, A₃=O, R₁=CH(CH₃)₂, R₂=CH₃, R₄=CH₃, R₅=CH₃, 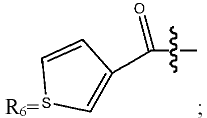
Compound 252: Q is selected from the structure of Q2, A₃=O, R₁=CH₃, R₂=CH₃, 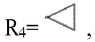 R₅=CH₃, 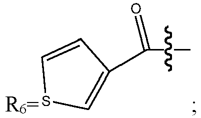
Compound 253: Q is selected from the structure of Q2, A₃=O, R₁=CH₂CH₃, R₂=CH₃, 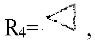 R₅=CH₃, 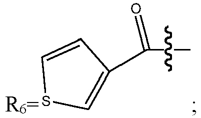
Compound 254: Q is selected from the structure of Q2, A₃=O, R₁=CH(CH₃)₂, R₂=CH₃, 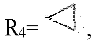 R₅=CH₃, 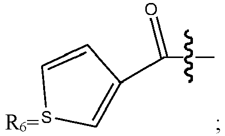
Compound 255: Q is selected from the structure of Q2, A₃=O, R₁=CH₃, R₂=CH₃, R₄=CH₃, R₅=CH₃, 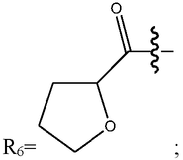
Compound 256: Q is selected from the structure of Q2, A₃=S, R₁=CH₃, R₂=CH₃, R₄=CH₃, R₅=CH₃, 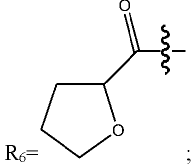
Compound 257: Q is selected from the structure of Q2, A₃=O, R₁=CH(CH₃)₂, R₂=CH₃, R₄=CH₃, R₅=CH₃, 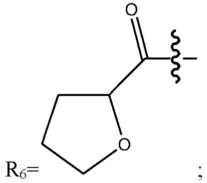
Compound 258: Q is selected from the structure of Q2, A₃=O, R₁=CH₃, R₂=CH₃, 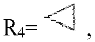 R₅=CH₃, 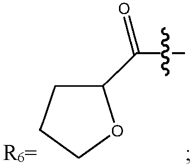
Compound 259: Q is selected from the structure of Q2, A₃=O, R₁=CH₂CH₃, R₂=CH₃, 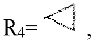 R₅=CH₃, 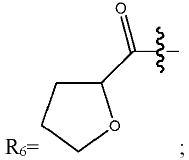
Compound 260: Q is selected from the structure of Q2, A₃=O, R₁=CH(CH₃)₂, R₂=CH₃, 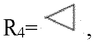 R₅=CH₃,

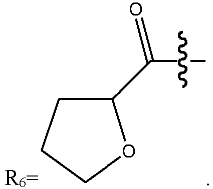

8. Use of the oxadiazolone compound or tautomer thereof of any one of claims 1-7 for controlling weeds.

9. The use of claim 8, **characterized in that** the weed is one or more selected from the group consisting of broadleaf weeds and grassy weeds.

10. A herbicidal composition, comprising the oxadiazolone compound or tautomer thereof of any one of claims 1-7 and an excipient.

11. A method for controlling weeds, **characterized by** comprising applying the herbicidal composition of claim 10 to crops.
